(19) Europäisches Patentamt
European Patent Office
Office européen des brevets

(11) EP 0 725 655 B2

(12) **NEW EUROPEAN PATENT SPECIFICATION**
After opposition procedure

(45) Date of publication and mention
of the opposition decision:
**15.04.2009 Bulletin 2009/16**

(45) Mention of the grant of the patent:
**27.08.2003 Bulletin 2003/35**

(21) Application number: **95902460.5**

(22) Date of filing: **07.11.1994**

(51) Int Cl.:
***A61K 39/395*** (2006.01)

(86) International application number:
**PCT/US1994/012779**

(87) International publication number:
**WO 1995/012412 (11.05.1995 Gazette 1995/20)**

(54) **Use of compounds capable of binding to glycoprotein gpIIb/IIIa for the prevention of restenosis**

Verwendung von an Glycoprotein gpIIb/IIIa bindenden Substanzen zur Vermeidung von Restenosis

Utilisation des substances capable d'attacher au glycoproteine gpIIb/IIIa pour la prévention de resténosis

(84) Designated Contracting States:
**AT BE CH DE DK ES FR GB GR IE IT LI LU MC NL PT SE**

(30) Priority: **05.11.1993 US 147928**

(43) Date of publication of application:
**14.08.1996 Bulletin 1996/33**

(60) Divisional application:
**02079444.2 / 1 334 730**

(73) Proprietors:
• **CENTOCOR INC.**
**Malvern,**
**Pennsylvania 19355-1307 (US)**
• **THE RESEARCH FOUNDATION OF STATE UNIVERSITY OF NEW YORK**
**Albany, NY 12210 (US)**

(72) Inventors:
• **COLLER, Barry S.**
**New York 10128 (US)**
• **Weisman, Harlan F.**
**Bryn Mawr Pennsylvania 19010 (US)**

(74) Representative: **Mercer, Christopher Paul et al**
**Carpmaels & Ransford**
**43-45 Bloomsbury Square**
**London WC1A 2RA (GB)**

(56) References cited:
EP-A- 0 206 532          EP-A- 0 206 533
WO-A-89/11538            WO-A-90/11783
WO-A-93/06863

• CARDIOVASCULAR DRUGS AND THERAPY, vol. 7, no.SUP2, 1993, NORWELL MA, USA page 459 W. ROTE ET AL. 'Prevention of carotid artery thrombosis: Effects of c7E3 Fab, aspirin and/or heparin in cynomolgus monkeys.'
• CIRCULATION, vol.84, no.4SUP, October 1991, NEW YORK NY, USA page II-590 J. TCHENG ET AL. 'Chimeric antiplatelet gpIIb/IIIa receptor antibody (C-7E3) in elective PTCA: Safety and platelet function inhibition.'
• CIRCULATION, vol.84, no.4S2, November 1991, NEW YORK NY, USA page II-247 J. IULIUCCI ET AL. 'Anti-platelet activity and safety of chimeric anti-platelet monoclonal antibody 7E3 Fab combined with streptokinase and anticoagulant drugs.'
• JOURNAL OF THE AMERICAN COLLEGE OF CARDIOLOGY, vol.21, no.2SA, 1993 page 269A M. SIMOONS ET AL. 'Chimeric 7E3 antiplatelet antibody Fab for treatment of refractory unstable angina, a placebo-controlled pilot study.'
• JOURNAL OF THE AMERICAN COLLEGE OF CARDIOLOGY, vol.21, no.2SA, 1993, NEW YORK NY, USA page 85A C. KOHMURA ET AL. 'A novel chimeric human-murine gpIIb-IIIa antiplatelet antibody enhances thrombolysis in the baboon.'
• BLOOD COAGULATION AND FIBRINOLYSIS, vol. 4, no.5, October 1993, OXFORD, GB pages 729 - 737 C. CHRISTOPOULOS ET AL. 'Flow cytometric observations on the in vivo use of Fab fragments of a chimaeric monoclonal antibody to platelet glycoprotein IIb-IIIa.'
• EUROPEAN HEART JOURNAL, vol.12, no.SUP, 1991, LONDON, GB page 26 S. BHATTACHARYA ET AL. 'Chimeric humanized monoclonal antiplatelet antibody 7E3 produces prolonged dose-dependent inhibition of platelet function.'

EP 0 725 655 B2

- THE LANCET, vol.343, no.8902, 9 April 1994, LONDON, GB pages 881 - 886 E. TOPOL ET AL. 'Randomised trial of coronary intervention with antibody against platelet IIb/IIIa integrin for reduction of clinical restenosis: results at six months.'
- Lawrence & Harker, Am J. Cardiol vol 60, 1987, p. 20B - 28B
- Anderson, Vessels, vol. 2, No1, 1996, p. 4 - 14
- Stedman's Medical Dictionary, 24th ed, 1982, Williams & Wilkins, p. 9741336, 1351,
- Bovsun, Biotechnology Newswatch vol, April 1994, p. 1, 3, 4

**Description**

Background of the Invention

Platelet aggregation is an essential event in the formation of blood clots. Under normal circumstances, blood clots serve to prevent the escape of blood cells from the vascular system. However, during certain disease states, clots can restrict or totally occlude blood flow resulting in cellular necrosis.

For example, platelet aggregation and subsequent thrombosis at the site of an atherosclerotic plaque is an important causative factor in the genesis of conditions such as angina, acute myocardial infarction, and reocclusion following successful thrombolysis and angioplasty. Heart attack patients are typically treated with thrombolytic agents such as tissue plasminogen activator or streptokinase, which dissolve the fibrin component of clots. A major complication associated with fibrinolysis is reocclusion based on platelet aggregation which can result in further heart damage. Since glycoprotein (GP)IIb/IIIa receptors are known to be responsible for platelet aggregation, reagents which block these receptors are expected to reduce or prevent reocclusion following thrombolytic therapy and to accelerate the rate of thrombolysis. Such reagents are also expected to be useful in therapy of other vaso-occlusive and thromboembolic disorders.

One approach to blocking platelet aggregation involves monoclonal antibodies specific for GPIIb/IIIa receptors. A murine monoclonal antibody, designated 7E3, that inhibits platelet aggregation and appears useful in the treatment of human thrombotic diseases is described in published European Patent Application Nos. 205, 207 and 206, 532. It is known in the art that murine antibodies have characteristics which may severely limit their use in human therapy. As foreign proteins, murine antibodies may elicit immune reactions that reduce or destroy their therapeutic efficacy and/or evoke allergic or hypersensitivity reactions in patients. The need for readministration of such therapeutic modalities in thromboembolic disorders increases the likelihood of these types of immune reactions.

Chimeric antibodies consisting of non-human binding regions joined to human constant regions have been suggested as a means to circumvent the immunoreactivity problems of murine antibodies. See Proc. Natl. Acad. Sci. USA, 81:6851 (1984) and PCT Application No. PCT/GB85 00392 (WO 86/01533). Since the constant region is largely responsible for immunoreactivity of an antibody molecule, chimeric antibodies with constant regions of human origin should be less likely to evoke an anti-murine response in humans. However, it is unpredictable whether the joining of a human constant region to a murine binding region of a desired specificity will reduce immunoreactivity and/or alter the binding capability of the resulting chimeric antibody.

WO 89/11538 describes platelet-specific chimeric antibodies and fragments including the chimeric antibody 7E3, its preparation and fragments thereof. It is stated that these antibodies and fragments can be used as anti-thrombotic agents.

Journal of the American College of Cardiology 21/2SA, 1993, 269A, abstract 768-1 describes the treatment of patients with unstable angina. A chimeric 7E3 fragment was administered to patients prior to PTCA. Treatment with the chimeric 7E3 fragment, heparin and aspirin appeared to be safe.

Circulation 84/4SUP, 1991, II-590, abstract 2344 describes the use of "C-7E3" for inhibiting platelet function during PTCA.

WO93/06863 describes a method for inhibiting vascular narrowing at an intravascular site. The disclosed method comprises administering an anti-PADGEM antibody.

Summary of the Invention

This invention pertains to the use of a compound that selectively binds to glycoprotein IIb/IIIa, for the manufacture of a medicament for inhibiting restenosis in a human. The compound is a platelet-specific chimeric immunoglobulin comprising a variable or antigen binding region of non-human origin and a constant region of human origin. These antibodies bind to platelets and can block platelet. aggregation and thus are useful as antithrombotic agents, in the prevention of restenosis.

The present invention is defined by the appended claims .

Brief Description of the Figures

Figure 1 shows a Northern analysis of heavy chain and light chain mRNAs for the 7E3 monoclonal antibody using cloned variable regions as probes.

Figures 2A and 2B are schematic representations of the plasmids p7E3V$_\kappa$hC$_\kappa$ (Fig. 2A) and p7E3V$_H$hC$_{G4}$ (Fig. 2B), which carry the chimeric gene constructs encoding the light and heavy chains, respectively, of a chimeric 7E3 immunoglobulin.

Figure 3 shows the binding of the chimeric 7E3 immunoglobulin encoded by vectors $p7E3V_\kappa hC_\kappa$ and $p7E3V_H hC_{G4}$ to platelets.

Figure 4 shows the inhibition of platelet aggregation by a chimeric 7E3 (c7E3) immunoglobulin.

Figure 5 is a graphic illustration of a plot of the plasma antibody concentration (ng/mL) versus time (days) which demonstrates the rapid initial clearance of c7E3 Fab ($\gamma_1$, $\kappa$) from the plasma in three patients with stable coronary disease, following a 0.25-mg/kg dose of c7E3 Fab administered intravenously as a five minute infusion.

Figures 6 (A-C) are illustrations summarizing the effect on platelet activity of a single bolus dose of chimeric 7E3 Fab (0.15 mg/kg, 0.20 mg/kg or 0.25 mg/kg) 2 hours after administration of antibody ($\gamma_1$, $\kappa$). A dose response is evident when platelet activity is assayed in terms of receptor blockade (Figure 6A, top), platelet aggregation (Figure 6B, middle), and bleeding time (Figure 6C, bottom). The lines represent median values.

Figures 7 (A-C) are illustrations of the duration of anti-platelet effect of chimeric 7E3 Fab ($\gamma_1$, $\kappa$) administered prior to angioplasty in a bolus dose of 0.25 mg/kg. The lines indicate the median values from time zero at baseline through 24 hours for receptor blockade (Figure 7A, top), platelet aggregation (Figure 7B, middle), and bleeding time (Figure 7C, bottom).

Figures 8 (A-C) are illustrations summarizing the anti-platelet activity of a 0.25 mg/kg bolus dose followed by a 12 hour continuous infusion (10 $\mu$g/minute) of chimeric 7E3 Fab ($\gamma_1$, $\kappa$) in 11 patients. The lines represent median values determined for percent receptor blockade (Figure 8A, top), percent of pre-dose (baseline at time zero) platelet aggregation (Figure 8B, middle), and bleeding times (Figure 8C, bottom).

Figure 9 is an illustration of the absolute change in hematocrit from baseline to a time 24 hours following the end of infusion for 47 patients described in Example 4.

Figure 10 is a Kaplan-Meier plot illustrating the probability of no urgent repeat percutaneous revascularization procedures from the time of randomization for the three treatment groups.

Figure 11 is a graph displaying the odds ratios and 95% confidence intervals for key subgroups (listed at right) entered in the trial. Data are presented for the primary efficacy endpoint (death, nonfatal infarction, urgent angioplasty or surgery, or placement of coronary stent or intraaortic balloon pump for refractory ischemia). In addition, the absolute event rates for the primary endpoint for each subgroup are tabulated at the left (Event Rates (%)).

Figure 12 is a graph illustrating the fraction of all patients with no event over the 6 month follow-up period.

Figure 13 is a graph illustrating the fraction of patients with no event over the 6 month follow-up period among those patients who had a successful intervention and no events until after 30 days.

Figure 14 is a graph illustrating the fraction of patients with no event over the 6 month follow-up period considering events after the first 48 hours among patients with an initially successful intervention.

Figure 15 is a graph illustrating the fraction of all patients with no attempt to revascularize the procedure related artery (PRA, procedure related artery) over the 6 month follow-up period.

Detailed Description of the Invention

The chimeric immunoglobulins used in the present invention may be comprised of individual chimeric heavy and light immunoglobulin chains. The chimeric heavy chain is comprised of an antigen-binding region from the heavy chain of non-human antibody 7E3 specific for platelets (specific for the GpIIb/IIIa receptor) linked to a human heavy chain constant region. The chimeric light chain comprises an antigen binding region from the light chain of the non-human antibody 7E3 linked to a human light chain constant region.

The present immunoglobulins can be monovalent, divalent or polyvalent. Monovalent immunoglobulins are dimers (HL) formed of a chimeric heavy chain associated through disulfide bridges with a chimeric light chain. Divalent immunoglobulins are tetramers ($H_2L_2$) formed of two dimers associated through at least one disulfide bridge. Polyvalent immunoglobulins can also be produced, for example, by employing a heavy chain constant region that aggregates (e.g., $\mu$ heavy chain constant regions). Chimeric immunoglobulin fragments such as Fab, Fab' or F(ab')$_2$ can also be produced.

The non-human antigen binding regions of the chimeric immunoglobulin are from immunoglobulin 7E3 specific for platelet GPIIb/IIIa receptors which can block ligand binding to the glycoprotein IIb/IIIa receptor complex.

Thrombosis begins with the adhesion of platelets at sites of vessel wall injury. The adhesion of platelets is mediated by platelet surface receptors which bind to extracellular matrix proteins in the exposed subendothelium, such as von Willebrand factor, collagen, fibronectin, vitronectin, and laminin. Platelet adhesion results in a monolayer of platelets. Subsequently, platelet activation occurs in response to agonists such as epinephrine, ADP, collagen, and thrombin. Activation leads to the exposure of the glycoprotein IIb/IIIa receptor (GPIIb/IIIa) on the platelet surface. GPIIb/IIIa on activated platelets is then available to bind to fibrinogen, which can mediate platelet aggregation. The binding of GPIIb/IIIa to other adhesive proteins, such as von Willebrand factor can also cause platelet cross-linking and aggregation. Thus, the binding of adhesive molecules, such as fibrinogen or von Willebrand factor, to GPIIb/IIIa to cause aggregation of platelets is a common step in thrombus formation, making the GPIIb/IIIa receptor an attractive target for therapeutic agents which can interfere with the interaction of GPIIb/IIIa with these molecules. Furthermore, by use of an anti-GPIIb/IIIa chimeric antibody, the aggregation of activated platelets is expected to be inhibited, without interfering with the initial adhesion of platelets. This selective inhibition of platelet aggregation may be desirable because platelet adhesion, without aggregation, may contribute to maintaining hemostasis.

The 7E3 antibody is specific for the complexed form of the GPIIb/IIIa receptor.

The constant regions of the chimeric antibodies are derived from human immunoglobulins. The heavy chain constant region can be selected from any of the five isotypes alpha, delta, epsilon, gamma or mu. Further, heavy chains of various subclasses (such as the IgG subclasses of heavy chains) are responsible for different effector functions and thus, by choosing the desired heavy chain constant region, chimeric antibodies with desired effector function can be produced. Preferred constant regions are gamma 1 (IgG1), gamma 3 (IgG3) and gamma 4 (IgG4). The light chain constant region can be of the kappa or lambda type.

In general, the chimeric antibodies are produced by preparing, for each of the light and heavy chain components of the chimeric immunoglobulin, a fused gene comprising a first DNA segment that encodes at least the functional portion of the variable region of 7E3 (e.g., functionally rearranged variable region with joining segment) linked to a second DNA segment encoding at least a part of a human constant region. Each fused gene is assembled in or inserted into an expression vector, yielding an expression vector containing a fused gene in expressible form. In one embodiment, DNA comprising the antigen binding region is covalently joined to the constant region via an intervening sequence. In another embodiment, constructs lacking one or more intervening sequences can be constructed or obtained. Recipient cells capable of expressing the gene products are then transfected with the genes. The transfected recipient cells are cultured under conditions that permit expression of the incorporated genes and the expressed immunoglobulins or immunoglobulin chains are recovered.

Genes encoding the variable region of Ig light and heavy chains can be obtained from lymphoid cells that produce 7E3 antibodies.

Constant regions can be obtained from human antibody-producing cells by standard cloning techniques. Alternatively, because genes representing the two classes of light chains and the five classes of heavy chains have been cloned, constant regions of human origin are readily available from these clones. Chimeric antibody binding fragments such as $F(ab')_2$ and Fab fragments can be prepared by designing a chimeric heavy chain gene in truncated form. For example, a chimeric gene encoding a $F(ab')_2$ heavy chain portion would include DNA sequences encoding the $CH_1$ domain and hinge region of the heavy chain. Alternatively, such fragments can be obtained by enzymatic cleavage of a chimeric immunoglobulin. For instance, papain or pepsin cleavage can generate Fab or $F(ab')_2$ fragments, respectively.

Preferably, the fused genes encoding the light and heavy chimeric chains (or portions thereof) are assembled in two different expression vectors that can be used to cotransfect a recipient cell. Each vector contains two selectable genes--one for selection in a bacterial system and one for selection in a eukaryotic system--each vector having a different pair of genes. These vectors allow production and amplification of the fused genes in bacterial systems, and subsequent cotransfection of eukaryotic cells and selection of the cotransfected cells. Examples of selectable genes for the bacterial system are the genes that confer ampicillin resistance and the gene that confers chloramphenicol resistance. Two selectable genes for selection of eukaryotic transfectants are preferred: (i) the xanthine-guanine phosphoribosyltransferase gene (gpt), and (ii) the phosphotransferase gene from Tn5 (designated neo). Selection with gpt is based on the ability of the enzyme encoded by this gene to use xanthine as a substrate for purine nucleotide synthesis; the analogous endogenous enzyme cannot. In a medium containing xanthine and mycophenolic acid, which blocks the conversion of inosine monophosphate to xanthine monophosphate, only cells expressing the gpt gene can survive. The product of the neo gene blocks the inhibition of protein synthesis in eukaryotic cells caused by the antibiotic G418 and other antibiotics of its class. The two selection procedures can be used simultaneously or sequentially to select for the expression of immunoglobulin chain genes introduced on two different DNA vectors into a eukaryotic cell.

The preferred recipient cell line is a myeloma cell. Myeloma cells can synthesize, assemble and secrete immunoglobulins encoded by transfected Ig genes. Further, they possess the mechanism for glycosylation of the immunoglobulin. A particularly preferred recipient cell is an Ig-non-producing myeloma cell line such as Sp2/0. These cell lines produce only the immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Several methods exist for transfecting lymphoid cell with vectors containing immunoglobulin encoding genes. A preferred way of introducing DNA into lymphoid cells is by electroporation. In this procedure recipient cells are subjected to an electric pulse in the presence of the DNA to be incorporated. See e.g., Potter et al., Proc. Natl. Acad. Sci. USA, 81:7161 (1984). Another way to introduce DNA is by protoplast fusion. In this method, lysozyme is used to strip cell walls from bacteria harboring the recombinant plasmid containing the chimeric Ig gene. The resulting spheroplasts are fused with myeloma cells with polyethylene glycol. After protoplast fusion, the transfectants are selected and isolated. Another technique that can be used to introduce DNA into many cell types is calcium phosphate precipitation.

The chimeric immunoglobulin genes can also be expressed in nonlymphoid cells such as bacteria or yeast.

When expressed in bacteria, the immunoglobulin heavy chains and light chains become part of inclusion bodies. Thus, the chains must be isolated and purified and then assembled into functional immunoglobulin molecules. Other strategies for expression in E. coli are available (see e.g., Pluckthun, A., Bio/Technology, 9:545-551 (1991); Skerra, A. et al., Bio/Technology, 9:273-278 (1991)), including secretion from E. coli as fusion proteins comprising a signal sequence.

Utility of Platelet-specific Chimeric Immunoglobulin

The chimeric platelet-specific antibodies of this invention (or fragments thereof) can be used in a variety of situations where restenosis is to be inhibited (reduced, delayed or prevented).

For example, patients at risk of or having coronary artery disease can benefit from the administration of an effective amount of a chimeric anti-platelet antibody fragment of the present invention (e.g., a chimeric 7E3 antibody or preferably a fragment thereof, such as chimeric 7E3 Fab or F(ab')$_2$) to inhibit restenosis of vessels.

For example, the antibody can be administered to a human before, during or after angioplasty procedures performed by balloon, coronary atherectomy, laser angioplasty or other suitable methods. Antibody can be administered prior to the angioplasty procedure (pre-angioplasty), during angioplasty, or post-angioplasty. For reference such treatment can prevent thrombosis and thereby reduce the rate of thrombotic complications following angioplasty, such as death, myocardial infarction, or recurrent ischemic events necessitating PTCA or coronary bypass surgery (acute ischemic events). In addition, such treatment can yield a longer-term benefit by reduction of ischemic events or complications of a coronary artery intervention procedure (e.g., angioplasty), such as death, myocardial infarction, or recurrent ischemic events necessitating PTCA or coronary bypass surgery (revascularization procedures), indicative of reduction, delay or prevention of restenosis.

For instance as shown in Reference Example 4, administration of a chimeric anti-platelet antibody (chimeric 7E3 Fab fragment) as adjuvant therapy prior to angioplasty (percutaneous transluminal coronary angioplasty, PTCA) effectively increased bleeding times and reduced agonist-induced platelet aggregation as assayed by ex vivo platelet aggregation assays. The results of the experiments reported in Reference Examples 4 and 5 also suggest that blockade of platelet GPIIb/IIIa and inhibition of aggregation by c7E3 antibody (a Fab fragment) translates into in vivo antithrombotic efficacy in humans.

The results of a randomized, double-blind, placebo-controlled study of administration of a chimeric 7E3 antibody fragment are presented in Reference Example 6 and Example 7. The data presented in Reference Example 6 reveal that administration of chimeric 7E3 antibody fragment to patients undergoing angioplasty and at high risk of abrupt closure (reocclusion) can prevent abrupt closure (reocclusion), decreasing the incidence of acute ischemia. As is further illustrated in Example 7, administration of a chimeric 7E3 antibody fragment to patients undergoing angioplasty and at high risk of abrupt closure (reocclusion) can reduce, delay and/or prevent restenosis at later times.

The long-term benefit as shown by reduction of non-acute ischemic complications observed upon administration of a chimeric anti-GPIIb/IIIa antibody fragment (see Example 7) is unprecedented. Additional chimeric immunoglobulins or immunoglobulin fragments of the invention can be used to prevent or reduce restenosis, with the clinical benefit of reducing the occurrence of non-acute ischemic complications. Patients at risk of or having coronary artery disease can benefit from the administration of an effective amount of chimeric immunoglobulins or immunoglobulin fragments of the invention thereby inhibiting restenosis of vessels.

The medical uses of the present invention are defined in the appended claims.

Administration

The aggregation of platelets activates the coagulation cascade and produces a more stable fibrin meshwork and occlusive clot, which can be lysed by thrombolytic agents. The antibody of the present invention can be administered to a human alone or in conjunction with a thrombolytic agent, such as a plasminogen activator (e.g., tissue plasminogen activator, urokinase, streptokinase, recombinant tissue plasminogen activator), or an anticoagulant (e.g., an antithrombin agent) or anti-platelet agent, such as aspirin, heparin, hirulog, hirudin, or a coumarin anticoagulant (e.g., warfarin), to prevent or reduce reocclusion that can occur after thrombolysis and to accelerate clot lysis, for example. The antibody or fragment thereof can also be administered before, along with or subsequent to administration of a thrombolytic agent, antithrombin agent, anticoagulant or anti-platelet agent, in amounts sufficient to delay or prevent restenosis.

An effective amount (i.e., an amount sufficient to achieve the desired therapeutic effect, such as an amount sufficient an amount sufficient to reduce/delay or prevent restenosis or ischemic events) of a compound or an antibody or antibody fragment can be given parenterally, preferably intravenously, in a pharmaceutically acceptable vehicle such as sterile saline. Buffered media may be included. The antibody formulation can contain additional additives, such as a stabilizer (e.g., Polysorbate 80, USP/NF). The antibody can be administered in a single dose, continuously, or in multiple infusions (e.g., a bolus injection, followed by continuous infusion). Alternatively, the compound or antibody could be administered by a controlled release mechanism (e.g., by a polymer or patch delivery system) or by another suitable method. The amount to be administered will depend on a variety of factors such as the clinical symptoms, weight of the individual, whether other drugs (e.g., thrombolytic agents) are administered.

During repeat therapy with anti-platelet antibodies, drug-induced thrombocytopenia may occur; this may be a result of the body recognizing the antibody-coated platelets as foreign proteins, raising antibodies against them, and then clearing them via the reticuloendothelial system more rapidly than normal. Because of the uniquely high density of the GPIIb/IIIa receptor on the platelet surface ($\sim$80,000 receptors per platelet) and the large number of platelets in the circulation (-0.25 -0.5 X $10^6$ per $\mu$l), thrombocytopenia may be an important complication of treatment with anti-platelet antibodies. The use of a chimeric 7E3 antibody can avoid this problem. The chimeric anti-platelet antibodies of the present invention may minimize (reduce or prevent) the thrombocytopenia which might otherwise occur on administration of an anti-platelet antibody. For example, minimal thrombocytopenia was observed on administration of chimeric 7E3 Fab (see e.g., Examples 6 and 7).

Secondly, upon administration to humans, chimeric 7E3 Fab antibody fragment displays a surprisingly reduced incidence of induced immunogenicity as compared with its murine counterpart (see e.g., Examples 4 and 7). Upon binding platelets, the majority of the murine component of the anti-platelet chimeric antibody will be bound to the platelet surface, e.g., via the GPIIb/IIIa receptor, and thus will be inaccessible to the immune system, rendering the chimeric antibody functionally indistinguishable from a human antibody directed against the same epitope. Therefore, other chimeric anti-platelet antibodies of the present invention may be similarly non-immunogenic in spite of their murine antigen binding region.

The invention is further described by the following examples, which are not intended to be limiting in any way.

EXEMPLIFICATION

Example 1

Production of chimeric platelet specific IgG4

A. General Strategy

The strategy for cloning the variable regions for the heavy and light chain genes from the 7E3 hybridoma was based upon the linkage in the genome between the variable region and the corresponding J (joining) region for functionally rearranged (and expressed) Ig genes. J region DNA probes can be used to screen genomic libraries to isolate DNA linked to the J regions; DNA in the germline configuration (unrearranged) would also hybridize to J probes but is not linked to a variable region sequence and can be identified by restriction enzyme analysis of the isolated clones.

The cloning strategy, therefore, was to isolate variable regions from rearranged heavy and light chain genes using $J_H$ and $J_K$. These clones were tested to see if their sequences were expressed in the 7E3 hybridoma by Northern analysis. Those clones that contained expressed sequences were put into expression vectors containing human constant regions and transfected into mouse myeloma cells to determine if an antibody was produced. The antibody from producing cells was then tested for binding specificity and functionality in comparison with the 7E3 murine antibody.

A deposit of cell line derivative, murine hybridoma 7E3, was made with the American Type Culture Collection, 12301 Parklawn Drive, Rockville, MD 20852, on May 30, 1985. The accession number HB8832 was assigned after successful viability testing.

B. Materials and Methods

All parts and percentages are by weight, and degrees are Celsius unless otherwise stated.

Heavy Chain Genomic Library Construction

To isolate the heavy chain variable region gene from the 7E3 hybridoma, a size-selected genomic library was constructed using the phage lambda vector gtlO. Southern analysis of EcoRI digested 7E3 DNA using a $J_H$ probe revealed a single 3.5 kb band corresponding to a rearranged heavy chain locus. It was likely that this fragment contained the 7E3 heavy chain variable region gene. High molecular weight DNA was isolated from 7E3 hybridoma cells and digested to completion with restriction endonuclease EcoRI. The DNA was then fractionated on a 0.7% agarose gel and the DNA of size range 3-4 kb was isolated directly from the gel. After phenol/chloroform extraction and Sephadex G-50 gel filtration, the 3-4 kb fragments were ligated with lambda gtlO arms (Promega Biotech, Inc.) and packaged into phage particles in vitro using Packagene from Promega Biotech. This library was screened directly at a density of approximately 30,000 plaques per 150 mm petri dish using a $^{32}$P-labeled $J_H$ probe. Plaque hybridizations were carried out in 5X SSC, 50% formamide, 2X Denhardt's reagent, 200 $\mu$g/ml denatured salmon sperm DNA at 42 degrees C for 18-20 hours.

Final washes were in 0.5X SSC, 0.1% SDS at 65 degrees. Positive clones were identified after autoradiography.

Light Chain Genomic Library Construction

To isolate the variable region gene for the 7E3 light chain, a genomic library was constructed in the lambda vector EMBL-3. High molecular weight DNA was partially digested with restriction endonuclease Sau3A and size-fractionated on a 10-40% sucrose density gradient. DNA fragments of 18-23kb were ligated with EMBL-3 arms and packaged into phage particles in vitro using Packagene. This library was screened at a density of 30,000 plaques per 150 mm plate using a $J_\kappa$ probe. Hybridization and wash conditions were identical to those used for the heavy chain library.

DNA Probes

The mouse heavy chain $J_H$ probe is a 2 kb BamHI/EcoRI fragment containing both J3 and J4 segments. The mouse light chain $J_\kappa$ probe is a 2.7 kb HindIII fragment containing all five $J_\kappa$ segments. $^{32}$P-labeled probes were prepared by nick translation using a kit obtained from Amersham, Inc. Free nucleotides were removed by centrifugation through a Sephadex G-50 column. The specific activities of the probes were approximately $10^9$ cpm/$\mu$g.

Northern Analysis

15 $\mu$g total cellular RNA was subjected to electrophoresis on 1% agarose/formaldehyde gels (Maniatis, et al, Molecular Cloning) and transferred to nitrocellulose. Blots were hybridized with nick translated DNA probes in 50% formamide, 2X Denhardt's solution, 5X SSC, and 200 $\mu$g/ml denatured salmon sperm DNA at 42 degrees for 10 hours. Final wash conditions were 0.5X SSC, 0.1% SDS at 65 degrees.

DNA Transfection using Electroporation

Plasmid DNA to be transfected was purified by centrifuging to equilibrium in ethidium bromide/cesium chloride gradients two times. 10-50 $\mu$g of plasmid DNA was added to 8 x $10^6$ SP2/0 cells in PBS on ice and the mixture placed in a Biorad electroporation apparatus. Electroporation was at 200 volts and the cells were plated out in 96 well microtiter plates. Appropriate drug selection was applied after 48 hours and drug resistant colonies were identified after 1-2 weeks.

Quantitation of Antibody production

Tissue culture supernatant was analyzed for IgG protein content by particle concentration fluorescence immunoassay (Jolley, M.E. et al., (1984) J. Immunol. Meth. 67:21) using standard curves generated with purified IgG. Concentration of chimeric 7E3 antibody with human constant regions was determined using goat antihuman IgG Fc antibody-coated polystyrene beads and fluorescein conjugated goat anti-human IgG Fc antibody. The assay was carried out with an automated instrument (Pandex Laboratories, Inc.).

Purification of Platelet-specific Chimeric IgG4 Antibody

Tissue culture supernatant was concentrated with a Diaflo YM100 ultrafiltration membrane (Amicon), and loaded onto a protein A-sepharose column. The chimeric antibody was eluted from the protein A column with a sodium citrate pH gradient from pH 6.5 to pH 3.5. The purified antibody was concentrated using a Diaflo YM100 ultrafiltration membrane. Antibody concentration was measured by determining the absorbance at 280 nm.

Binding Inhibition Assay

Purified antibody (either murine 7E3 or chimeric 7E3) was used to compete with radioiodinated 7E3 antibody for binding to human platelets. Platelet-rich plasma (PRP) was prepared by centrifugation of citrated whole human blood at 1875 rpm for 3.5 minutes. $^{125}$I-labeled 7E3 antibody (150,000 cpm) was added to the appropriate dilution of the purified test antibody and the reaction was initiated by the addition of 150 $\mu$l PRP. Incubation was for 1-2 hours at room temperature and the platelets with bound antibody were separated from free antibody by centrifugation through 30% sucrose at 12,000 g for 4 minutes in a 0.4 ml microfuge tube. The tube tip containing the platelet/antibody pellet was cut off and counted in a gamma counter. The competition for binding to platelets between iodinated 7E3 and chimeric 7E3 was compared to the competition between iodinated 7E3 and unlabeled 7E3 IgG.

Inhibition of Platelet Aggregation

Purified 7E3 or chimeric 7E3 antibody was added to citrated whole human blood and incubated at 37 degrees for 10 minutes. The rate of platelet aggregation was measured after activation with collagen or ADP using a whole blood aggregometer (Chronolog Corp.).

C. Results

Cloning of the Platelet-specific Variable Gene Regions

Several positive clones were isolated from the heavy and light chain libraries after screening and approximately one million plaques using the $J_H$ and $J_\kappa$ probes, respectively. Following at least three rounds of plaque purification, bacteriophage DNA was isolated for each positive clone, digested with either EcoRI (heavy chain clones) or HindIII (light chain clones) and fractionated on 1% agarose gels.

The DNA was transferred to nitrocellulose and the blots were hybridized with $J_H$ (heavy chain) or $J_\kappa$ $^{32}$P-labeled DNA probes. For the heavy chain, 2 clones were obtained that contained 3.5 kb EcoRI DNA fragments that hybridized to the $J_H$ probe. Two size classes of HindIII fragments of 3.0 and 6.0 kb were identified with the $J_\kappa$ probe.

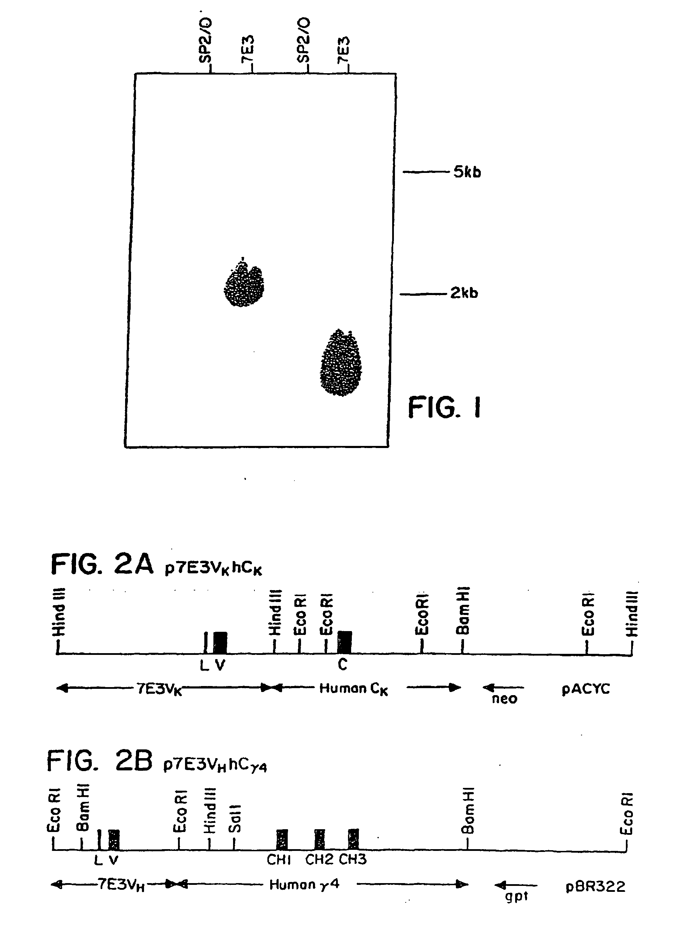

Cloned DNA corresponding to the authentic heavy and light chain variable regions from the 7E3 hybridoma should hybridize to mRNA isolated from the hybridoma. Non-functional DNA rearrangements at either the heavy or light chain loci should not be expressed. Figure 1 shows a Northern analysis that demonstrates that the 3.5 kb EcoRI putative heavy chain fragment and the 6.0 kb HindIII putative light chain fragment each hybridizes to the appropriate size mRNA from the 7E3 hybridoma. The subcloned fragments were labeled with $^{32}$P by nick translation and hybridized to northern blots containing total RNA derived from SP2/0 (the fusion partner of the 7E3 hybridoma) or from 7E3, as indicated in Figure 1. The 3.5 kb EcoRI heavy chain fragment hybridized with a 2 kb mRNA in 7E3 RNA but not in SP2/0 RNA. Similarly, the 6.0 kb light chain HindIII fragment hybridized with a 1250 bp mRNA in 7E3 RNA but not in SP2/0 RNA. These are the correct sizes for heavy and light chain mRNAs, respectively. Because the cloned DNA fragments contain sequences expressed in the 7E3 hybridoma, these data suggest that the clones contain the correct variable region sequences from the 7E3 hybridoma. The final functional test, however, is the demonstration that these sequences, when combined with appropriate constant region sequences, are capable of directing the synthesis of an antibody with a specificity and affinity similar to that of the murine 7E3 antibody.

Vectors and Expression Systems

The putative light and heavy chain V genes cloned from the 7E3 hybridoma were joined to human $\kappa$ and $G_4$ constant region genes in expression vectors described previously (Sun, L. et al., Proc. Natl. Acad. Sci. USA 84:214-218 (1987)). The 17-1A $V_\kappa$ HindIII fragment of pSV184ΔHneo17-1A$V_\kappa$h$C_\kappa$ was replaced with the 6.0 kb HindIII fragment corresponding to the putative light chain variable region gene from 7E3. Similarly, the 17-1A $V_H$ EcoRI fragment of pSV2ΔHgpt17-1A$V_H$-h$C_{G4}$ was replaced with the 3.5 kb EcoRI fragment corresponding to the putative heavy chain V region gene from 7E3. The structures of the resulting plasmids, designated p7E3$V_\kappa$h$C_{H\kappa}$ and p7E3$V_H$h$C_{G4}$, are shown in Figure 2.

To express the chimeric heavy and light chain genes, the two plasmids were cotransfected into the nonproducing mouse myeloma cell line SP2/0. The light chain plasmid confers resistance to G418 and the heavy chain plasmid confers resistance to mycophenolic acid, thus allowing a double selection to be used to obtain clones carrying and expressing genes from each plasmid. Colonies resistant to G418 and mycophenolic acid were expanded to stable cell lines and maintained in the presence of both drugs. Tissue culture supernatant from these cell lines was tested for antibody using a particle concentration fluorescence immunoassay with polystyrene beads coated with goat anti-human IgG Fc antibody and the same antibody labeled with fluorescein. Out of the first 10 lines checked, one (designated c-7E3F6) that produced approximately 2μg/ml was selected for further study.

Platelet Binding Activity Assay

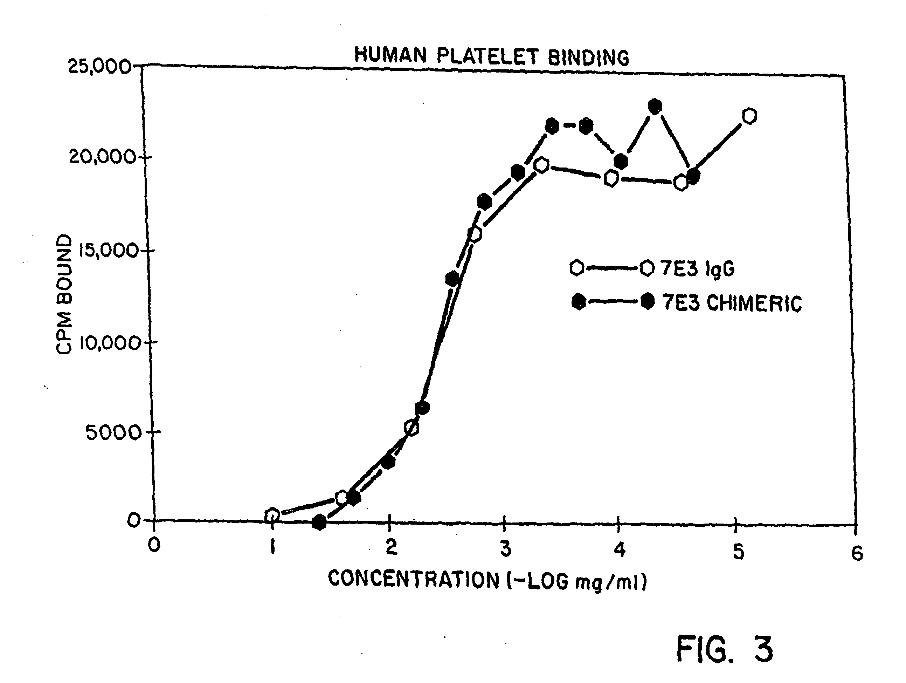

After purification of c-7E3F6 antibody using a protein A-sepharose column, the antibody was concentrated and compared to murine 7E3 IgG in the platelet binding activity assay. Figure 3 shows that murine 7E3 and c-7E3F6 (the putative chimeric antibody) compete with radiolabeled 7E3 for platelet binding to the same extent; the binding curves are superimposable indicating that the binding characteristics of murine and chimeric 7E3 are identical by this criterion.

Inhibition of Platelet Aggregation by C-7E3F6

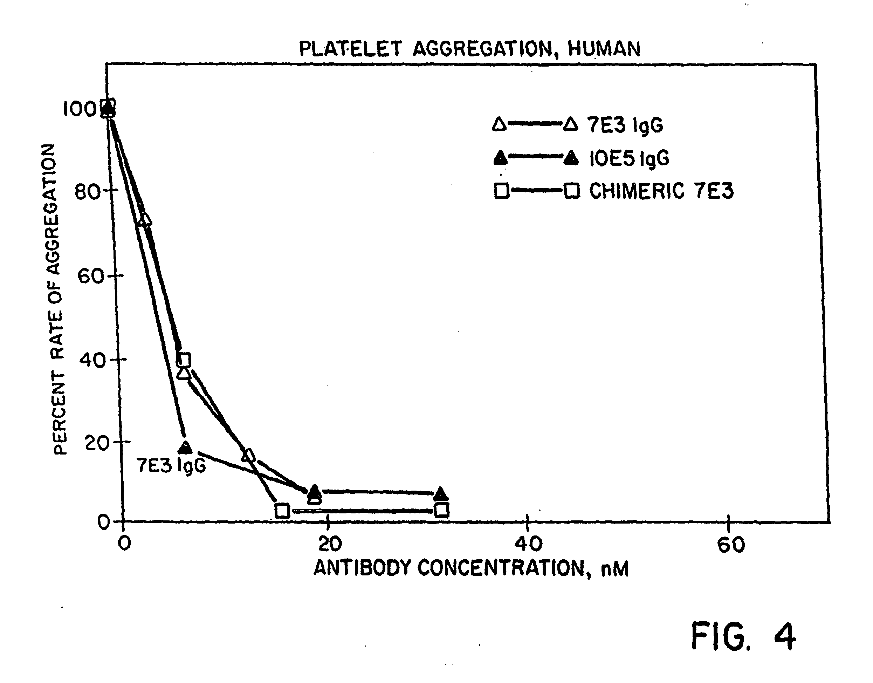

Purified c-7E3F6 was compared to murine 7E3 in a functional assay that measures the ability of the test antibody to inhibit aggregation of human platelets. The results of such an assay are shown in Figure 4 and demonstrate that both antibodies inhibit collagen-induced platelet aggregation to the same extent at the same antibody concentration. c-7E3F6 also inhibits ADP-induced platelet aggregation to a similar extent.

The results of the platelet binding assay and the inhibition of platelet aggregation assay demonstrate that: (1) the correct variable region genes were indeed cloned from the 7E3 hybridoma; and (2) the substitution of the human constant regions for the murine constant regions has no effect on the binding or functional characteristics of the 7E3 variable regions as measured by these assays.

Fibrinogen-coated Bead Assay

The chimeric c-7E3F6 antibody was found positive in a qualitative, functional assay that measures the ability of an antibody to inhibit the agglutination between platelets and fibrinogen-coated beads. Coller, B. et al. (1983) J. Clin. Invest. 73:325-338.

Example 2

Production of Chimeric IgG1 and IgG3

The DNA segment encoding the variable region of the heavy chain from the murine 7E3 antibody was linked to the human $\gamma 1$ and $\gamma 3$ constant regions present on the expression vectors pSV2ΔHgpt17-1AV$_H$-hC$_{G1}$ and pSV2ΔHgpt17-1AV$_H$-hC$_{G3}$ (Sun et al., Proc. Natl. Acad. Sci. USA 84:214-218 (1987)), by replacing the 17-1A heavy or light chain variable region fragments with the corresponding 7E3 variable region fragments. The resulting chimeric heavy chain genes were cotransfected with the chimeric light chain gene into SP2/0 cells to generate stable cell lines secreting $\gamma 1$,K, and $\gamma 3$,K antibodies.

Example 3 (for reference)

Initial Studies of Use of Chimeric 7E3 Fab in Humans

Preparation of Chimeric 7E3 Fab Fragments

The Fab fragment of chimeric 7E3 (c7E3) was produced by enzymatic digestion of purified chimeric 7E3 IgG (gamma 1 heavy chain, kappa light chain) with the proteolytic enzyme papain. The Fab fragment was isolated by a series of purification steps designed to yield a product which was free of other digestion fragments and other contaminating components (e.g., protein, nucleic acid, viruses). The final product was prepared as a sterile, non-pyrogenic solution containing 2 mg of monoclonal chimeric 7E3 Fab per ml of 0.15 M sodium chloride, 0.01 M sodium phosphate, pH7.2. In certain preparations, polysorbate 80 was included at a final concentration of 0.001% (w/v). Prior to use, the product was filtered through a 0.22 micron low protein binding filter. The product was stored at 2-8 °C.

Pharmacokinetics: Plasma Clearance of c7E3 Fab in Humans

The plasma clearance of chimeric 7E3 (c7E3) Fab fragment was studied in three patients with stable coronary disease. Following a 0.25-mg/kg dose of c7E3 Fab administered intravenously as a five minute infusion, blood samples were taken at various times from two minutes to 72 hours. It was anticipated that a certain portion of the antibody would exist in an unbound state in plasma. To quantify this unbound antibody component, it was necessary to rapidly separate the plasma from the platelets to prevent further binding ex vivo. The plasma concentration of free c7E3 Fab was measured by solid-phase enzyme immunoassay (EIA). The assay employed affinity isolated anti-murine 7E3 IgG purified from rabbit antisera for solid-phase capture and a detection system based on a biotinylated derivative of the same rabbit anti-7E3 antibody preparation. The results are presented in Table 1.

TABLE 1

| PLASMA CONCENTRATION OF c7E3 Fab IN PATIENTS TREATED WITH A 0.25-MG/KG DOSE | | | |
|---|---|---|---|
| Time* | Patient A | c7E3 Fab (μg/mL) Patient B | Patient C |
| Pre-dose | ND | ND | ND |
| 2 min | NA | 2.554 | 2.312 |
| 5 min | 1.149 | 1.873 | 1.411 |
| 10 min | 0.714 | 1.331 | 1.111 |
| 15 min | 0.610 | 0.916 | 0.852 |
| 20 min | 0.499 | 0.985 | 0.756 |
| 30 min | 0.464 | 0.815 | 0.515 |
| 45 min | 0.340 | 0.704 | 0.405 |
| 1 hr | 0.309 | 0.437 | 0.195 |
| 2 hr | 0.288 | 0.262 | 0.149 |
| 6 hr | 0.204 | 0.095 | 0.105 |
| 12 hr | 0.112 | 0.072 | 0.064 |
| 24 hr | 0.065 | 0.058 | 0.046 |
| 48 hr | 0.055 | 1.47 | 0.175 |
| 72 hr | 0.196 | ND | 0.076 |
| *Interval between end of infusion and blood drawing. Note that platelets were in contact with the plasma for an additional 2 minutes after the blood was drawn (i.e., for the time required to separate the plasma by centrifugation). ND = Not detected/below the detectable level of the assay (0.025 μg/mL). NA = Not available. | | | |

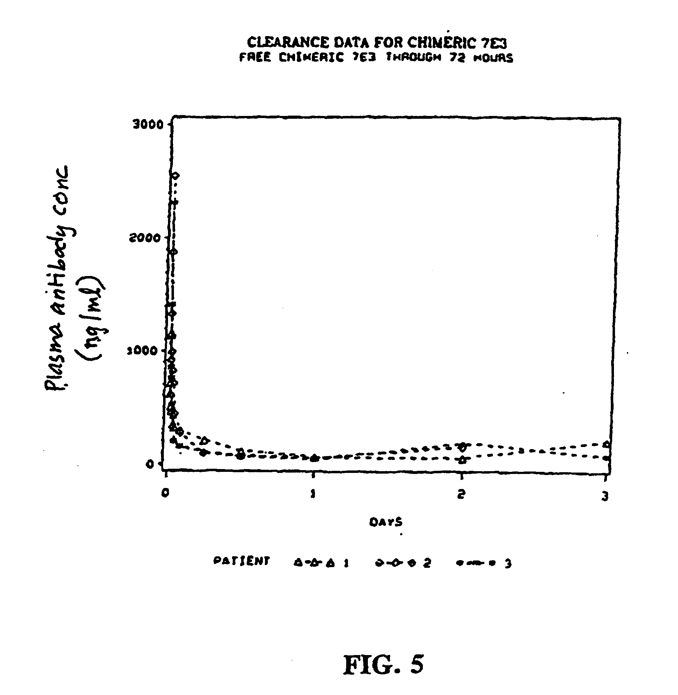

If the entire injected dose of 7E3 were detected as free antibody in plasma, the theoretical maximum antibody concentration would be approximately 6.25 μg/mL (0.25 mg/kg divided by 40mL of plasma/kg). However, this theoretical maximum concentration would never be attained because of the large component of injected antibody which binds to platelets. In fact, at the earliest measurement time (2 minutes), the average plasma concentration (n=2) of c7E3 Fab was 2.43 μg/mL; this value was the observed maximum plasma concentration ($C_{max}$). The data obtained at subsequent post-injection times show a rapid initial decrease in the plasma concentration of c7E3 Fab. By the 1-hour and 24-hour measurements, the administered antibody remaining in the plasma (n=3) was less than 0.5 μg/mL and 0.1 μg/mL, respectively. A plot of the plasma antibody concentration (ng/mL) versus time data, presented in Figure 5 graphically demonstrates the rapid initial clearance of c7E3 Fab from the plasma in all three patients.

A preliminary analysis of the pharmacokinetic characteristics of c7E3 Fab fragment was undertaken. Several models, including several mixed (random and fixed effects) linear models as well as standard two-compartmental and non-compartmental models, were used to fit the plasma concentration data. The free plasma antibody data did not adequately fit standard pharmacokinetic models. As the site of action for c7E3 Fab is a receptor located on platelets, it is not unexpected that the plasma concentration of free antibody would not be related to its concentration at its site of action in any simple way. The rapid initial clearance of c7E3 Fab from the plasma reflects, in part, the rapid antibody binding to platelet GPIIb/IIIa receptors. Of the models examined, the random effects linear model was shown to best fit the plasma concentration data. Using this model, preliminary values for the pharmacokinetic parameters, $Cl_p$, $V_d$, and $t_{1/2}$, were determined and are presented in Table 2.

TABLE 2

| PHARMACOKINETIC VALUES FOR c7E3 Fab* | |
|---|---|
| Parameter | Value |
| $Cl_p$ (proportion/hr) | 15.6 |
| $V_d$ (L) | 6.8 |

(continued)

| PHARMACOKINETIC VALUES FOR c7E3 Fab* | |
|---|---|
| Parameter | Value |
| $t_{1/2}$ (hr) | 0.1 (6 min) |
| * A random effects linear model was used to fit the data. $Cl_p$ = Plasma clearance is defined as the rate of decrease in plasma concentration divided by the concentration and is computed as a rate per hour, i.e., if the rate at a given time continued for an hour, the computed rate would be the proportion of drug removed in that hour. $V_d$ = Volume of distribution is defined as the dose administered divided by the measured plasma concentration multiplied by the plasma volume. A 3-L plasma volume typical for a 70-kg person was used in the calculations. $t_{1/2}$ = Elimination half-life. | |

Urinary Excretion in Humans

Urine samples were collected from three patients with stable coronary disease who were treated intravenously with 0.25-mg/kg of c7E3 Fab (plasma clearance data for these same three patients are discussed above). Total urine output was collected for the following post-injection time periods: 0 to 2 hours, 2 to 6 hours, 6 to 12 hours, and 12 to 24 hours. In addition, a sample of predose urine was also collected. Representative samples of the collected urine samples were analyzed for free 7E3 Fab using a slight modification of the EIA described above. In all cases, no c7E3 Fab was detected in the urine.

Preclinical Toxicology

Preclinical toxicology studies have been performed in 18 monkeys (Cyonomolgus and Rhesus), using chimeric 7E3 Fab. Bolus doses of up to 0.6 mg/kg, followed by infusion of up to 0.8 $\mu$g/kg/min for 96 hours were administered (includes studies with heparin, aspirin and recombinant tissue plasminogen activator). In all monkeys, at all doses, in all combinations, 7E3 was safe and well-tolerated, with no significant bleeding complications or other adverse events.

Dose Escalation of Chimeric 7E3 Fab in Stable Angina Patients

A dose escalation study was conducted enrolling 52 stable angina patients (males from 43 to 75 years old) who were off anti-platelet therapy for more than 10 days. A variety of dosing regimens were administered. Patients received either single intravenous bolus injections of 0.15 to 0.30 mg/kg of chimeric 7E3 Fab (20 patients) or a bolus loading followed by continuous intravenous infusions (10 $\mu$g/minute) from 12 to 96 hours in duration (32 patients).

Platelet GPIIb/IIIa receptor blockade, platelet aggregation in response to 20 $\mu$M ADP (agonist), and bleeding times were determined 2 hours after administration of a bolus dose of c7E3 Fab (0.15-0.30 mg/kg). Receptor blockade and platelet aggregation in response to agonist were determined essentially as described (Gold, H.K. et al., J. Clin. Invest. 86:651-659 (1990)). Bleeding times were determined by the Simplate method. With increasing doses there was a progressive increase in receptor blockade, as indicated by the percent of receptors blocked (determined from the availability of receptor binding sites). The increase in receptor blockade was paralleled by inhibition of platelet aggregation (measured as a percent of the pre-dose value or baseline), and by an increase in bleeding time.

The peak effect in terms of all three parameters was observed at 0.25 mg/kg. This dose corresponds to a plasma concentration of 5 $\mu$g/ml--the concentration at which peak inhibition was seen in a platelet-rich plasma from a normal subject which had been incubated for 15 minutes in an aggregometer cuvette in the presence of increasing concentrations of chimeric 7E3 Fab. (The extent of aggregation of the plasma of the normal subject was measured by the percent of light transmitted through the cuvette. Prior to the addition of an agonist, the plasma was relatively opaque and the percent of light transmitted was set at zero. When the agonist ADP was added to a control sample without antibody, the light transmission progressively increased as aggregation progressed. However, when c7E3 Fab is present, a dose-dependent block of aggregation was observed with complete inhibition at 5 $\mu$g/ml c7E3 Fab.)

The duration of action in terms of receptor blockade, inhibition of platelet aggregation, and bleeding time was determined. Peak effects on receptor blockade, platelet aggregation, and bleeding time were seen at 2 hours, with gradual recovery over time. Bleeding times returned to near normal values by 6-12 hours.

Because peak receptor blockade and functional inhibition were achieved with 0.25 mg/kg, the duration of platelet inhibition by continuous infusions following this loading dose were assayed to determine if the duration of platelet inhibition could be prolonged. The degree of receptor blockade, inhibition of platelet aggregation, and prolongation of bleeding time were maintained for the duration of continuous infusion in five patients who received a 10 μg/minute continuous infusion of chimeric 7E3 Fab for 72 hours following the 0.25 mg/kg loading dose. Recovery started as soon as the infusion was discontinued. Similar results were seen with 12, 24, 48 and 96 hour infusions.

None of the patients experienced a hypersensitivity reaction. There were no significant treatment related trends in hematology or chemistry laboratory values. Nor were there any major bleeding events. Insignificant bleeding events were rare and included transient mild nose bleed and mild gum oozing in patients with periodontal disease. The results of the trial indicated that chimeric 7E3 Fab can be administered to patients using dosing regimens that produce profound inhibition of platelet function for periods as long as several days.

Immunogenicity Results

In trials with murine 7E3 F(ab')$_2$ and Fab (150 patients), immune responses detected using a sensitive enzyme-linked immunoassay system occurred in 16% (24/150) of the patients. All reactions were of low titer, typically in the range of 1:50 to 1:200. The treatment group included normal volunteers treated with 0.01 - 0.25 mg/kg murine 7E3 F(ab')$_2$, unstable angina patients treated with 0.05 - 0.20 mg/kg murine 7E3 F(ab')$_2$, and PTCA patients treated with 0.1 mg/kg murine 7E3 F(ab')$_2$, or 0.15 - 0.35 mg/kg murine Fab, as well as stable angina patients treated with a single bolus intravenous injection of 0.10 - 0.30 mg/kg of murine 7E3 Fab, a single bolus dose of either 0.25 or 0.30 mg/kg followed by continuous infusion for 12-36 hours (0.15 μg/kg/min or 10μg/min) of murine Fab, or with two injections of murine Fab separated by six hours (a single bolus of 0.2 mg/kg - 0.30 mg/kg followed by a bolus of 0.05 mg/kg).

Immunogenicity was notably reduced with the human-mouse chimeric 7E3 Fab. None of the 52 patients having stable angina enrolled in the dose escalation study and treated with chimeric 7E3 Fab (see above) showed an immune response to treatment as measured by a similar assay adapted to the chimeric Fab.

Reversibility of Anti-platelet Activity

Chimeric 7E3 Fab ($\gamma_1$, $\kappa$) has a slow off rate from platelets and free plasma chimeric 7E3 Fab clears from circulation rapidly (see above). Thus, the antiplatelet effects of chimeric 7E3 are readily reversible by administration of random donor platelets. This reversal or antidote effect by transfusion of platelets has been demonstrated in 2 patients who had received either murine Fab or chimeric Fab and who received random donor platelets during a time when they had nearly complete inhibition of platlet aggregation. Restoration of platelet function was determined by measuring bleeding times. This property is useful in situtations where a bleeding event necessitates restoration of platelet function in a patient.

Example 4 (for reference)

Use of Chimeric 7E3 Antibody in the Prevention of Thrombotic Complications of Elective Coronary Angioplasty

Percutaneous transluminal coronary angioplasty (PTCA), by balloon or coronary atherectomy, for example, is an effective method of enlarging the lumen of stenosed coronary arteries. In this procedure, there is an inherent risk of acute coronary occulusion during and after angioplasty. The reported rate of coronary occlusion varies from approximately 3%-6% of elective angioplasty cases (Detre, K.M. et al., Circulation 82:739-750 (1991)), and is the major cause of in-hospital morbidity and mortality. In high risk patients, the incidence of major cardiac events caused by thrombosis is between 10-20%.

Acute coronary occlusion during or immediately after coronary angioplasty appears to be caused by the combination of deep arterial wall injury with resultant partially occlusive "intimal flaps" with or without superimposed thrombus formation, or thrombus formation alone at a site of vessel wall injury. In animal models, reocclusion after successful thrombolysis is preceded by periods of cyclical reductions and restorations in coronary blood flow termed "cyclic flow variations" (CFVs). These CFVs are almost entirely a platelet-mediated phenomenon, and are due to repetitive accumulation and dislodgement of platelet aggregates at sites of coronary stenosis and endothelial injury. Cyclic flow variations after coronary angioplasty have been described in humans. Chimeric 7E3 antibody can be used to inhibit platelet function during angioplasty thereby preventing platelet aggregation and thrombosis. Chimeric 7E3 antibody is particularly useful in patients at high risk of thrombotic occlusion. These patients can be identified on the basis of anatomic (e.g., angiographically defined characteristics of a lesion at a site of stenosis) or clinical risk factors (e.g., myocardial infarction, unstable angina, diabetes, women 65 years or older), which predispose to acute coronary thrombosis and produce the clinical syndromes of acute myocardial infarction, unstable angina or abrupt closure.

Chimeric Anti-platelet Antibody in Elective PTCA

The trial was conducted in two stages. The primary objective of the first stage was to determine the safety and optimal dose of single dose chimeric 7E3 Fab in patients undergoing elective percutaneous transluminal coronary angioplasy (PTCA). Stage II was conducted to evaluate the safety and preliminary efficacy of chimeric 7E3 (c7E3) when administered by bolus infusion followed by various continuous infusion durations. The Stage II study comprised elective coronary angioplasty patients who were at risk for ischemic cardiac complications. High risk patients included those with unstable angina or stable coronary disease with Type B or C lesion specific characteristics. Table 3 lists the definitional criteria for high risk patients, and Table 4 lists the angiographically defined lesion-specific characteristics. Preliminary efficacy was measured as inhibition of platelet function and prevention of thrombotic complications. Men, between 18 and 76 years of age, and women not of child bearing potential, between 18 and 76 years of age, were eligible to enroll in both stages of the trial.

TABLE 3

| ENROLLMENT CRITERIA FOR PATIENTS AT HIGH RISK FOR ISCHEMIC COMPLICATIONS | | |
|---|---|---|
| Moderately high risk: | | |
| | 1) | Unstable angina with no lesion-specific characteristic defined. |
| | 2) | A stenosis with a single Type B lesion-specific characteristic. |
| Highest risk: | | |
| | 1) | A stenosis with ≥ two Type B lesion-specific characteristics. |
| | 2) | Unstable angina with a stenosis with at least one Type B lesion-specific characteristic. |
| | 3) | Diabetes mellitus with a stenosis with at least one Type B lesion-specific characteristic. |
| | 4) | Women ≥ 65 years of age with a stenosis with at least one Type B lesion-specific characteristic. |
| | 5) | A stenosis with at least one Type C lesion-specific characteristic. |

TABLE 4

| LESION-SPECIFIC CHARACTERISTICS | |
|---|---|
| Type A Lesions (high success, > 85%; low risk) | |
| -Discrete (< 10 mm length)<br>-Concentric<br>-Readily accessible<br>-Nonangulated segment, <45°<br>-Smooth contour | -Little or no calcification<br>-Less than totally occlusive<br>-Not ostial in location<br>-No major branch involvement<br>-Absence of thrombus |
| Type B Lesions (moderate success, 60-85%; moderate risk) | |
| -Tubular (10- 20 mm length)<br>-Eccentric<br>-Moderate tortuosity of proximal segment<br>-Moderately angulated segment, >45°, <90°<br>-Irregular contour | -Moderate to heavy calcification<br>-Total occlusions <3 months old<br>-Ostial in location<br>-Bifurcation lesions requiring double guide wires<br>-Some thrombus present |

(continued)

| Type C Lesions (low success, < 60%; high risk) | |
|---|---|
| -Diffuse | -Total occlusion > 3 months old |
| -Excessive tortuosity of proximal segment | -Inability to protect major side branches |
| -Extremely angulated segments > 90° | -Degenerated vein grafts with friable lesions |

Stage I

In Stage I, patients were enrolled in groups receiving a single bolus intravenous injection of chimeric 7E3 ($\gamma_1$, $\kappa$) Fab fragment (prepared and formulated as described in Example 3). A total of 15 patients, (3 women and 12 men) were treated. The median age of patients was 62 years (range 46 years to 76 years). A demographic profile is listed in Tables 5A and 5B for all single dose patients and for patients within the individual dose groups.

Five patients (n = 5) each received single doses of 0.15 mg/kg, 0.20 mg/kg or 0.25 mg/kg of c7E3 Fab within about 30 minutes prior to elective PTCA in a dose-escalation protocol. All patients were treated with aspirin (standard dose) and fully anticoagulated with heparin (standard dose) at the time of the procedure.

Although the PTCA procedures were classified as elective for Stage I patients, six of the 15 patients had unstable rest angina. The coronary location of the dilatations is summarized in Table 6 (bottom). Seven of the 15 Stage I patients underwent PTCA of one lesion in a single vessel, 6 underwent multi-lesion PTCA in a single vessel, and 2 patients had multi-vessel PTCA performed (Table 6).

The efficacy criteria for obtaining the optimal single dose of c7E3 were prospectively defined as the minimum dose that achieved median values of the following at 2 hours post-infusion: (1) prolongation of bleeding time of at least 20 minutes; (2) blockade of GPIIb/IIIa receptors such that there were greater than 80% of baseline receptor sites blocked; and (3) an inhibition of platelet aggregation in response to 20 $\mu$M ADP to $\leq$ 20% of baseline.

TABLE 5A
CHIMERIC 7E3 ANTI-PLATELET ANTIBODY
Patient Classification of Age, Weight, Height, Sex and Race

| | | Continuous | Control | Single Dose |
|---|---|---|---|---|
| TOTAL PATIENTS | | 32 | 9 | 15 |
| AGE | | | | |
| | Mean | 57.4 | 54.2 | 60.1 |
| | Median | 57.0 | 56.0 | 62.0 |
| | Minimum | 38 | 37 | 46 |
| | Maximum | 76 | 74 | 76 |
| | Std. Dev. | 9.7 | 10.5 | 9.7 |
| WEIGHT (kg) | | | | |
| | Mean | 82.8 | 88.2 | 85.5 |
| | Median | 84.8 | 84.2 | 84.0 |
| | Minimum | 42.3 | 67.3 | 70.5 |
| | Maximum | 113.0 | 122.7 | 107.0 |
| | Std. Dev. | 16.6 | 19.0 | 11.1 |
| HEIGHT (cm) | | | | |
| | Mean | 171.2 | 176.7 | 173.6 |
| | Median | 172.8 | 177.8 | 175.2 |
| | Minimum | 152.4 | 157.5 | 160.0 |

(continued)

HEIGHT (cm)

| | | | | |
|---|---|---|---|---|
| Maximum | 185.0 | 185.4 | 188.0 | |
| Std. Dev. | 7.9 | 8.8 | 7.8 | |

SEX

| | | | | |
|---|---|---|---|---|
| Female | 8 | 1 | 3 | |
| Male | 24 | 8 | 12 | |

RACE

| | | | | |
|---|---|---|---|---|
| | 0 | 0 | 1 | |
| White | 26 | 8 | 11 | |
| Black | 5 | 1 | 2 | |
| Asian | 0 | 0 | 1 | |
| Hispanic | 1 | 0 | 0 | |

TABLE 5B

CHIMERIC 7E3 ANTI-PLATELET ANTIBODY

Patient Classification of Age. Weight. Height. Sex and Race

| | | 0.15 mg/kg 7E3 | 0.20 mg/kg 7E3 | 0.25 mg/kg 7E3 | 0.25 mg/kg 7E3* 10 mcg/min for 6 hrs | 0.25 mg/kg 7E3* 10 mcg/mg for 12 hrs | 0.25 mg/kg 7E3* 10 mcg/min for 24 hrs | Placebo |
|---|---|---|---|---|---|---|---|---|
| TOTAL PATIENTS | | 5 | 5 | 5 | 11 | 11 | 10 | 9 |
| AGE | | | | | | | | |
| | Mean | 60.6 | 57.2 | 62.6 | 59.1 | 55.0 | 58.1 | 54.2 |
| | Median | 63.0 | 56.0 | 65.0 | 60.0 | 53.0 | 58.5 | 58.0 |
| | Minimum | 47 | 46 | 51 | 40 | 42 | 38 | 37 |
| | Maximum | 73 | 68 | 76 | 76 | 73 | 75 | 74 |
| | Std. Dev. | 10.5 | 8.3 | 11.3 | 10.5 | 8.9 | 10.3 | 10.5 |
| WEIGHT (kg) | | | | | | | | |
| | Mean | 83.8 | 80.5 | 92.2 | 85.8 | 83.8 | 78.3 | 88.2 |
| | Median | 76.3 | 78.0 | 96.0 | 89.0 | 85.0 | 79.9 | 84.2 |
| | Minimu | 74.5 | 70.5 | 82.0 | 60.4 | 42.3 | 62.3 | 67.3 |
| | Maximum | 107.0 | 95.0 | 99.1 | 113.0 | 111.8 | 95.0 | 122.7 |
| | Std. Dev. | 13.8 | 9.5 | 7.7 | 17.8 | 19.6 | 10.7 | 19.0 |
| HEIGHT (cm) | | | | | | | | |
| | Mean | 170.8 | 171.9 | 178.1 | 169.3 | 170.9 | 173.7 | 176.7 |
| | Median | 173.0 | 175.2 | 177.8 | 167.6 | 172.7 | 175.2 | 177.8 |
| | Minimum | 160.0 | 160.8 | 169.0 | 157.5 | 152.4 | 165.1 | 157.5 |
| | Maximum | 177.8 | 182.9 | 188.0 | 177.8 | 185.0 | 180.3 | 185.4 |
| | Std. Dev. | 7.0 | 8.8 | 7.0 | 6.4 | 10.8 | 5.4 | 8.8 |
| SEX | | | | | | | | |
| | Female | 3 | 0 | 0 | 3 | 4 | 1 | 1 |

(continued)

| SEX | | | | | | | | |
|---|---|---|---|---|---|---|---|---|
| Male | | 2 | 5 | 5 | 8 | 7 | 9 | 8 |
| RACE | | | | | | | | |
| | | 0 | 0 | 1 | 0 | 0 | 0 | 0 |
| | White | 3 | 4 | 4 | 8 | 9 | 9 | 8 |
| | Black | 2 | 0 | 0 | 3 | 2 | 0 | 1 |
| | Asian | 0 | 1 | 0 | 0 | 0 | 0 | 0 |
| | Hispanic | 0 | 0 | 0 | 0 | 0 | 1 | 0 |

*In patients with multivessel disease, both vessels are counted.

TABLE 6

ANGIOGRAPHIC CHARACTERISTICS

| Number of Lesions Attempted: | Control | c7E3 Fab Stage I | c7E3 Fab Stage II |
|---|---|---|---|
| Single vessel/single lesion | 8 | 7 | 21 |
| Single vessel/multilesion | 0 | 6 | 7 |
| Multivessel/single lesion | 1 | 2 | 1 |
| Multivessel/multilesion | 0 | 0 | 2 |
| Unknown | 0 | 0 | 1 |
| | | | |
| Location of Attempted Lesions: | | | |
| RCA | 3 | 9 | 13 |
| LCX | 4 | 3 | 14 |
| LAD | 3 | 9 | 17 |

RCA = Right coronary artery
LCX = Left circumflex coronary artery
LAD = Left anterior descending coronary artery

Stage II

In Stage II, patients were treated with a 0.25 mg/kg bolus dose followed by a continuous infusion of 10 μg/min of c7E3 Fab for 6, 12, or 24 hours. A total of 32 patients (8 women and 24 men) were entered into the treatment group of Stage II of the study. The median age of the c7E3 Fab-treated patients was 57 years (range 38-76). Nine control patients (1 woman, 8 men) were entered. The median age of control patients was 56 years (range 37-74). Control patients were high risk patients as defined above, who did not receive c7E3, but were monitored and followed in the same fashion as treated patients. A demographic profile for all Stage II patients and for patients within the individual dose groups is listed in Tables 5A and 5B.

Treatment with c7E3 Fab was initiated 30 minutes prior to balloon inflation for PTCA. Aspirin and heparin were given as clinically indicated, with the recommendation that following angioplasty heparin be given at the rate of 800 units per hour. Eleven patients each were entered into the 6 and 12 hour groups, and ten patients were entered into the 24 hour group.

Of the 32 c7E3 Fab-treated patients, 21 patients underwent PTCA of one lesion in a single vessel, 7 patients underwent multi-lesion PTCA in a single vessel, and 3 patients had multi-vessel PTCA performed (Table 6). The type of PTCA was not specified in one patient. The coronary location of the dilatations for stage II patients is summarized in Table 6. of the 9 control patients, 8 underwent PTCA of one lesion in a single vessel, and one patient had multi-vessel PTCA. The 32 c7E3 Fab-treated patients and the 9 control patients had clinical or angiographic characteristics that would classify them as high risk for ischemic cardiac complications of PTCA. Two c7E3 Fab-treated patients and one control patient had unspecified risk factors. The remaining 30 c7E3 Fab-treated patients and 8 control patients had at least one identifiable clinical feature or angiographic characteristic placing them at increased risk of ischemic complication, and most had more than one risk factor. Table 7 summarizes these risk factors for the control and c7E3 treatment groups, and individual listings of risk factors by patient within each dose group are presented in Tables 8A through 8D.

TABLE 7

| HIGH RISK CHARACTERISTICS | | |
|---|---|---|
| Stage II | | |
| **Risk Factors** | **Control (n = 9)** | **c7E3 Treatment (n= 32)** |
| One Type B characteristic | 5[1] | 4 |
| Two or more Type B characteristics | 1[2] | 7[3] |
| One Type C characteristic | 0 | 2 |
| Unstable angina with no lesion characteristics identified | 1 | 1[4] |
| Unstable angina + Type B characteristic | 0 | 8[5] |
| Unstable angina + $\geq 2$ Type B characteristics | 1 | 7[6] |
| Unstable angina + Type C characteristic | 0 | 1 |
| Unspecified risk characteristic | 1 | 2 |
| 1 Patient 04-006 had diabetes<br>2 This patient (04-007) had diabetes<br>3 Patients 03-001 and 02-007 had diabetes<br>4 This patient (04-004) had the following additional risk factors: female, age > 65, and diabetes<br>5 Patients 03-003 and 05-001 had diabetes<br>6 Patient 01-018 had the following additional risk factors: female and age > 65. Patient 03-002 had diabetes. | | |

TABLE 8A

| PREDISPOSING HIGH RISK CHARACTERISTICS | | |
|---|---|---|
| Control Patients | | |
| Patient Number | Type of Risk(s)[1] | Segment[2] |
| 01-023 | 1. Unstable angina | LAD |
| 01-024 | 1. One Type B charasteristic[3] | LAD |
| 04-006 | 1. Diabetes<br>2. One Type B characteristic (eccentric) | LCX |
| 04-007 | 1. Diabetes<br>2. Two Type B characteristics (eccentric; moderately angulated segment; > 45°, <90°) | RCA |
| 04-008 | 1. One Type B characteristic (eccentric) | LCX |
| 04-009 | 1. Our Type B characteristic (eccentric) | LCX |
| 01-021 | 1. One Type B characteristic[3] | RCA |
| 01-022 | 1. Unstable angina<br>2. Two Type B characteristics (tubular; irregular contour) | OM |
| 03-005 | 1. Unspecified risk characteristic | RCA |
| 1 Potential characteristics listed in Tables 3 and 4<br>2 RCA = right coronary artery<br>LCX = left circumflex coronary artery<br>LAD = left anterior descending coronary artery<br>OM = obtuse marginal branches of LCX<br>3 Characteristic not designated | | |

TABLE 8B
PREDISPOSING HIGH RISK CHARACTERISTICS
6-Hour Continuous Infusions

| Patient Number | Type of Risk(s)[1] | | Segment[2] |
|---|---|---|---|
| 04-001 | 1. | Unstable angina | RCA |
| | 2. | One Type B characteristic (eccentric) | |
| 06-001 | 1. | Unstable angina | LCX |
| | 2. | One Type B charasteristic (thrombus) | |
| 06-002 | 1. | One Type B characteristic (tubular [10 to 20mm lesion]) | LAD |
| 01-014 | 1. | Unstable angina | LAD |
| | 2. | One Type C characteristic (Diffuse > 2 cm length) | |
| 01-013 | 1. | Unstable angina | RCA |
| | 2. | Two Type B characteristics (eccentric, some thrombus) | |
| 01-015 | 1. | Two or more Type B characteristics[3] | LAD, LADD |
| 01-017 | 1. | One Type B characteristic (eccentric) | LCX |
| 02-005 | 1. | Unstable angina | LAD |
| | 2. | Three Type B characteristics (tubular, (10 to 20mm length); irregular contour; ostial in location) | |
| 03-001 | 1. | Diabetes | LAD |
| | 2. | Four Type B characteristics (eccentric; moderate angulation, > 45°, <90°; irregular contour; moderate to heavy calcification) | |
| 01-012 | 1. | One Type C characteristic (Diffuse >2 cm length) | LAD |
| 01-016 | 1. | One Type B characteristic | RCA |

(continued)
PREDISPOSING HIGH RISK CHARACTERISTICS
6-Hour Continuous Infusions

| Patient Number | Type of Risk(s)[1] | Segment[2] |
|---|---|---|
| | (some thrombus) | $OM_n$ |

[1] Potential characteristics listed in Tables 3 and 4
[2] RCA = right coronary artery
LCX = left circumflex coronary artery
LAD = left anterior descending coronary artery
LADD = diagonal branch of LAD
$OM_n$ = obtuse marginal branches of LCX
[3] Characteristic not designated

TABLE 8C
PREDISPOSING HIGH RISK CHARACTERISTICS
12-Hour Continuous Infusion

| Patient Number | Type of Risk(s)[1] | | Segment[2] |
|---|---|---|---|
| 01-018 | 1. | Unstable angina | $OM_n$ |
| | 2. | Female over 65 | |
| | 3. | Two or more Type B characteristics | |
| 01-019 | 1. | Unstable angina | Circumflex |
| | 2. | Two or more Type B characteristics | |
| 02-006 | 1. | Unstable angina | RCA |
| | 2. | One Type B characteristic (total occlusion <3 months old) | |
| 02-007 | 1. | Diabetes | LCX |
| | 2. | Five Type B characteristics (eccentric, moderate tortuosity of proximal segment; moderately angulated segment, >45°, <90°; bifurcation lesions requiring double guidewires; total occlusions <3 mo) | $OM_1$, LADD |
| 03-002 | 1. | Unstable angina | LAD |
| | 2. | Diabetes | |
| | 3. | Two Type B characteristics (moderate tortuosity segment; irregular contour) | |
| 03-003 | 1. | Unstable angina | RCA |
| | 2. | Diabetes | |
| | 3. | One Type B characteristic | |

(continued)
PREDISPOSING HIGH RISK CHARACTERISTICS
12-Hour Continuous Infusion

| Patient Number | Type of Risk(s)[1] | | Segment[2] |
|---|---|---|---|
| | (irregular contour) | | |
| 05-001 | 1. | Unstable angina | LADD |
| | 2. | Diabetes | RCA |
| | 3. | One Type B characteristic[3] (eccentric) | |
| 05-002 | 1. | One Type B characteristic (tubular) | RCA |
| | 2. | One Type C characteristic (total occlusion >3 months) | LADD |
| 05-003 | 1. | Two Type B characteristics (moderately angulated segment, >45°, <90°; some thrombus) | LAD |
| 06-003 | 1. | Unstable angina | RCA |
| | 2. | One Type B characteristic (some thrombus) | |
| 04-002 | 1. | Unstable angina | LCX |
| | 2. | One Type B characteristic (tubular 10 to 20 mm) | |

[1] Potential characteristics listed in Tables 3 and 4
[2] RCA = right coronary artery
LCX - left circumflex coronary artery
LAD = left anterior descending coronary artery
LADD = diagonal branch of LAD
OM$_n$ = obtuse marginal branches of LCX
[2] RCA = right coronary artery LCX = left circumflex coronary artery LAD = left anterior descending coronary artery
[3] OM = obtuse marginal branches of LCX

TABLE 8D
PREDISPOSING HIGH-RISK CHARACTERISTICS
24-Hour Continuous Infusion

| Patient Number | Type of Risk(s)[1] | | | Segment[2] |
|---|---|---|---|---|
| 01-020 | 1. | Two Type B characteristics (irregular contour, some thrombus) | | RCA |
| 02-008 | 1. | Unstable angina | | |
| | 2. | Type B characteristics | | |
| | | a) | 4 characteristics (tubular; eccentric; moderate tortuosity | LCX |

(continued)
PREDISPOSING HIGH-RISK CHARACTERISTICS
24-Hour Continuous Infusion

| Patient Number | Type of Risk(s)[1] | | | Segment[2] |
|---|---|---|---|---|
| | | | of proximal segment; irregular contour) | |
| | b) | | 3 characteristics (eccentric; moderately angulated segment >45°, <90°) | LCX |
| 05-005 | 1. | Unstable angina | | RCA |
| | 2. | One Type B characteristic[3] | | |
| 05-006 | 1. | Unstable angina | | RCA |
| | 2. | One Type C characteristic (diffuse (>2 cm length)) | | |
| 04-003 | 1. | Two Type B characteristics (eccentric; bifurcation with double guidewires) | | LAD |
| 04-004 | 1. | Unstable angina | | LAD |
| | 2. | Diabetes | | |
| | 3. | Female over 65 | | |
| 04-005 | 1. | Two Type B characteristics (eccentric, some thrombus) | | RCA |
| 02-009 | 1. | Unstable angina | | |
| | 2. | Type B characteristics | | |
| | a) | | 3 characteristics (irregular contour, some thrombus; bifurcation lesions requiring double guidewires) | LAD |
| | b) | | 1 characteristic (bifurcation lesions requiring double guidewires) | LAD |
| | c) | | 2 characteristics (eccentric; bifurcation lesions requiring double guidewires) | DB |
| 05-004 | 1. | One Type B characteristic (bifurcation requiring double | | OM$_1$ |

22

(continued)

PREDISPOSING HIGH-RISK CHARACTERISTICS

24-Hour Continuous Infusion

| Patient Number | Type of Risk(s)[1] | Segment[2] |
|---|---|---|
| | guidewires) | OM$_2$ |

[1] Potential characteristics listed in Tables 3 and 4

[2] RCA = right coronary artery LCX = left circumflex coronary artery LAD = left anterior descending coronary artery LADD = diagonal branch of LAD

OM$_n$ = obtuse marginal branches of LCX

[3] Characteristic not designated

Inhibition of Platelet Function (Stage I Results)

To assess activity of the chimeric 7E3 Fab in inhibiting platelet function, GPIIb/IIIa receptor binding site availability (recorded as median percent GPIIb/IIIa blocked), median inhibition of agonist-induced platelet aggregation in response to 20 $\mu$M ADP, and median bleeding times, were serially measured. Receptor blockade and platelet aggregation in response to agonist were determined essentially as described (Gold, H.K. et al., J. Clin. Invest., 86:651-659 (1990)). For receptor blockade measurements, receptor availability was measured at time 0 and the number of receptors available were taken as 0% receptors blocked (baseline). Other time points are relative to the number of receptors available at baseline or the pre-dose measurement. Bleeding times were determined by the Simplate method.

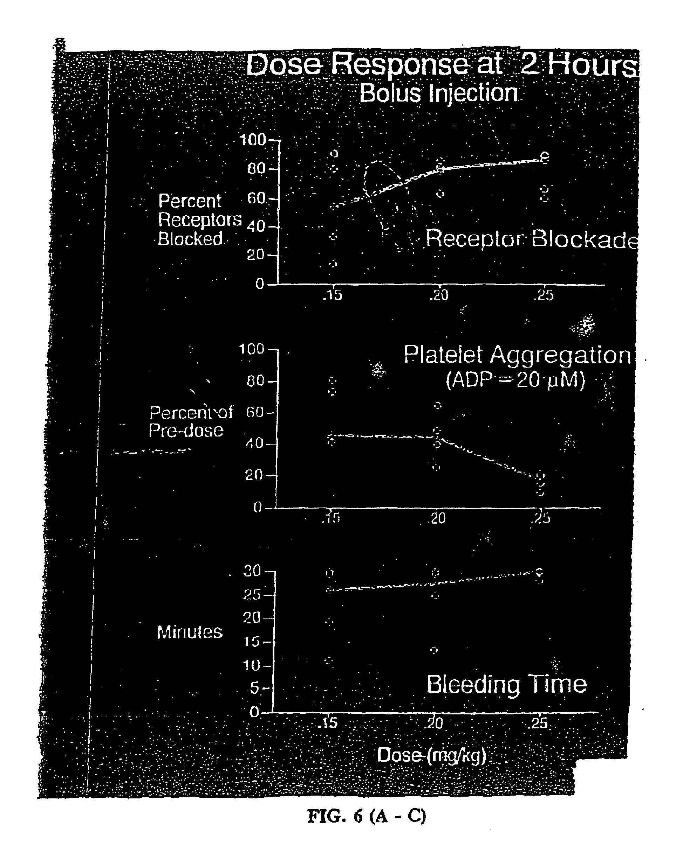

Figure 6 summarizes the dose response 2 hours following a single bolus dose of chimeric 7E3 Fab, in terms of receptor blockade (Figure 6A), platelet aggregation (Figure 6B), and bleeding time (Figure 6C). The solid lines in Figure 6 indicate the median values of the 5 patients studied at each dose group. With increasing doses of c7E3 Fab there was a progressive increase in receptor blockade as shown in percent of receptors that are blocked (Figure 6A). The median number of receptors blocked at two hours was 53.8% for the 0.15 mg/kg, 80.2% for the 0.20 mg/kg, and 86.6% for the 0.25 mg/kg dose groups. The increase in receptor blockade was paralleled by inhibition of platelet aggregation, depicted as a percent of the pre-dose value (Figure 6B). Median platelet aggregation at 2 hours was 46.1%, 44.6%, and 17.9% of baseline for the 0.15 mg/kg, 0.20 mg/kg, and 0.25 mg/kg dose groups, respectively. Likewise, a dose-related prolongation of bleeding time was seen at 2 hours post-infusion (bleeding time measurements were truncated at 30 minutes; Figure 6C). The median bleeding times were 26.0 minutes, 27.5 minutes, and 30 minutes for the 0.15 mg/kg, 0.20 mg/kg, and 0.25 mg/kg doses, respectively. Under the conditions used, and as measured by these assays, the optimal dose for anti-platelet activity was determined to be 0.25 mg/kg.

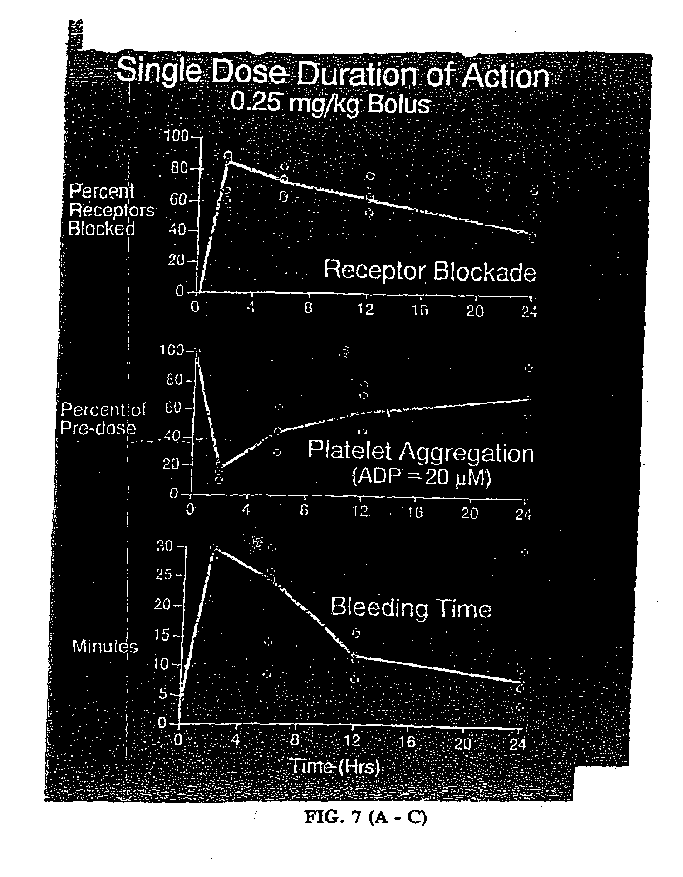

Figure 7 shows the duration of action following a single bolus dose of 0.25 mg/kg, the dose at which maximum platelet effects were seen. The lines indicate the median values from time zero (baseline) through 24 hours, as shown on the x-axis, in terms of receptor blockade in the top panel (Figure 7A), platelet aggregation in the middle panel (Figure 7B), and bleeding time in the bottom panel (Figure 7C). Peak effects on recepor blockade, platelet aggregation, and bleeding time are seen at 2 hours, with gradual recovery over time. Bleeding times return to near normal values by 12 hours. None of the patients experienced thrombocytopenia.

Inhibition of Platelet Function (Stage II Results)

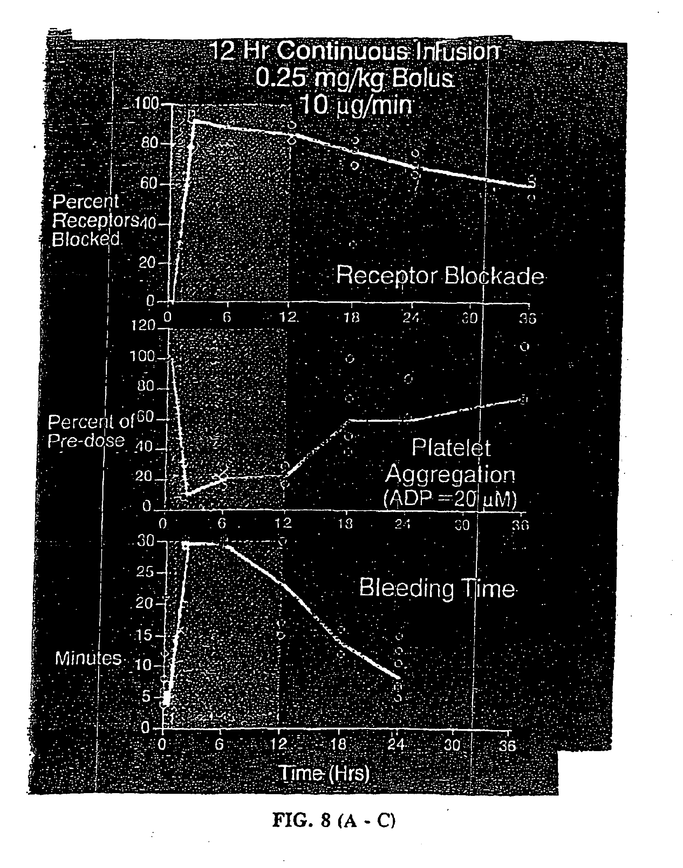

In Stage II, GPIIb/IIIa receptor and platelet aggregation data were not obtained in all patients, and only two patients in the 24 hour infusion had these studies performed. Therefore, only the 6 and 12 hour data are summarized. In both the 6-hour and 12-hour infusion groups, median receptor blockade was maintained to greater than 80% of baseline through the duration of infusion. Median 20 $\mu$M ADP-induced platelet aggregation for the 6 and 12-hour infusion groups was 13% and 15% of baseline at 2 hours, respectively, and in the 12 hour group remained below 25% for the duration of the infusion. The 2-hour median bleeding time for all three infusion durations was greater than 30 minutes. Figure 8 reviews the results observed in patients who received a 10 $\mu$g/minute continuous infusion of chimeric 7E3 Fab for 12 hours following the 0.25 mg/kg loading dose. The 12-hour infusion period is shown in the lightly shaded areas, and the lines represent the median values. The degree of receptor blockade, inhibition of platelet aggregation, and prolongation of bleeding time are maintained for the entire duration of the infusion, with recovery starting as soon as the infusion is discontinued.

TABLE 9

| MEDIAN DATA FROM FIGURE 8 | | | |
|---|---|---|---|
| 0.25 mg/kg + 10 $\mu$g/min for 12 hours | | | |
| Time following | Median | Median | Median |
| On-set of infusion Hours | Bleeding Time Minutes | Aggregation % Baseline | Binding % Baseline |
| 0 | 5.5 | 100 | 0.0 |
| 2 | 30 | 14.7 | 93.5 |
| 6 | 30 | 22.4 | 89.1 |
| 12 | 23.5 | 24.4 | 85.6 |
| 18 | 13.9 | 61.1 | 72.9 |
| 24 | 8.6 | 60.9 | 69.2 |
| 36 | 14.5 | 75.0 | 60.6 |

Clinical Outcomes of Stage I and Stage II Patients

None of the 47 c7E3 Fab-treated patients experienced a thrombotic event during or after PTCA. All but two of the 47 c7E3 treated patients had a successful PTCA as defined angiographically by a reduction of the lesion(s) to less than 50% luminal diameter narrowing. Of the two unsuccessful dilatations, patient 01-012 had a reduction of a 90% narrowing of the left anterior descending coronary artery to 70%, but further dilation was technically not possible. The second patient, (patient 01-019), reviewed below, had an initially successful dilation, but required intracoronary stent placement for a major longitudinal dissection (without evident thrombus). One of the 9 control patients (01-022) experienced thrombotic abrupt closure 15 minutes into the procedure, requiring emergency coronary artery bypass surgery (CABG), from which he recovered. The other 8 control patients had successful dilatations to 50% or less residual narrowing.

Patient 01-019 (12-hour infusion group) had a balloon dilatation of a 95% lesion of the left circumflex coronary artery with a 50% residual narrowing. After the procedure, the patient experienced an apparent vasovagal episode, leading to bradycardia, hypotension, and transient asystole. He was returned to the catheterization laboratory and had urgent intracoronary stent placement for a persistent major longitudinal dissection. The stent became dislodged in the left main coronary artery, and the patient was sent for emergency coronary artery bypass surgery. According to the investigator, there was no evidence of intracoronary thrombosis angiographically or intraoperatively. This patient also experienced a peri-operative myocardial infarction. The patient recovered and was discharged 8 days after surgery.

There were 3 c7E3 Fab-treated patients who each experienced an isolated episode of chest pain post-PTCA of uncertain significance. Patient 01-009 (0.25 mg/kg single dose group) experienced chest pain 9 hours post-c7E3, patient 05-003 (12-hour infusion group) experienced angina 21 hours post-c7E3, and patient 06-003 (12-hour infusion group) experienced angina 2 days post-c7E3. The investigators reported that these episodes of chest pain were unrelated to ischemic symptoms signifying reocclusion.

Patient 02-004 (0.25 mg/kg single dose group) experienced prolonged periods of chest pain prior to c7E3 Fab treatment which continued during the PTCA procedure. The following day ECG changes accompanied by elevated cardiac enzymes (drawn the preceding day) indicated that this patient had experienced a peri-procedural non Q-wave myocardial infarction (peak creatinine kinase = 462, MB fraction = 64).

There was one death in the trial which occurred 52 days after c7E3 Fab admininstration. Patient 06-002 (6-hour infusion group), who had a history of interstitial lung disease, congestive heart failure and unstable angina, underwent successful PTCA of the proximal left anterior descending coronary artery. During the procedure the patient developed sustained ventricular fibrillation, twice requiring electrical defibrillation, but thereafter the procedure proceeded uneventfully. After leaving the catheterization laboratory, the patient developed cyanosis, which initially responded to diuretics and oxygen therapy. However, this patient subsequently developed progressive respiratory impairment and later required ventilatory support. The patient's subsequent hospital course was complicated by sepsis, adult respiratory distress syndrome, anemia (requiring multiple transfusions), and cardiac ischemia. This patient died 52 days post-c7E3 Fab due to multi-system failure.

Safety: Stage I and Stage II Observations

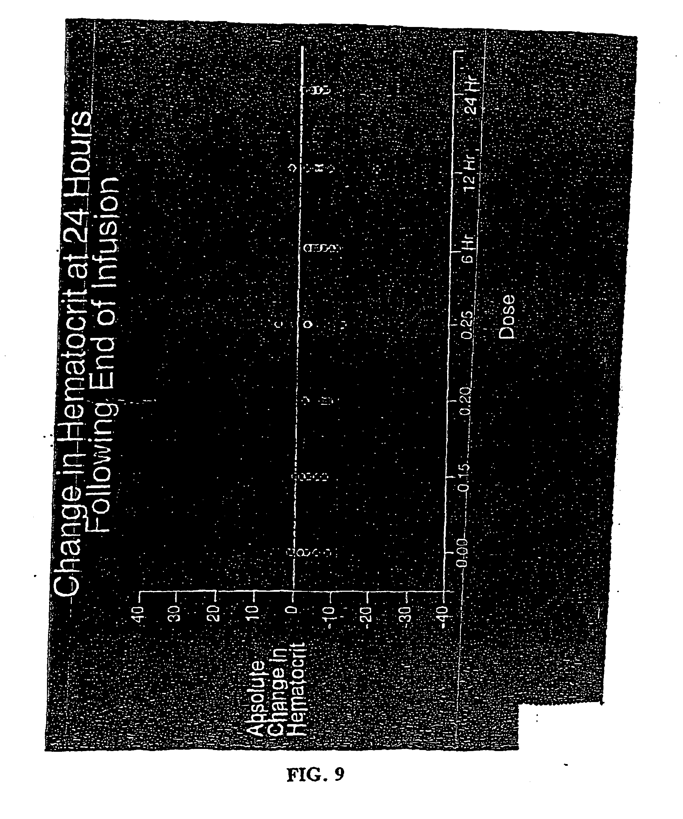

Figure 9 shows the absolute change in hematocrit from baseline to 24 hours following the end of infusion for all patients by dose group. For reference, a line indicating the zero change point is shown. The hematocrit data from one control patient (01-022) and one c7E3 Fab-treated patient (01-019) are not plotted because both patients required blood transfusions following urgent coronary bypass surgery in the first 24 hours (see below). A second lower line at -12 indicates the change in hematocrit needed to be designated as a minor bleed using the Thrombolysis in Myocardial Infarction (TIMI) criteria (Rao et al., J. Am. Coll. Cardiol. 11:1-11 (1988)). The changes in hematocrit are similar between the control patients and all c7E3 Fab dose groups. Table 10 summarizes the median change in platelet count at 24 hours following the end of infusion. The change in platelet count in the untreated control and c7E3 Fab-treated groups had similar distributions, with no apparent dose-related effect.

TABLE 10

| MEDIAN % CHANGE IN PLATELET COUNT AT 24 HOURS FOLLOWING THE END OF INFUSION | | |
|---|---|---|
| Dose | % Change | Range |
| Controls (n=9) | -0.7 | (-20.2, +13.2) |
| 0.15 mg/kg (n=5) | -17.4 | (-24.5, +19.5) |
| 0.20 mg/kg (n=5) | -11.6 | (-20.8, +4.7) |
| 0.25 mg/kg (n=5) | +2.9 | (-8.9, +4.4) |
| 6 hours* (n=11) | -7.7 | (-28.3, +19.4) |
| 12 hours* (n = 11) | 0.0 | (-24.3, +24.4) |
| 24 hours* (n=10) | -3.9 | (-9.0, +50.0) |
| *Period of infusion (10 $\mu$g/min) of c7E3 Fab following a bolus injection of 0.25 mg/kg | | |

Discussion of Stage I and Stage II Results

Stage I of this study established that c7E3 exhibits the same dose response characteristic in the PTCA population treated with aspirin and heparin as was seen in stable angina patients in a dose escalation trial (Example 3). Chimeric 7E3 produces a dose-dependent blockade of platelet GPIIb/IIIa receptors, and this receptor blockade correlates with inhibition of platelet function. In addition, Stage II results demonstrate that prolonged inhibition of platelet Fab function up to 24 hours can be achieved by a continuous infusion. In all patients, platelet functional recovery begins by 6 to 12 hours after cessation of the infusion, regardless of the duration of infusion.

The clinical outcome of the c7E3-treated patients, using both angiographic and clinical endpoints, was considerably better than expected based on their risk profile. No patient in the c7E3 treatment group experienced a thrombotic event during or after the procedure. In addition, all but 2 patients had angiographically successful procedures. All patients enrolled into Stage II and 6 of the 15 patients enrolled in Stage I were high risk patients on the basis of clinical or angiographic characteristics. An individual clinical factor (such as unstable angina, diabetes, women over age 65 years) or angiographic lesion-specific characteristic (such as type B or C) places a patient at increased risk of complications, and the effect of multiple factors are cumulative.

In Stage II, 17 treated patients had unstable angina with or without additional clinical or angiographic lesion-specific risk factors. In addition, 6 patients in Stage I were identified as having unstable angina. Published series have identified unstable angina patients as having a major complication (death, myocardial infarction, urgent coronary bypass surgery, or repeat PTCA) rate of 10 to 15% (De Feyter, P.J.: Editorial. Am. Heart J. 118: 860-868 (1989) and Rupprecht, H.J. et al. Eur Heart J. 11: 964-973, (1990)). Angiographic characteristics similarly are highly predictive of PTCA complications (Ellis, S.G., 1990, "Elective coronary angioplasty: technique and complications", In: Textbook of Interventional Cardiology, E.J. Topol, Ed., (W.B. Saunders Co., Philadelphia); De Feyter, P.J. et al., Circulation 83:927-936 (1991); Ellis, S.G. and Topol, E.J, Am. J. Cardiol. 66:932-937 (1990); and ACC/AHA Task Force Report: Guidelines for percutaneous transluminal coronary angioplasty, J. Am. Coll. Cardiol. 12:529-545 (1988)). Twenty-nine Stage II c7E3-treated patients met the eligibility criteria by means of lesion-specific characteristics. Of these, 12 patients had one Type B lesion, 14 had 2 or more Type B lesions, and three had Type C lesions. In addition, many of the patients in the trial had multiple lesions dilatated in a single or more than one vessel, which also potentially increases the risk of the procedure (Samson, M. et al., Am. Heart J. 120:1-12 (1990)). On the basis of both the number and severity of high risk angiographically defined risk factors in these patients, ischemic complications would have been expected in the range of 10 to 20% (Ellis, S.G.: Elective coronary angioplasty: technique and complications, In: Textbook of Interventional Cardiology, (Ed. E.J. Topol), W.B. Saunders Co., Philadelphia (1990); De Feyter, P.J., Circulation 83:927-936 (1991); Ellis, S.G. and Topol, E.J, Am. J. Cardiol. 66:932-937 (1990)).

The control group also was comprised of high risk patients. However, in general, the number and severity of risk factors was lower in the control patients. Five of the 9 control patients had single risk factors of either one type B lesion (4 patients) or unstable angina (1 patient), whereas 26 of 32 Stage II c7E3-treated patients had either a type C lesion or two or more other high risk characteristics. This difference in risk status between the 2 groups is significantly different (Fisher's exact test p = 0.018). Interestingly, the control patient with the abrupt closure (patient 01-022, unstable angina with 2 type B characteristics) was one of 3 patients identified as having more than one risk factor (one control patient had unspecified risk characteristics). Thus, whereas one of three control patients at highest risk had a thrombotic event, none of the 26 c7E3 Fab patients in this highest risk category had a thrombotic event.

This study also demonstrates that the potent antiplatelet effects of c7E3 can be achieved safely in patients already being treated with intravenous heparin and oral aspirin. Bleeding events were comparable in the control and c7E3-treated patients with no difference in hematocrit changes from baseline between dosing groups. Other adverse events were infrequent and typically of mild or moderate severity. There was one death in the trial, and this occurred almost 2 months after c7E3 Fab treatment in a patient with interstitial lung disease and heart failure who had progressive respiratory failure following PTCA, complicated by sepsis, adult respiratory distress syndrome, and eventually multiple organ failure.

Finally, none of the twenty patients in whom results were available experienced a human anti-chimeric antibody immune response.

In conclusion, chimeric 7E3 Fab potently inhibits platelet function safely in patients treated with aspirin and intravenous heparin who are undergoing PTCA. The antiplatelet action can be maintained for as long as 24 hours without a significant increase in bleeding risk and without immune system reactivity. Among patients at high risk of thrombotic complications, no thrombotic events occurred in the group treated with c7E3, suggesting that c7E3 can reduce the risk of thrombotic complications in this patient population.

Example 5 (for reference)

Treatment of Abrupt Closure During Coronary Angioplasty

Abrupt coronary arterial closure during coronary angioplasty is the major determinant of morbidity and mortality in this procedure. It occurs in approximately 3%-6% of elective angioplasty cases (Detre, K.M. et al., Circulation 82: 739-750 (1991)), but has been noted to occur in up to 20%-40% of patients who undergo angioplasty for unstable angina pectoris or after acute myocardial infarction (Ellis, S.G. et al., Circulation 77:372-379 (1988); DeFeyter, P.J. et el., Circulation 83:927-936 (1991)). The mechanism of abrupt closure is acute thrombosis at the arterial site where angioplasty has created or extended an area of endothelial injury. Usually there are disturbed flow patterns due to altered geometry of the vessel, often from disruption of the plaque material, and there is exposure of subendothelial elements including intimal and often medial dissection. Since initiation of the thrombus requires adhesion and aggregation of platelets, chimeric 7E3 Fab antibody fragment was used for the treatment of abrupt coronary arterial closure complicating a coronary angioplasty procedure.

Case Report

The patient is a 45 year old male physician who had been in excellent health previously. Beginning one week prior to his angioplasty procedure, he had begun to experience chest and neck discomfort. When these symptoms persisted and worsened over several days, he sought the advice of a colleague. An electrocardiogram (EKG) revealed anterior precordial T wave inversions. The patient was then hospitalized in the coronary care unit of a local hospital and placed on intravenous nitroglycerin and heparin, and oral aspirin. Serial Cardiac isoenzyme determinations over the next 24 hours did not reveal elevation above the normal range. Serial EKG recordings over the next two days revealed persistent flattening of the anterior precordial T waves but no evolutionary changes of myocardial infarction. On the second day after hospitalization the patient was taken to the cardiac catheterization laboratory, where left ventriculography revealed overall normal left ventricular function with a very small hypokinetic area in the anterolateral left ventricular wall, and another hypokinetic area in the inferoposterobasilar zone. The left ventricular ejection fraction was 72%. Coronary arteriography demonstrated a left-dominant coronary system with a small and totally occluded right coronary artery. There was a significant stenosis in the mid portion of the left anterior descending (LAD) coronary artery. A small and diffusely diseased diagonal branch originated just distal to the mid LAD stenosis.

The patient was returned to the coronary care unit and remained on intravenous nitroglycerin and heparin for another 48 hours. He was pain free during this time, cardiac isoenzymes did not rise, and daily EKGs revealed only the persistent flattening of the anterior precordial T waves. He was transferred to Hermann hospital (Houston, TX) for angioplasty.

Prior to the angioplasty procedure the patient continued to receive intravenous nitroglycerin and heparin, oral aspirin, and he was started on an oral calcium channel blocking agent. The partial thromboplastin time (PTT) had remained in the 70-90 seconds range for several days. At the start of his angioplasty procedure the activated clotting time (ACT) was 173 seconds. The patient received 5000 units heparin intravenously. The left coronary ostium was engaged with a number 8 French JL 3.5 guiding catheter. The LAD coronary artery was visualized in the caudal right anterior oblique and cranial left anterior oblique projections. The LAD was first instrumented with a 0.018 inch Doppler guidewire (Cardiometrics, Inc., Mountain View, CA). This guidewire is used by us routinely for flow monitoring in patients at higher risk for abrupt closure. Flow-velocity signals from the LAD proximal and distal to the lesion were recorded. A 2.5 mm coronary balloon catheter (Intrepid, Baxter, Inc., Irvine, CA) was advanced over the Doppler guidewire while the wire was held stationary in the coronary artery. The balloon was positioned so that it straddled the LAD lesion. Sequential brief balloon inflations were made to 6 atmospheres pressure. The severity of the stenosis was reduced as visualized by angiography as well as by increase in the flow velocity signal from a peak flow velocity (APV) of 12 cm/sec to 33 cm/sec.

During several minutes of observation following these dilations it was noted that the flow signal began to diminish. A contrast injection revealed renarrowing of the angioplasty site from elastic recoil, plaque disruption, and formation of thrombus. The balloon was reintroduced to the site of the lesion and another balloon inflation was performed. The artery was reexpanded and the flow signal again returned to an APV of 34 cm/sec.

During several more minutes of monitoring the signal again declined. within 5 minutes the signal was quite low, at average peak velocity of 3 cm/sec. The patient began to experience chest pain. The EKG monitor of an anterior precordial lead revealed ST segment elevation. Angiography revealed that the artery was completely occluded. The activated clotting time obtained just a few minutes before was 344 seconds.

Chimeric 7E3 monoclonal antibody Fab fragment specific for the platelet GP IIb/IIIa receptor (c7E3 Fab, $\gamma_1$, $\kappa$) was administered. The dose was 0.25 mg per kilogram given intravenously over 1 minute. Within approximately 1 to 2 minutes after administration of c7E3 Fab, the coronary flow velocity began to increase. An injection of contrast revealed restoration of coronary patency with Thrombolysis In Myocardial Infarction Trial Grade-I (TIMI 1) flow. Over the subsequent 15 minutes coronary flow continued to increase and stabilized at an APV of 23 cm/sec. Several other injections of contrast demonstrated improved coronary flow. The patient's chest pain subsided and the ST segment observed in the monitor lead returned to baseline.

Fifteen minutes after administration of c7E3 Fab, an angiogram was made according to protocol. This angiogram revealed TIMI3 coronary flow. The flow velocity signal at this time was 20 cm/sec. Continuous monitoring through the subsequent 5 minutes revealed no further improvement in the coronary flow. During that time the video replay of the angiogram confirmed that there was a small amount of thrombus still visible at the angioplasty site. For this reason it was decided to administer intracoronary urokinase 250,000 units. This thrombolytic agent was infused over approximately the next 10 minutes. During that time there was no further improvement in flow as measured by the Doppler guidewire. After completion of the intracoronary urokinase infusion, at the 33rd minute after administration of c7E3 Fab, another coronary angiogram was made. The artery was patent with TIMI 3 flow. Some moderate but definite residual stenosis persisted at the lesion site. In addition, it was observed that the thrombus had diminished further in size but had not been completely dissolved. The decision was made to perform another balloon inflation in order to try to reduce the residual stenosis.

The balloon catheter was again advanced over the guidewire to the site of the lesion. A final balloon inflation to 6 atmospheres for 2 minutes was then performed. Then, the balloon catheter was withdrawn while the wire remained in place. The flow signal increased to an APV of 29 cm/sec and remained stable over several minutes. An angiogram demonstrated adequate reduction in the residual stenosis which had been present. The guidewire was then withdrawn proximal to the stenosis and another flow velocity recording was made. The guidewire, balloon catheter and guiding catheter were withdrawn. This completed the procedure.

The patient was then taken to the coronary care unit. He remained on oral aspirin, nitrates, a calcium channel blocking agent, and intravenous heparin for several days in order to keep the PTT in the 70-90 seconds range. Serial EKGs demonstrated resolution of the anterior precordial T wave inversions and all subsequent EKGs were normal. Serial creatine kinase (CK) isoenzyme values were consistently < 100 U/L. The platelet count prior to the PTCA procedure was 248,000, and subsequent platelet counts at 2h, 6h, 12h, 24h, and 48h after c7E3 Fab administration were 304,000, 279,000, 246,000, 185,000 and 220,000, respectively. Platelet aggregation induced by 10 $\mu$M ADP was 73% by optical densitometry prior to the procedure, and subsequent values at 2h, 6h, 12h, 24h, and 48h were 0%, 13%, 26%, 45%, and 51%, respectively. One week after the angioplasty procedure, the patient had a follow-up catheterization. The LAD coronary artery was found to be widely patent with TIMI 3 flow. He was discharged home later that same day.

Discussion of Case Report

In this patient, the combination of 0.25 mg/kg c7E3 Fab intravenously, 250,000 U intracoronary urokinase, and repeat dilatation, successfully treated the acute ischemic coronary syndrome of abrupt closure during coronary angioplasty. These results suggest that antiplatelet therapy that inhibits platelet glycoprotein IIb/IIIa receptor binding and platelet cross-bridging may be efficacious in helping to achieve stable reperfusion of acutely occluded coronary arteries in similar clinical settings.

Example 6 (for reference)

A Randomized, Double-Blinded, Evaluation Anti-GPIIb/IIIa Chimeric Antibody Fragment for Preventing Ischemic Complications of High Risk Angioplasty

Overview

Percutaneous myocardial revascularization has grown at a dramatic rate since the advent of coronary angioplasty in 1977 (Gruentzig, A.R. et al., N. Engl. J. Med., 316:1127-1132 (1987)). Although the procedure is associated with improvement in symptoms of ischemia and quality of life, (Parisi, A.F. et al., N. Engl. J. Med., 326: 10-16 (1992)), acute complications remain a major drawback. The treated vessel closes abruptly during or after the procedure in the hospital in 4 to 9% of cases, and reocclusion or abrupt closure is associated with considerable morbidity and an approximately 10-fold increase in mortality (Lincoff, A.M. et al., J. Am. Coll. Cardiol., 19: 926-938 (1992); Tenaglia, A.N. et al., Am. J. Cardiol, In press (1993); Detre, K.M. et al., Circulation, 82:739-750 (1990); Ellis, S.G. et al., Circulation, 77: 372-379 (1988)). Although the mechanism of abrupt vessel closure is often uncertain, thrombus formation and vessel dissection are contributory factors. Characteristics that identify patients at high risk of acute complications include the presence of a clinical syndrome associated with coronary thrombus (unstable angina, acute or recent myocardial infarction), diabetes, female gender and coronary morphologic characteristics (bend points, thrombus, bifurcation) indicating increased complexity of the individual lesion (Lincoff, A.M. et al., J. Am. Coll. Cardiol., 19:926-938 (1992); Ellis, S.G. et al., J. Am. coll. Cardiol, 17:(Suppl) B):89B-95B (1991); Moushmoush, B. et al., Cath. Cardiovasc. Diagn., 27: 97-103 (1992); and Myler, R.K. et al., Circulation, 82 (Suppl II):II-88-II-95 (1990)).

Although aspirin has been shown to reduce the risk of abrupt vessel closure and acute myocardial infarction in patients undergoing angioplasty (Schwartz, L. et al., N. Engl. J. Med., 318:1714-1719 (1988); Barnathon, E.S. et al., Circulation, 76:125-134 (1987)), its effects on platelet function are relatively weak, and ischemic events continue to occur at a rate of 10-20% in high-risk patients pretreated with aspirin (Tenaglia, A.M. et al., J. Am. Coll. Cardiol., (1993, in press)). In contrast, administration of the Fab fragment of chimeric 7E3 antibody to humans achieved substantial blockade of the GPIIb/IIIa receptors and inhibition of platelet aggregation (see Example 4, Inhibition of Platelet Function).

In an initial study of patients undergoing angioplasty, c7E3 Fab reduced the risk of abrupt vessel closure during and after percutaneous intervention (see Example 4 and Ellis, S.G. et al., Cor. Art. Dis., 4:167-75 (1993)). The current randomized study was designed to further evaluate the efficacy of chimeric antibody fragments which bind selectively to the glycoprotein IIb/IIIa receptor in preventing ischemic complications (EPIC trial, Evaluation of c7E3 Fab to Prevent Ischemic Complications). In particular, the clinical efficacy of a chimeric 7E3 Fab fragment (c7E3 Fab) in patients undergoing angioplasty who were at high risk of procedural complications was evaluated in a prospective, double-blind, placebo-controlled, randomized clinical trial. The trial included 2099 patients at 56 sites who were scheduled to undergo coronary angioplasty or directional coronary atherectomy in high-risk clinical situations: severe unstable angina with rest pain and documented electrocardiographic changes, evolving acute myocardial infarction, or clinical and coronary lesion morphological characteristics associated with a high risk of peri-procedure complications. Patients received either (a) placebo bolus and infusion, (b) a bolus of c7E3 Fab and placebo infusion, or (c) a bolus and infusion of c7E3 Fab. The primary endpoint was a composite which included the occurrence of any one of the following components: death, nonfatal myocardial infarction, unplanned surgical revascularization or repeat percutaneous procedure, unplanned coronary stent implantation, or intraaortic balloon pump insertion for refractory ischemia.

The bolus and infusion resulted in a 35% reduction in the primary endpoint (12.8 vs 8.3%, P = 0.008), while an 11 percent reduction was observed with the bolus alone (12.8 vs 11.4%, P = 0.43). The reduction in events achieved by administration of bolus plus infusion was consistent within each of the endpoint components, and in addition, in major patient subgroups including age, gender, preexistence of intercoronary thrombus, and acute coronary syndromes (myocardial infarction, unstable angina). Bleeding episodes and transfusions were increased in the bolus and infusion group and intermediate for the bolus alone regimen. This controlled trial of high-risk patients undergoing coronary intervention revealed that administration of an antibody fragment directed against the platelet IIb/IIIa receptor led to sustained clinical benefit through a significant reduction in ischemic complications.

Methods

Based on previous studies stratifying risk with conventional percutaneous intervention, patients were eligible if they were at high risk for abrupt vessel closure and had no major contraindication due to high risk of bleeding. Patients were considered at high risk if they were in one of three clinical groups: (1) acute evolving myocardial infarction within 12 hours of the onset of symptoms undergoing either direct or "rescue" percutaneous intervention; (2) early postinfarction angina or unstable angina with at least 2 episodes of rest angina associated with resting electrocardiographic changes in the previous 24 hours despite medical therapy; or (3) high-risk clinical and/or angiographic criteria using the American Heart Association/American College of Cardiology criteria (Ryan, T.J. et al., J. Am. Coll. Cardiol., 12:529-45 (1988)) as modified by (Ellis, S.G. et al., J. Am. Coll. Cardiol., 17 (suppl B):89B-95B (1991)). These high-risk clinical and angiographic criteria included either two Type B characteristics or 1 Type C characteristic in the target lesion, or one Type B characteristic in women over age 65 or in patients with diabetes.

The specific inclusion criteria are presented in more detail below:

(I) Referred for elective or urgent coronary balloon angioplasty or atherectomy with an FDA approved device in one of the following settings:

(A) Unstable Angina and/or Non-Q Wave Myocardial Infarction defined as:

1) Angina at rest: two or more episodes of angina at rest with ischemic ST segment or T wave abnormalities; or
2) Recurrent angina: recurrent angina with ischemic ST segment or T-wave abnormalities while hospitalized not responsive to standard pharmacologic intervention; or
3) early post-infarction angina: angina within 7 days of documented myocardia infarction, with angina at rest accompanied by ischemic ST segment or T wave changes; or angina provoked by minimum exertion (2 mets),

wherein transient ischemic ST segment or T wave abnormalities were defined as:

a) $\geq$ 1 mm ST segment depression (80 msec after the J point) or elevation (20 msec after the J point), and/or
b) T wave changes (usually inversion), and wherein creatine kinase (CK) must have been less than 2 times normal, for all patients at the time of enrollment.

(B) Acute Q-wave Myocardial Infarction

1) Direct intervention during myocardial infarction without antecedent thrombolytic therapy, or
2) Rescue angioplasty for failed thrombolytic therapy during myocardial infarction,

wherein an enrolling myocardial infarction was defined as the presence of at least two of the following three criteria:

(1) prolonged angina (greater than 30 minutes);
(2) total creatine kinase elevation to greater than 2 times the upper limit of normal (confirmed by CK-MB isoenzyme elevation)
(3) ECG evidence of infarction defined as:

a) ST segment elevation of at least 0.1 mV (measured 0.2 seconds after the J point) in at least one of three locations:

i) at least 2 of 3 inferior leads (II, III, aVF); or

ii) at least 2 of 6 precordial leads ($V_1$-$V_6$); or

iii) leads I and aVL; or

iv) ST segment depression of the precordial leads $V_1$-$V_4$ consistent with posterior current of injury (mirror rule); or

v) in the presence of left bundle branch block, primary ST changes in the inferior or anterior leads;

b) new significant Q wave of $\geq$ 0.04 seconds duration or having a depth $\geq$ one-fourth of the corresponding R wave amplitude, or both.

(C) High Risk Clinical/Morphological Characteristics

1) Stenosis with two or more type B lesion-specific characteristics in the artery to be dilated. Lesion specific-characteristics were based on ACC/AHA criteria;

2) Stenosis with one or more type C characteristics in the artery to be dilated;

3) Age ≥ 65 years combined with female gender and stenosis with at least one type B characteristic;

4) Diabetes mellitus and stenosis with at least one type B characteristic in the artery to be dilated; or

5) Angioplasty of an infarct-related lesion within 7 days of myocardial infarction documented by characteristic CK-MB isoenzyme elevation.

II. Men between the ages of 18 and 80, and women between the ages of 18 and 80, not of childbearing potential (i.e., surgically sterilized or post-menopausal, defined as not having a menstrual period for at least one year.

III. Written informed consent provided before initiation of protocol-specific procedures and study agent administration.

Patients who otherwise qualified for the study were excluded from participation for any of the following reasons:

(1) History of hemorrhagic diathesis;

(2) Major surgery within 6 weeks of study enrollment;

(3) Recent (within 6 weeks of enrollment) gastrointestinal or genitourinary bleeding of clinical significance;

(4) Stroke within 2 years prior to enrollment or any stroke with significant residual neurological deficit;

(5) A greater than 50% occlusion of the left main coronary artery;

(6) Presumed or documented history of vasculitis;

(7) Participation in other clinical research studies involving the evaluation of an investigational drug or device within 7 days prior to proposed study agent infusion;

(8) Administration of oral anticoagulants within 7 days prior to randomization to study agent, unless the pre-randomization prothrombin time is ≤ 1.2 times control;

(9) Use of intravenous dextran before or planned for during the treatment angioplasty

(10) History of prior administration of murine monoclonal antibodies or known allergies to murine proteins; or

(11) Inability to give informed consent.

Institutional review board approval was obtained at all institutions and informed consent was obtained from all patients. Recruitment into the trial occurred between December 1991 and November 1992; 2099 patients were enrolled at 56 institutions in the United States.

Study Protocol

All patients were treated with aspirin and heparin. Aspirin was administered orally at a dose of 325 mg at least 2 hours before the procedure, and were maintained a dose of 325 mg once a day thereafter. Heparin (porcine) was administered as an initial bolus of 10,000 to 12,000 units intravenously followed by incremental boluses of up to 3,000 units at 15 minute intervals, but not exceeding a total of 20,000 units; the goal was to keep the activated clotting time at a "therapeutic" range, generally considered to be 300 to 350 seconds during the procedure (Dougherty, K.G. et al., Abstracts of the 63rd Scientific Sessions, III-189 (1991); Rath, B. et al., Br. Heart. J., 63:18-21 (1990); Ogilby J.D. et al., Cath. Cardiovasc. Diag., 18:206-9 (1989)). Heparin was continued by constant infusion at a rate of 1,000 units per hour for at least 12 hours. Intravenous and intracoronary nitrates could be used as clinically indicated. Chimeric 7E3 Fab (-$\gamma_1$, $\kappa$) was supplied as a sterile, nonpyrogenic solution containing 2 mg of monoclonal Fab per mL of 0.15 M sodium chloride, 0.01 M sodium phosphate and 0.001% polysorbate 80, pH 7.2. The only medication required at discharge was aspirin at a dose of 325 mg per day.

Patients were randomized equally into one of three treatment arms with a double-blind study design. One group of patients was to receive a bolus of c7E3 Fab at a dose of 0.25 mg/kg followed by a 12-hour continuous infusion of c7E3 Fab at a dose of 10 $\mu$g/min. A second group was to receive a bolus dose of 0.25 mg/kg c7E3 Fab and 12-hour continuous infusion of placebo solution. A third group was to receive a placebo bolus and 12-hour continuous infusion of placebo solution. The bolus was started at least 10 minutes before the procedure and given over 5 minutes, and the infusion was continued for 12 hours unless a clinical contraindication developed.

Blood samples for platelet counts were drawn 30 minutes, 2, 12 and 24 hours following initiation of the drug and then daily until hospital discharge to carefully examine for evidence of thrombocytopenia. A predesigned algorithm was used to evaluate and treat life-threatening bleeding and thrombocytopenia (Sane, D.C. et al., Ann. Intern. Med., 111:1010-22 (1989)). The protocol contained no specific indications governing red blood cell transfusion; instead, transfusions were prescribed according to local practice patterns existing at each site. Angioplasty was performed according to standard protocols. Pre- and post-procedure angiography were performed after coronary vasodilation with 150-300 $\mu$grams of intracoronary nitroglycerin. After the procedure, vascular sheaths were maintained for at least 6 hours after the end of study agent infusion. Additionally, sheaths were left in place until at least 4 hours after the end of the heparin infusion and until an acceptable activated partial thromboplastin time was achieved to maintain hemostasis.

Study Endpoints

An independent Clinical Endpoint Committee reviewed all episodes that might have represented a study endpoint or a major adverse event. This committee, which remained blinded to treatment throughout the study, reviewed the case report forms, electrocardiograms and pertinent medical records when needed. A consensus of two reviewers was required to classify an event.

The primary endpoint of the trial was a composite clinical endpoint including the occurrence of any one of the following events in the first 30 days after randomization:

(1) Death from any cause;
(2) Nonfatal myocardial infarction; or
(3) Urgent intervention:

(a) Second angioplasty. Repeat percutaneous intervention for recurrent acute ischemia (balloon angioplasty or coronary atherectomy). Scheduled (e.g., staged procedures) were not considered endpoint events;
(b) Coronary artery bypass graft. Urgent (non- elective) surgical intervention to treat recurrent acute ischemia;
(c) Insertion of a coronary endovascular stent. Intracoronary stent placement in order to maintain the immediate patency of the dilated vessel; or
(d) Insertion of an intraaortic counterpulsation balloon pump. Balloon pump placed for recurrent ischemia in patients not considered candidates for repeat angioplasty or surgical intervention.

For the purposes of this study, "patency" was defined as TIMI Grade 2-3 flow with a less than or equal to 50% visual stenosis as determined by the operator and no ECG evidence of ischemia.

Endpoint myocardial infarctions were defined as follows:

1. In patients randomized within 24 hours of an acute evolving myocardial infarction, one of two enzymatic criteria was required for the diagnosis of a subsequent nonfatal infarction: (a) a creatine kinase (CK) or creatine kinase MB at least 3 times the upper limit of normal, representing an increase of at least 33% from the previous "valley" (defined as a 25% decrease from a prior peak value but remaining at least twice the upper limit of normal); or (b) an increase in CK or CK-MB of at least 100% and remaining three times the upper limit of normal following a 50% decrease from a prior peak level and a "valley" level less than twice the upper limit of normal. A documented new episode of prolonged angina (greater than 20 minutes) accompanying the re-elevation of cardiac enzymes was used to establish the time of onset of reinfarction. In the absence of documented angina, the onset of reinfarction was set as the time of measurement of the valley enzyme level immediately preceding new elevation. Definition 1 was used only for those patients in whom the onset of recurrent angina and/or the valley enzyme level occurred within 24 hours of the onset of the myocardial infarction present at study entry. In all cases the CK-MB level was used unless it was not available, in which case the total creatine kinase values were used.

2. In patients entered into the trial more than 24 hours after an acute infarction or without a recent infarction, one of two criteria had to be met for a diagnosis of in-hospital myocardial infarction: (a) a new Q wave ≥ 0.04 sec in duration or with a depth > 1/4 the corresponding R wave amplitude in two or more contiguous leads; or (b) a CK-MB level at least 3 times the upper limit of normal and an increase in that level ≥ 50% over the previous "valley" level. For this definition, the time of onset of reinfarction in patients with acute myocardial infarction at study entry was either the time of occurrence of a new episode of prolonged angina (> 20 minutes) or the time of measurement of a valley enzyme level preceding the new enzyme elevation. For this definition to be applicable, either time must have been greater than 24 hours following the initial infarction.

3. After hospital discharge, one of the two following criteria was required to diagnose a myocardial infarction: (a) a new significant Q wave ≥ 0.04 sec in duration or with a depth ≥ 1/4 the corresponding R wave amplitude, or both, in two or more contiguous leads; or (b) a CK or CK-MB level greater than twice the upper limit of normal.

Another component of the primary endpoint was the need for urgent repeat intervention, defined as an unplanned return to the angioplasty suite for intervention; planned staged procedures were not included in the primary endpoint. Similarly, only urgent coronary surgery to treat recurrent ischemia or a failed angioplasty procedure was counted as a primary endpoint. Intracoronary stent placement was considered a primary endpoint when the stent was placed to treat threatened or actual abrupt closure of the vessel undergoing angioplasty. Intraaortic balloon pump placement was considered to be a primary endpoint when the pump was placed for recurrent ischemia in a patient not undergoing a repeat revascularization procedure.

Bleeding events were classified as major, minor or insignificant using the Thrombolysis in Myocardial Infarction Study Group criteria (Rao, A.K. et al., J. Am. Coll. Cardiol., 11:1-11 (1988)). Major hemorrhages were defined as intracranial bleeding or bleeding associated with a decrease in hemoglobin greater than 5 g/dl (or, when hemoglobin was not available, a hematocrit decrease of at least 15%). Minor bleeding was either spontaneous and observed as gross hematuria or hematemesis, associated with a drop in hemoglobin greater than 3 g/dl (or, when hemoglobin was not available, a decrease in hematocrit of at least 10%), or when no bleeding site was identified, a decrease in hemoglobin greater than 4 g/dl (or, when hemoglobin was not available, a hematocrit decrease of at least 12%). In patients who underwent blood transfusion, the number of units transfused was added to the observed drop in hematocrit divided by 3 in order to obtain the total decrease in hemoglobin used for deciding whether major or minor bleeding occurred (Landefield, C.S. et al., Am. J. Med., 82:703-13 (1987)).

Data Management and Statistics

Patients were randomized via telephone communication with the Data coordinating Center at Duke University. Randomization was stratified by study site and according to whether the patient was having an acute evolving myocardial infarction. Based on previous data, a sample size of 2,100 patients was planned in order to detect a 33% reduction in the primary endpoint (predicted to be 15% in the placebo group) with a power of 0.8 and $\alpha = 0.05$.

Data were collected by study coordinators on case report forms and monitored by blinded study monitors prior to data entry. The sponsor remained blinded to the randomization code and study results until all patients were enrolled and all endpoints had been adjudicated by the clinical endpoints committee.

Baseline characteristics are displayed as medians and 25th and 75th percentiles for continuous variables and as percentages for discrete variables in the tables below. The primary endpoint of the trial was analyzed by considering the time until the first occurrence of any one of the components of the composite endpoint within the first 30 days after enrollment. If no event occurred within the 30-day interval, the patient's follow-up was censored after 30 days. Kaplan-Meier survival curves for each treatment were used to graphically display the results (Kaplan, E.L. et al., J. Am. Stat. Assn., 53:457-81 (1958)). All treatment comparisons were performed using the intention-to-treat principle. For the primary endpoint, a log-rank test for trend (dose-response) was performed, considering the bolus only patients intermediate to the bolus and infusion group (Kalbfleisch, J.D. and R.L. Prentice, The Statistical Analysis of Failure Time Data, John Wiley and Sons, New York (1980)). The analysis plan then called for pairwise log-rank comparisons between the control group and each of the two c7E3 Fab groups if the test for trend was significant. Interim analyses of safety were performed when data were available on one-third and approximately two-thirds of the patients. The nominal alpha level used for judging significance of the test for dose-response at each interim analysis was prespecified to maintain an overall type I error rate ≤ 0.05. At the final analysis, the significance level used for this comparison was 0.036. A similar strategy (test for trend followed by pairwise treatment comparisons as appropriate) was employed in the final analysis to explore the relationship of treatment to each component of the composite endpoint, although these comparisons were primarily for explanatory purposes. The final analysis also employed this strategy to compare treatments with respect to measures of bleeding complications, using conventional chi-square testing. Odds ratios and confidence intervals for the treatment effect in major subgroups (age, sex, weight, clinical subgroup) were calculated and graphically displayed.

Results

The baseline clinical characteristics of the patients groups show that the population was at increased risk for acute complications of angioplasty because of high proportions of diabetes, recent myocardial infarctions, advanced age and women in combination with the lesion characteristics comprising the entry criteria (see Table 11). The majority of patients had one or two vessel disease with good left ventricular function. Details of the interventional procedures are listed in Table 12. No substantial differences according to treatment assignment are evident.

The overall primary endpoint and its components are shown in Table 13. Compared with placebo, a graded effect of c7E3 Fab is demonstrated (p = 0.009), with an 11% reduction in the composite event rates in the bolus alone group (p = 0.43) and a 35% reduction in event rates in the bolus plus infusion group (p = 0.008). The same graded effect was observed for each of the most important ischemic endpoints shown in Table 13. Thus, the sustained glycoprotein IIb/IIIa blockade reduced nonfatal infarction, emergency coronary bypass surgery and emergency percutaneous revascularization, while an insignificant trend in the same direction was present with the short term blockade produced by the bolus alone. Three of the deaths in the bolus and infusion group occurred in patients who died after randomization but before receiving the drug; despite this, these deaths are included in the analysis according to the intention-to-treat principle.

Since nonfatal ischemic events were prevented by c7E3 Fab, the severity of the nonfatal myocardial infarctions prevented is of interest. As shown in Table 14, both Q wave infarctions and infarctions associated with large enzyme elevations were prevented, and a dose-response effect was present.

Table 11

| Baseline Characteristics | | | |
|---|---|---|---|
|  | Placebo (N=696) | Bolus (H=695) | Bolus + Infusion (N=708) |
| Age (yr)+ | 62 (53, 69) | 61 (52, 68) | 63 (53, 69) |
| Male (%) | 73 | 72 | 71 |
| Weight (kg) | 84 | 82 | 82 |
| Risk Factors (%) | | | |
| Diabetes | 26 | 23 | 23 |
| Hypertension | 55 | 55 | 54 |
| Elevated cholesterol | 57 | 59 | 55 |
| Smoking | 65 | 71 | 68 |
| Vascular Disease (%) | | | |
| Peripheral | 9 | 9 | 9 |
| Cerebral | 3 | 4 | 4 |
| Previous MI (%) | | | |
| None | 34 | 30 | 30 |
| >30 days | 25 | 28 | 27 |
| 8-30 days | 13 | 13 | 14 |
| <8 days | 28 | 29 | 29 |
| Prior Procedure (%) | | | |
| Angioplasty | 25 | 20 | 22 |
| Bypass Surgery | 15 | 14 | 16 |
| Coronary Anatomy (%) | | | |
| 1 vessel disease | 54 | 51 | 55 |
| 2 vessel disease | 29 | 34 | 31 |
| 3 vessel disease | 16 | 15 | 13 |
| MI = myocardial infarction.<br>+Median (25th, 75th percentiles) | | | |

**Table 12**
**Interventional Procedural Details**

|  | Placebo (N=696) | Bolus (N=695) | Bolus + Infusion (N=708) |
|---|---|---|---|
| Procedure (%) | | | |
| Balloon angioplasty | 90 | 90 | 90 |
| Atherectomy | 5 | 4 | 5 |
| Both | 5 | 6 | 5 |
| Time in lab (min) + | 134, (71, 394) | 129 (75, 401) | 141 (69,513) |
| Contrast used (ml)+ | 200 (150,286) | 200 (150,277) | 200 (150,285) |
| Thrombolytics (%) | 3.2 | 3.7 | 2.3 |

(continued)
**Interventional Procedural Details**

|  | Placebo (N=696) | Bolus (N=695) | Bolus + Infusion (N=708) |
|---|---|---|---|
| Procedure (%) |  |  |  |
| Lowest ACT (sec)+ | 271 (190,345) | 284 (190,371) | 285 (185,388) |

ACT = activated clotting time
+Median (25th, 75th percentiles)

**Table 13**
**Primary Outcome Events**

|  | Placebo (N=696) | Bolus (N=695) | Bolus and Infusion (N=708) | Dose-response p-values |
|---|---|---|---|---|
| Primary Endpoint* | 89 (12.8%) | 79 (11.4%) | 59 (8.3%) | 0.009 |
| Composites of Primary Endpoint: |  |  |  |  |
| Death | 12 (1.7%) | 9 (1.3%) | 12 (1.7%) † | 0.96 |
| Nonfatal MI | 60 (8.6%) | 43 (6.2%) | 37 (5.2%) | 0.013 |
| Emergency PTCA | 31 (4.5%) | 25 (3.6%) | 6 (0.8%) | <0.0001 |
| Emergency CABG | 25 (3.6%) | 16 (2.3%) | 17 (2.4%) | 0.177 |
| Stent | 4 (0.6%) | 12 (1.7%) | 4 (0.6%) | 0.97 |
| Balloon pump | 1 (0.1%) | 1 (0.1%) | 1 (0.1%) | 0.99 |

* p = 0.009 for overall test for trend;
p = 0.43 for comparison of placebo with bolus;
p = 0.008 for comparison of placebo with bolus and infusion.
† Three patients who died were assigned to this treatment limb but never actually received therapy.
MI = myocardial infarction;
PTCA = percutaneous angioplasty or atherectomy;
CABG = coronary artery bypass grafting.

**Table 14**
**Effect of c7E3 Fab on Myocardial Infarction**

|  | Placebo (Nn696) | Bolus (N=695) | Bolus and infusion (N=708) | Done-response p-values |
|---|---|---|---|---|
| Q wave MI (%) | 2.3 | 0.9 | 0.8 | 0.018 |
| Large non-Q wave MI (%) | 4.0 | 2.4 | 2.8 | 0.197 |
| Small non-Q wave MI (%) | 2.3 | 2.9 | 1.6 | 0.342 |
| Total (%) | 8.6 | 6.2 | 5.2 | 0.013 |

MI = myocardial infarction
Large non-Q wave MI is defined by a peak CK-MB or total CK > 5 times upper limit of normal;
Small non-Q wave MI is defined by a peak CK-MB or total CK 3 to 5 times upper limit of normal.

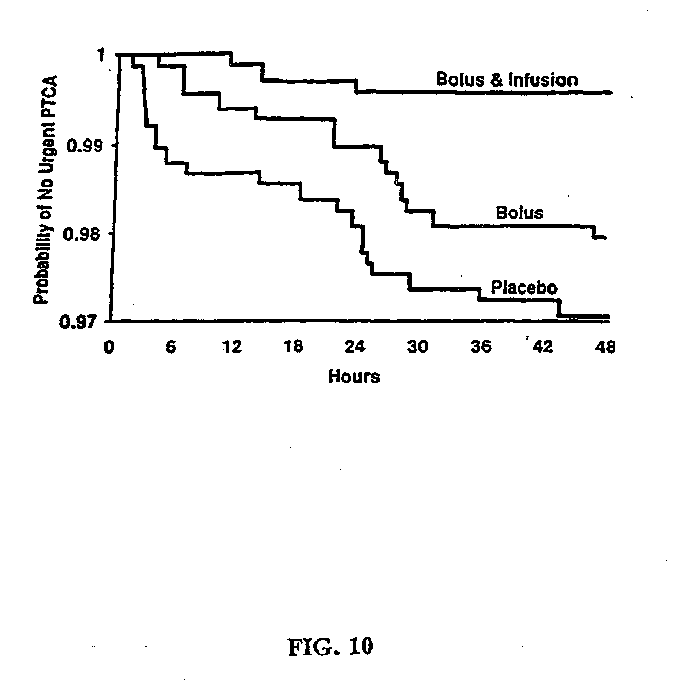

The timing of nonfatal ischemic events was different in the 3 groups for urgent repeat angioplasty, an event that could be timed accurately (See Figure 10). The majority of events in the placebo group occurred in the first hours after the index procedure, while a delay of several hours (-6-12 hours) until the occurrence of events was evident among the bolus group, corresponding with the time of maximal receptor blockade. There was a marked delay in the onset of ischemic events in the bolus plus infusion group as well as a marked reduction in their absolute frequency.

The profile of bleeding complications during hospitalization is shown in Table 15. As with the primary endpoint for efficacy, a graded effect of treatment on bleeding is evident. Patients in the bolus plus infusion group displayed substantial increases in both major bleeding rate and transfusion rate, while patients receiving bolus alone displayed only a moderate increase. The majority of bleeding episodes occurred during coronary artery bypass grafting or at the site of vascular puncture in the groin, although the rate of surgical vascular repair was evenly distributed (1% in the placebo and bolus plus infusion groups and 2% in the bolus only group). Similarly, six patients had an intracranial hemorrhage, with two events in the placebo group, one in the bolus only group and three in the bolus plus infusion group, one of whom did not receive the drug because the event occurred after randomization but prior to the angioplasty.

**Table 15**
**Bleeding Complications and Hematologic Measures**

| | Placebo | Bolus | Bolus and Infusion |
|---|---|---|---|
| | (N=696) | (N=695) | (N=708) |
| Major bleed (%)* | 46 (7%) | 76 (11%) | 97 (14%) |
| Transfusions ** | | | |
|     Red cells | 49 (7%) | 92 (13%) | 109 (15%) |
|     Platelets | 18 (3%) | 29 (4%) | 39 (6%) |
| Blood count | | | |
|     Nadir hematocrit † | 35 (32,38) | 34 (29, 38) | 33 (29,37) |
|     Δ hematocrit † | 5.3 (3.2,7.9) | 6.5 (4.2,9.8) | 6.8 (4.1,10.5) |
|     Index † | 1.8 (1.1,2.7) | 2.2 (1.4,3.5) | 2.3 (1.4,3.8) |
|     Nadir platelet † | 196 (159,240) | 194 (153,236) | 193 (154,231) |
|     Platelet count < 100,000 | 24 (3.5%) | 29 (4.2%) | 42 (5.9%) |

\* $p = 0.001$
\*\*$p < 0.001$
† Median (25th, 75th percentiles).

The frequency of secondary clinical events was low and no major differences in these outcomes were observed according to treatment. The 30-day rates of heart failure (2.3%, 2.4%, 2.3%), sustained hypotension (3.0%, 3.6%, 4.1%), ventricular fibrillation (3.0%, 2.6%, 3.4%) and clinical occurrence of ischemia (21%, 17%, 18%) were similar in the placebo, bolus and bolus and infusion groups, respectively.

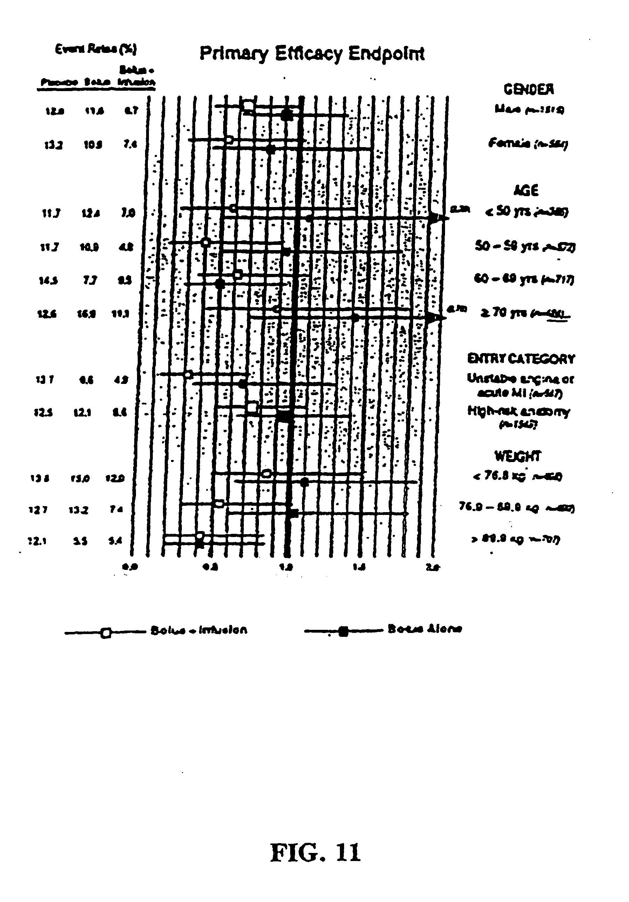

When the treatment effect was evaluated in subgroups (see Figure 11) defined according to whether patients were enrolled with an acute infarction, unstable angina, or high risk anatomy, the benefit of c7E3 Fab was present in all three enrollment categories. Similarly, the treatment effect was homogeneous across subgroups defined by age and by gender. As a function of weight, the efficacy benefit of c7E3 Fab bolus plus infusion was present across the entire spectrum of patients, although the treatment effect was more pronounced in heavier patients.

The risk of major bleeding was increased in lighter weight patients as compared with heavier patients for both the bolus only and the bolus plus infusion regimens. In the lightest tertile of patients, major bleeding occurred in 21 versus 15 versus 7 percent, respectively, for bolus plus infusion, bolus only and placebo-treated patients, whereas in the heaviest tertile, the corresponding bleeding rates were 8, 7 and 7 percent. Transfusion of packed red blood cells in the lightest tertile of patients occurred in 24, 20 and 11 percent, respectively, for bolus plus infusion, bolus only and placebo-treated patients, while in the heaviest tertile, the corresponding rates were 11, 7 and 4 percent.

Discussion

These results confirm the importance of platelets, presumably platelet-derived mediators, and platelet function in the occurrence of acute ischemic events in patients undergoing percutaneous transluminal coronary angioplasty. The EPIC trial was designed specifically to enroll a cohort of patients at high risk for abrupt vessel closure (reocclusion) and its attendant complications. Analysis of previous databases indicated that some patients could be classified as high risk based on clinical indicators of thrombus in the vessel, such as acute infarction (Stack, R.S. et al., J. Am. Coll. Cardiol.,

11:1141-49 (1988)), severe unstable angina (Myler, R.K. et al., Circulation, 82 (Suppl II):II-88-II-95 (1990)) or the angiographic appearance of thrombus (Sugrue, D.D. et al., Br. Heart J., 56:62-66 (1986); Hettleman, B.D. et al., J. Am. Coll. Cardiol., 15:154A (1990)). Other patients were at high risk because of mechanical factors, such as small vessel caliber, diffuse disease or unfavorable vessel morphology (Sinclair, I.N. et al., Am. J. Cardiol., 61:61G-66G (1988); Ruocco, N.A. et al., Am. J. Cardiol., 69:69-76 (1992); Ellis, S.G. et al., Am. J. Cardiol., 63:30-4 (1989)). A substantial number of both types of patients were enrolled in the trial, allowing us to provide some insight into the relevance of the inhibition of platelet aggregation for each type of patient. The inclusion of these high-risk patients was expected to result in an ischemic event rate of 15% in the placebo-treated patients, despite the use of aspirin and high-dose heparin in every patient; this expected event rate was nearly realized.

The administration of c7E3 Fab resulted in a 35% reduction in the composite event rate, with the primary effect observed in the reduction in nonfatal myocardial infarction, need for emergency angioplasty and emergency coronary bypass surgery. The bolus of c7E3 Fab produced a delay in the onset of these events, corresponding to length of time that platelet aggregation is affected. Four to six hours after the bolus, however, ischemic events began to occur. This interval corresponds to the period following in which platelet aggregation has been shown to return to approximately 50% of its basal value following a bolus of murine 7E3 Fab.

In addition to delaying events, the combined bolus and infusion treatment, which produces profound and prolonged platelet inhibition (see Example 4), also *prevented* acute ischemic events from occurring. The composite endpoint provides an overall estimate of the impact of this therapeutic approach on ischemic events in the periangioplasty period.

One of the most important findings from this trial is the consistency of the event reduction profile across the various endpoints. The reduction in myocardial infarction was substantial and consistent with the simultaneous reduction in the clinical need for subsequent emergency procedures. The classification of nonfatal infarction has become a major issue in the evaluation of percutaneous coronary interventions. Elevations of creatine kinase MB isoenzyme above the upper limit of normal are common, ranging from 4 to 21% of patients in reported series (Klein, L.W. et al., J. Am. Coll. Cardiol., 17:621-6 (1991); Hunt, A.C. et al., Eur. Heart J., 12:690-3 (1991; Pauletto, P. et al., Am. J. Cardiol., 69:999-1000 (1987); Spadaro, J.J. et al., Cath. Cardiovasc. Diagn., 12:230-4 (1986)). When these enzyme elevations are not uniformly associated with discrete clinical events, no association with long term adverse outcomes has yet been documented. Thus, the prevention of isolated enzyme elevations without associated clinical ischemic events may not be prognostically meaningful. In order to assure objectivity in this subtle area, enzymes and electrocardiograms were systematically collected, a blinded endpoints committee was used, and at least a three-fold increase in myocardial specific enzymes to classify an event as a myocardial infarction was required. The finding that c7E3 Fab reduced the full spectrum of myocardial infarctions, including those associated with moderate enzyme elevations, large enzyme elevations and Q wave development, provides reassurance of the clinical importance of the events that were prevented, especially since the need for emergency coronary revascularization procedures were also reduced.

Although no effect on mortality was expected or observed, the occurrence of 3 deaths in the bolus and infusion group in patients who never received the drug should be noted. These deaths were counted in the primary analysis according to the intention-to-treat principle. All other patients who died actually received their assigned therapy. Given the low mortality associated with angioplasty, more than 20,000 patients would be required to detect a 25% beneficial or detrimental treatment effect on mortality.

The beneficial effect of glycoprotein IIb/IIIa receptor blockade on clinical endpoints in the setting of high-risk angioplasty is convincing, and is consistent with the positive results of a recent initial trial using the same antibody in patients undergoing angioplasty in the setting of refractory unstable angina (Simoons, M.L. et al., J. Am. Coll. Cardiol., 21:269A (1993)),

With this being the first large scale trial of IIb/IIIa receptor blockade, there was concern about inducing thrombocytopenia. However, there was only a small, clinically unimportant increase in thrombocytopenia with c7E3 Fab. In particular, analysis revealed that more patients in the bolus plus infusion treatment group (5.2%) experienced thrombocytopenia (platelet count < 100,000/$\mu$L) than in the bolus (3.6%) or placebo (3.4%) treatment groups. Thus, there was an increase in incidence of thrombocytopenia (platelet count < 100,000/$\mu$L) in the bolus plus infusion treatment group compared with the placebo group (pairwise p = 0.062). Severe thrombocytopenia (platelet count < 50,000/$\mu$L) occurred in 11 (1.6%) of the patients in the bolus plus infusion treatment group and 5 (0.7%) of the patients in the placebo treatment group. Only 4 patients (< 1%) in each of the bolus plus infusion and placebo treatment groups had both severe thrombocytopenia and serious, life-threatening, or fatal adverse events. All episodes of thrombocytopenia were transient and typically occurred during the first few days.

A significant increase in bleeding complications and transfusion was observed in treated patients. This increase occurred primarily as a result of bleeding at the femoral puncture site and did not result in a marked difference in nadir hematocrits or life-threatening complications among the three groups. The trends remained the same whether or not surgical patients were included in the analysis. In this blinded study, the uncertainty about which treatment was being given may have led to a lower threshold for transfusion at some sites due to concern about the ability to stop the bleeding. Our previous experience has demonstrated that a refined protocol for management of bleeding and administration of transfusions in patients treated with thrombolytic therapy can effectively reduce the administration of blood products (Wall, T.C. et al., J. Am. Coll. Cardiol., 21:597-603 (1993)).

The interplay between treatment benefit versus bleeding risk as a function of weight is more complex than was expected. Although the primary event rate and the risk of major bleeding did not vary appreciably according to weight in placebo-treated patients, there was a clear trend with decreasing weight toward a higher proportion of primary outcome events and a higher proportion of major bleeds in both the c7E3 Fab bolus only patients and the bolus plus infusion patients.

Judgment about the clinical utility of this approach to blocking the glycoprotein IIb/IIIa receptor in patients before, during or after percutaneous revascularization depends on the relative value of avoiding ischemic events versus that of giving blood products. In the high-risk patients enrolled in this trial, the balance appears to be favorable. The prognostic implications of acute myocardial infarction or emergency repeat revascularization are serious, and fortunately the risk of transfusion continues to decline (Donahue, J.G. et al., N. Engl. J. Med., 327:369-73 (1992); Dodd, R.Y. N. Engl. J. Med., 327:419-21 (1992); Nelson, K.E. et al., Ann. Intern. Med., 117:554-9 (1992)). Efforts to employ practice algorithms to reduce unnecessary bleeding and transfusions and to more effectively dose antithrombotic therapy, including antithrombin and antiplatelet effects, should further enhance the clinical benefits observed in this trial. In practice, potent, parenterally administered antithrombotic agents in instrumented patients must focus on weight adjusted dosing of antithrombotic therapy (e.g., heparin doses were not weight adjusted here). Use of more detailed mechanistic evaluation and protocols that define approaches to reduce bleeding to be applied consistently across participating centers can yield more information about the occurrence of bleeding complications.

The broad consistency of treatment effect across the patient population is strong evidence that although in some cases abrupt closure may be primarily thrombotic or primarily mechanical, thrombus formation has a more important role in many patients. The delay in events engendered by the bolus therapy and the prevention of events by the bolus and infusion implies that in most situations the surface of the disrupted artery has lost much of its thrombogenicity by 18 to 24 hours after the procedure. Therapeutic approaches should take into account the need for sustained antithrombotic effect in patients with a significant risk of abrupt vessel closure.

In summary, this trial demonstrates a beneficial effect of sustained blockade of the glycoprotein IIb/IIIa receptor in patients undergoing high-risk percutaneous revascularization procedures in reducing and/or preventing reocclusion or abrupt closure. Although this benefit was achieved at the risk of increased bleeding, consideration of the overall clinical outcomes favors this approach in the treatment of patients known to be at high risk of acute ischemic complications, based on clinical and angiographic predictors prior to the procedure. The trial provides the first validation of a meaningful therapeutic approach to inhibition of the function of cell integrins, paving the way for other selectin and integrin targets for biotechnology in the future, as well as non-antibody or peptide approaches to this specific IIb/IIIa glycoprotein.

Example 7

Reduction of Clinical Restenosis Following Coronary Intervention with Early Administration of Anti-GPIIb/IIIa Chimeric Antibody Fragment

The process of restenosis following balloon angioplasty and percutaneous coronary interventions is extremely common, leading to recurrence of anginal symptoms and the need for repeat revascutarization procedures in more than 25% of cases within 6 months, at an overall cost of more than $2 billion per year in the United States (Popma, J.J. et al., Circulation, 84:1426-1436 (1991); Topol, E.J. et al., Circulation, 87:1489-1497 (1993); Herrman, J.-P.R. et al., Drugs, 46:249-262 (1993)). The major biologic trigger of restenosis is vascular injury, induced by the inflated balloon or alternative device at the site of intervention, and accompanied by platelet-thrombus formation and change of phenotype of medial smooth muscle cells from their resting, contractile state to one capable of migratory, proliferative and secretory function (Forrester, J.S. et al., J. Am. Coll. Cardiol., 17:758-769 (1991); Ip, J.H. et al., J. Am. Coll. Cardiol., 17:77B-88B (1991); Casscells, W., Circulation, 86:723-729 (1993)). Although various pharmacologic agents have been successful in experimental models in modulating the characteristic myointimal growth that occurs after vascular injury and small studies have suggested an angiographic benefit, there has not yet been a large-scale clinical trial in patients that demonstrates an effective agent and, until now, there was no known pharmacologic treatment to reduce the likelihood of this event (Popma, J.J. et al., Circulation, 84:1426-1436 (1991); Herrman, J.-P.R. et al., Drugs, 46:249-262 (1993); Mercator Study Group, Circulation, 86:100-110 (1992)).

Coronary angioplasty is routinely performed with adjunctive oral aspirin and intravenous heparin. This antithrombotic approach, however, is only weakly inhibitory of platelet aggregation. A variety of agonists including thrombin, collagen, and adenosine diphosphate can stimulate platelets even in the face of aspirin therapy. Molecular biology of the platelet has elucidated the glycoprotein IIb/IIIa integrin as the receptor responsible for platelet aggregation (Plow, E.F. et al., Prog. Hemostas. Thromb., 296:320-331 (1988); Coller, B.S., J. Clin. Invest., 76:101-108, (1985)). The chimeric 7E3 antibody Fab fragment binds selectively to the platelet IIb/IIIa integrin. After initial studies confirmed preliminary safety and efficacy of the chimeric monoclonal antibody Fab fragment, a multicenter, double-blind, placebo-controlled trial in 2,099 patients was, performed (see Example 6). In addition to the primary efficacy endpoint of a reduction of major ischemic events in the acute phase, representing suppression of abrupt closure (see Example 6), it was determined that c7E3 is capable of reducing the incidence of clinical restenosis, as defined by ischemic events or the need for repeat revascularization during the subsequent 6-month follow-up phase.

Methods

Details of the study population and protocol are described in Example 6. To recapitulate, patients were eligible if they were undergoing coronary angioplasty or directional atherectomy and had an evolving or recent myocardial infarction, unstable angina, or high risk angiographic lesion morphology as defined by the American Heart Association/ American College of Cardiology criteria (ACC/AHA Task Force Report, J. Am. coll. Cardiol., 12:529-545 (1988)). Exclusion criteria were bleeding diathesis, age $\geq$ 80 years, stroke within 2 years, or major surgery within 6 weeks. The protocol was approved by the Institutional Review Board at all 56 participating sites and informed consent was obtained in all patients.

Patients received oral aspirin (325 mg per day), with the first dose administered at least 2 hours before the procedure. Intravenous heparin was given during the procedure to achieve an activating clotting time of at least 300 seconds. In addition to aspirin and heparin, patients were randomly assigned to one of three alternative regimens: (1) placebo bolus and placebo 12 hour infusion; (2) active c7E3 (Centocor, Malvern, PA) bolus at 0.25 mg/kg and placebo 12 hour infusion; or (3) active c7E3 bolus at the same dose followed immediately by c7E3 infusion at 10 $\mu$g/min for 12 hours. The bolus was given at least 10 minutes prior to the coronary intervention procedure.

The primary endpoint was the 30-day composite incidence of death from any cause, myocardial infarction, coronary artery bypass surgery for acute ischemia, repeat percutaneous coronary intervention for acute ischemia, need for an endoluminal stent or insertion of an intra-aortic balloon pump to treat ischemia. All of these events were reviewed by an independent Clinical Endpoints Committee which remained blinded to treatment throughout the study and required consensus of at least two reviewers for classification.

During 6 month follow-up, the double-blind was preserved. In addition to subsequent ischemic events of death or nonfatal myocardial infarction, patients were followed to determine the need for a repeat revascularization procedure consisting of percutaneous coronary interventions, or coronary artery bypass surgery, or both. Unlike the acute phase endpoint, stenting or the use of an intra-aortic balloon pump was not included as an outcome since the focus was the need for revascularization procedures rather than surrogates for a sudden ischemic event. The criteria for diagnosis after hospital discharge of myocardial infarction required either a new significant Q-wave $\geq$ 0.04 sec in duration or with a depth $\geq$ 1/4 the corresponding R wave amplitude in two or more contiguous leads; or creatine kinase or creatine kinase myocardial band greater than twice the upper limit of normal. Resvascularization data were collected as well as whether the original target vessel was subjected to repeat surgical or percutaneous revascularization. Follow-up was 97.2% complete.

To assess whether the 6 month outcomes were separate from the acute phase results, the analysis included all events from baseline to 6 months, events occurring after the 30 day endpoint in patients with an initial successful intervention (defined as achievement of a final stenosis less than 50% according to the reading of the clinical investigator and without an ischemic complication), and events occurring after 48 hours in patients with an initial successful intervention. The 30-day endpoint was prospectively selected because of precedence in many cardiovascular intervention trials. The 48 hour cutoff was used because it is known that, by definition, nearly all abrupt closure events that occur following coronary intervention take place within this time window (Detre, K.M. et al., J. Am. Coll. Cardiol., 13:230A (1989); Lincoff, A.M. et al., J. Am. Coll. Cardiol., 19:926-938 (1992); de Feyter, P.J. et al., Circulation, 83:927-936 (1991)).

Randomization was performed via a telephone call to the Duke Coordinating Center and stratified by study site and whether the patient was having an acute myocardial infarction. Data were collected by study coordinators on a separate 6 month case report form that was quality assured by source documentation by blinded study monitors prior to data entry. The sponsor remained blinded to the follow-up results until all patients had completed follow-up, the events were adjudicated by the Endpoints Committee, and the database was complete.

Statistical Analysis

All treatment comparisons were performed with the intention-to-treat principle. Event rates were estimated using the Kaplan-Meier method (Kaplan, E.L. and P. Meier, J. Am. Stat. Assn., 53: 457-481 (1958)) and survival curves were used to graphically display the results. A test for dose-response in event rates from placebo to bolus to bolus and infusion (with scores 0, 1, and 2, respectively) was performed using the generalized log-rank statistic. Pairwise comparisons between placebo and each of the c7E3 arms were also performed using the log-rank statistic. Proportional hazards (Cox) models were fit to examine possible associations between baseline character-istics and outcome. These were done with all treatment groups combined with the treatment differences modelled, and separately by treatment group to examine for differences between treatment arms. In addition, proportion hazards regression (Cox) models were fit for the all event composite endpoint after the 48 hour follow-up to examine factors which might be associated with late events or treatment effects. The factors included in this analysis were treatment, single lesion or multiple lesions revascularized, duration of the procedure, myocardial infarction or unstable angina at baseline or other high-risk entry criteria, gender, age $\geq$ 65 years or < 65 years, weight, and diabetes mellitus.

Results

Enrollment began December 1, 1991 and ended November 18, 1992 with 2099 patients. Features of the entire study cohort are presented above (see Example 6, Table 10). The baseline characteristics of the patients who had a successful initial angioplasty or atherectomy procedure, thus being eligible for subsequent clinical restenosis, are presented in Table 16. There were no significant differences in baseline features of the patients who had a successful initial procedure by treatment assignment.

Table 16

| Demographic Features of Patients with a Successful Initial Procedure | | | |
|---|---|---|---|
| | Placebo (N = 609) | Bolus (N = 605) | Bolus and Infusion (N = 620) |
| Age (years) | 60.0 $\pm$ 10.3 | 59.7 $\pm$ 10.4 | 60.2 $\pm$ 10.6 |
| Male (%) | 430 (71.7%) | 434 (71.7%) | 445 (71.8%) |
| Weight (kg) | 84.9 $\pm$ 16.0 | 83.5 $\pm$ 16.5 | 83.3 $\pm$ 15.7 |
| Risk Factors (%) | | | |
|     Diabetes | 155 (25.8%) | 146 (24.1%) | 139 (22.4%) |
|     Hypertension | 321 (53.7% | 329 (54.8%) | 314 (50.8%) |
|     Elevated Cholesterol | 316 (52.7%) | 337 (55.7%) | 324 (52.3%) |
|     Smoking | 377 (64.7%) | 428 (71.8%) | 423 (69.5%) |
| Vascular Disease (%) | | | |
|     Peripheral | 47 (7.9%) | 52 (8.7%) | 52 (8.5%) |
|     Cerebral | 21 (3.5%) | 19 (3.1%) | 27 (4.4%) |
| Previous MI (%) | | | |
|     None | 275 (45.8%) | 239 (39.5%) | 253 (40.8%) |
|     >30 days | 109 (18.2%) | 126 (20.8%) | 128 (20.6%) |
|     8-30 days | 45 (7.5%) | 55 (9.1%) | 57 (9.2%) |
|     < 8 days | 171 (28.5%) | 185 (30.6%) | 182 (29.4%) |
| Prior Procedure (%) | | | |
|     Angioplasty | 145 (24.3%) | 121 (20.0%) | 139 (22.6%) |
|     Bypass Surgery | 88 (14.7%) | 85 (14.0%) | 95 (15.3%) |
| Coronary Anatomy (%) | | | |
|     1 Vessel Disease | 337 (56.2%) | 324 (53.6%) | 354 (57.1%) |
|     2 Vessel Disease | 170 (28.3%) | 198 (32.7%) | 191 (30.8%) |
|     3 Vessel Disease | 93 (15.0%) | 83 (13.7%) | 75 (12.1%) |
| Type of Procedure | | | |
|     Balloon | 540 (90.0%) | 549 (90.7%) | 561 (90.5%) |

(continued)

| Type of Procedure | | | |
|---|---|---|---|
| Atherectomy | 37 (6.2%) | 27 (4.5%) | 34 (5.5%) |
| Both | 23 (3.8%) | 29 (4.8%) | 25 (4.0%) |
| Target Vessel | | | |
| LAD | 241 (40.2%) | 229 (37.9%) | 262 (42.3%) |
| LCX | 144 (24.0%) | 159 (26.3%) | 165 (26.6%) |
| RCA | 234 (39.0%) | 253 (41.8%) | 219 (35.3%) |
| Left Main | 4 (0.7%) | 1 (0.2%) | 3 (0.5%) |
| Graft | 35 (5.8%) | 35 (5.8%) | 43 (6.9%) |

Patients receiving c7E3 bolus or the bolus and infusion had a significant increase in bleeding complications, predominantly in the first 48 hours, with approximate doubling of transfusion rate (placebo 7%, bolus only 13%, bolus and infusion 15%, P < 0.001). The 12 hour infusion was not fully completed in 48 patients (7.0%) receiving placebo, 85 patients (12.5%) assigned to bolus only, and 107 patients (15.8%) in the bolus and infusion group.

There were no signifi ant increases in thrombocytopenia with c7E3 and no hypersensitivity or allergic effects were manifest. Positive human anti-chimeric antibody (HACA) responses occurred in 5.2% of the bolus-treated patients and 6.5% of the bolus plus infusion-treated patients. Most of the patients with a positive HACA response had low-titer responses. All of the 32 patients who had a positive HACA response in the bolus treatment group had a titer $\leq$ 1:1600. Thirty-four of the 40 patients in the bolus plus infusion treatment group who had a positive HACA response had a titer $\leq$ 1:1600. There were 6 patients in the bolus plus infusion treatment gorup, but none in the bolus treatment goup, with HACA titers ranging between 1:6400 and 1:51200.

At 30-days, there was a 35% reduction of major ischemic events (death, myocardial infarction, urgent revascularization) for patients treated with c7E3 bolus and infusion (8.3%) compared with placebo treatment (12.8%, P = 0.009) (see Example 6, Table 13). The 6-month data is presented in Table 17, which shows the outcomes of death, myocardial infarction, and the need for coronary artery bypass surgery or repeat coronary intervention, with target vessel revascularization for (a) all patients enrolled, (b) those patients with a successful procedure, without an ischemic complication in the first 48 hours after enrollment, and (c) for the cohort of patients who had an initially successful procedure and did not have an event in the first 30 days. At 6 months, there was a 23% reduction of ischemic events and revascularization (27% versus 35%, P = 0.001; see Table 17, All Patients Enrolled, Composite Death, MI, CABG, PTCA). The favorable long-term effect was chiefly due to less need for bypass surgery or repeat angioplasty in patients with an initial successful procedure, as repeat target vessel revascularization was lessened by 26% for c7E3 bolus and infusion (16.4%) versus placebo treatment (22.3%), P = 0.007; see Table 17). The bolus only patient group had an intermediate outcome which was not significantly better than placebo by the criteria used in this trial.

In a separate analysis, 6 month data of all *patients* not experiencing a primary efficacy event in the first 30 days were studied. The results of this analysis are shown in Table 18. These data suggest a 21% reduction of the need for repeat revascularization procedures upon the acute phase administration of c7E3 bolus plus infusion.

Table 17

| Outcomes at Six Months | | | | |
|---|---|---|---|---|
| | Placebo (N=696) | Bolus (N=695) | Bolus + Infusion (N=708) | P-Value |
| All Patients Enrolled | | | | |
| Death (%) | 3.4 | 2.6 | 3.0 | 0.827 |
| MI (%) | 10.5 | 8.0 | 6.9 | 0.016 |
| CABG (%) | 11.0 | 9.7 | 9.4 | 0.339 |
| PTCA (%) | 20.8 | 19.8 | 14.3 | 0.001 |
| Composite Death, MI, CABG, PTCA (%) | 35.0 | 32.4 | 26.9 | 0.001 |
| Any Revascularization (CABG/ PTCA) (%) | 29.4 | 27.2 | 23.1 | 0.008 |
| Target Vessel Repeat Revascularization (%) | 22.3 | 20.8 | 16.4 | 0.007 |

(continued)

| All Patients With a Successful Procedure at 48 Hours | | | | |
|---|---|---|---|---|
| | (N=606) | (N=618) | (N=639) | |
| Death (%) | 2.7 | 2.3 | 3.0 | 0.664 |
| MI (%) | 2.6 | 2.4 | 2.5 | 0.860 |
| CABG (%) | 7.6 | 7.4 | 7.0 | 0.701 |
| PTCA (%) | 16.4 | 17.2 | 11.5 | 0.110 |
| Composite Death, MI, CABG. PTCA (%) | 25.3 | 24.1 | 19.1 | 0. 7 |
| Any Revascularization (CABG/ PTCA) (%) (All patients) | 23.0 (N=636) | 22.5 (N=655) | 18.0 (N=666) | 0.025 |
| Target Vessel Repeat Vascularization (%) | 18.9% | 18.4% | 15.6% | 0.134 |
| All Patients With a Successful Procedure and No Event at 30 Days | | | | |
| | (N=549) | (N=576) | (N=598) | |
| Death (%) | 1.7 | 1.3 | 1.5 | 0.789 |
| MI (%) | 2.0 | 1.9 | 1.7 | 0.716 |
| CABG (%) | 5.6 | 5.7 | 4.7 | 0.438 |
| PTCA (%) | 12.5 | 14.4 | 10.0 | 0.180 |
| Composite Death, MI, CABG, PTCA (%) | 19.2 | 20.1 | 15.2 | 0.072 |
| Any Revascularization (CABG/ PTCA) (%) | 18.4 | 18.3 | 14.6 | 0.077 |
| Target Vessel Repeat Vascularization (%) | 16.8 | 16.4 | 14.3 | 0.265 |

**Table 18**
**Outcomes at 6 Months in All Patients Having No event at 30 Days**

| | Placebo | Bolus Only | Bolus + Infusion | P-value |
|---|---|---|---|---|
| | (N=601) | (N= 611) | (N=643) | |
| Death (%) | 1.8 | 1.3 | 1.0 | 0.38 |
| MI (%) | 2.4 | 2.5 | 2.2 | 0.85 |
| CABG (%) | 5.9 | 5.5 | 4.4 | 0.24 |
| PTCA (%) | 13.8 | 14.8 | 10.7 | 0.12 |
| CABG/PTCA (%) | 18.4 | 18.3 | 14.6 | 0.077 |
| Composite: Death, MI MI, CABG, PTCA (%) | 19.5 | 19.6 | 15.4 | 0.059 |
| Composite from Baseline (including 0-30 days) (%) | 34.8 | 32.2 | 26.9 | 0.001 |

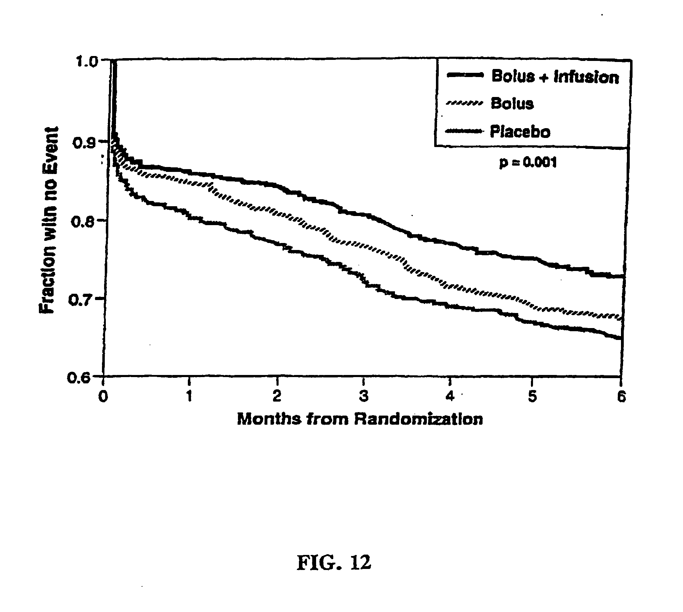

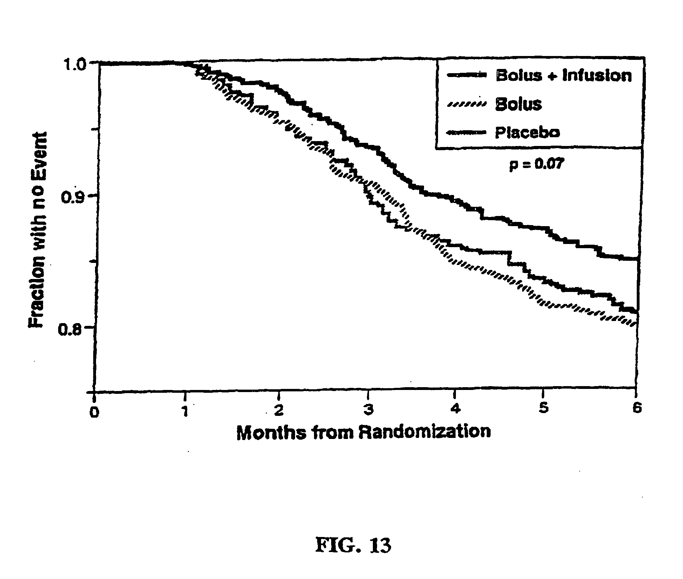

The data for all events (death, nonfatal infarction or need for coronary revascularization) for all patients who were entered into the trial are also presented in Figure 12. The data for patients who had a successful intervention and no events until 30 days are also shown in Figure 13.

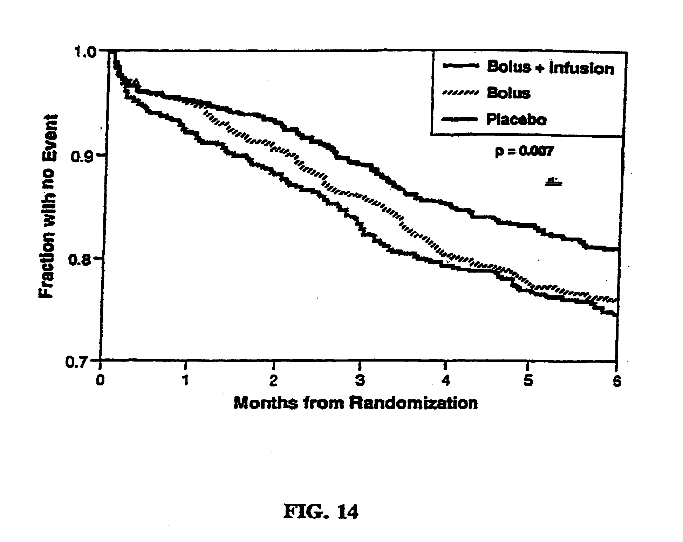

Of the acute phase endpoint, 81% of events had occurred by 48 hours. This was similar across treatment groups (82.0% placebo, 79.7% bolus, 81.4% bolus and infusion). By considering events after the first 48 hour in patients with initially successful intervention, the elective target vessel revascularization in the first 30 days is identified. As shown in Figure 14, there was little difference for subacute ischemic events between c7E3 bolus and the bolus plus infusion groups until after the 30 day endpoint.

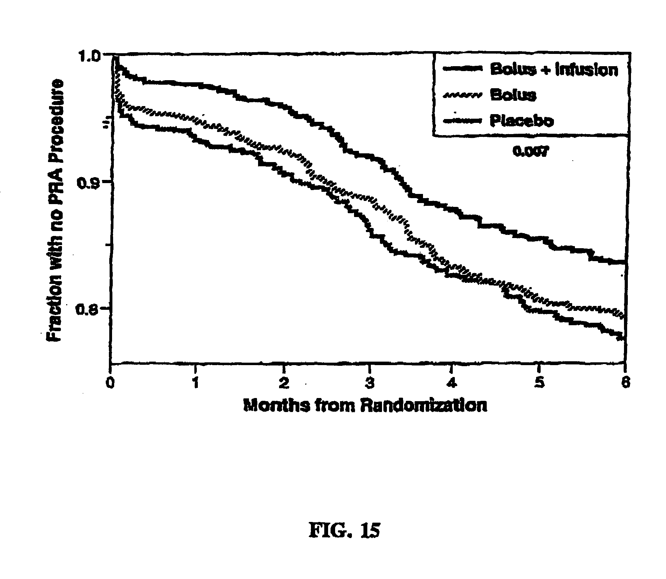

Instead of the composite of death, myocardial revascularization, and any revascularization, which would include vessels not initially approached, it is helpful to focus on target vessel revascularization only. For the entire patient cohort during the 6 month period, there was a significant 26% reduction in target vessel revascularization for the bolus and infusion group patients compared with the other treatment groups (see Figure 15). Of note, little effect of the bolus alone on target vessel revascularization during follow-up was observed under the conditions of the trial.

Subgroup analysis compared patients who at baseline were diagnosed to have acute coronary syndromes (unstable angina, recent or acute myocardial infarction) with the remaining patients who had stable angina but high-risk angiographic morphology (Table 19). This revealed a significant effect in reduction of composite events for both subgroups, but the reduction in need for repeat coronary interventions was only significant in the stable angina patients (Table 19). This finding was consistent, whether events were analyzed from baseline or after 48 hours in the patients with a successful procedure.

Table 19

| Subgroup Analysis of Events for Acute Coronary Syndrome Versus Stable Angina Patients | | | | |
|---|---|---|---|---|
| **Events from Baseline to Six Months** | | | | |
| | Placebo | Bolus | Bolus Infusion | P |
| Acute Coronary Syndrome | | | | |
| N<br>Composite Event<br>Re-PTCA | 288<br>33.1%<br>20.2% | 306<br>28.8%<br>16.9% | 299<br>25.5%<br>15.4% | <br>0.039<br>0.129 |
| Stable Angina | | | | |
| N<br>Composite<br>Re-PTCA | 408<br>36.3%<br>21.3% | 389<br>35.3%<br>22.1% | 409<br>28.0%<br>13.5% | <br>0.012<br>0.004 |
| **Events from 48 Hours to Six Months** | | | | |
| Acute Coronary Syndrome | | | | |
| N<br>Composite Event<br>Re-PTCA | 252<br>23.5%<br>14.8% | 277<br>21.4%<br>15.2% | 271<br>17.8%<br>11.9% | <br>0.094<br>0.325 |
| Stable Angina | | | | |
| N<br>Composite Event<br>Re-PTCA | 354<br>26.6%<br>17.5% | 341<br>26.4%<br>18.7% | 368<br>20.0%<br>11.1% | <br>0.034<br>0.013 |

Discussion

The current findings from a large-scale, multicenter, randomized trial support a reduction of clinical events reflecting less restenosis for patients undergoing coronary intervention who received a bolus and infusion of platelet IIb/IIIa integrin blockade. The extent of the benefit at 6 months was approximately 23% reduction of overall ischemic events, including death, nonfatal myocardial infarction, and need for revascularization, and a 26% decrease in target vessel revascularization. These results extend the benefit of c7E3 bolus and 12 hour infusion from reducing abrupt closure and acute phase adverse outcomes to a diminished need for subsequent coronary revascularization procedures.

The monoclonal Fab fragment used in the trial has potent affinity for binding to the platelet IIb/IIIa surface integrin with minimal dissociation. Previous studies in patients undergoing angioplasty with c7E3 have shown that even after the infusion of the antibody is terminated, there is persistent occupancy of the IIb/IIIa binding sites for at least 36 to 48 hours, and evidence of inhibition of platelet aggregation for at least 72 hours (see Example 4; see also, Ellis, S.G. et al., Cor. Art. Dis., 4:1675-175 (1993); Tcheng, J.E. et al., Circulation, 88: (1993)). While both of these effects diminish and revert to baseline over time, the duration of the c7E3 inhibitory effect on platelet aggregation is interesting in view of the observation that the bolus plus placebo infusion did not have a clinically meaningful effect on either acute or 6 month outcomes under the criteria and conditions used in this trial. This observation suggests that reduction of acute ischemic events or clinical restenosis via administration of anti-GPIIb-IIIa antibody may require a more prolonged exposure to agent (e.g., as achieved by bolus plus infusion of drug), and that more prolonged suppression of the GPIIb/IIIa integrin could be accompanied by further improvement of outcomes.

It is also noteworthy that c7E3 has been reported to bind to the vitronectin receptor (Hynes, R.O., Cell, 69: 11-25 (1992)), probably because this receptor contains the $\beta_3$ component of GPIIb/IIIa. This integrin (vitronectin) may have a role in modulating stenosis or restenosis, and binding of anti-GPIIb/IIIa to the vitronectin receptor may contribute to the effect observed. Other GPIIb/IIIa receptor inhibitors have varying degrees of specificity for the target and for homologous integrins (Sutton, J. et al., Clinical Research AFCR, 41:118A (1993)). comparative studies can be performed to dissect the contribution of these molecular interactions to clinical outcome.

In the current trial, systematic six month repeat angiography to quantitatively determine the extent of renarrowing in the treatment groups was not carried out. Although repeat angiography has been conducted in many restenosis trials (Forrester, J.S. et al., J. Am. Coll. Cardiol., 17:758-769 (1991); Ip, J.H. et al., J. Am. Coll. Cardiol., 17:77B-88B (1991); Casscells, W., Circulation, 86:723-729, (1993); Topol, E.J. et al., N. Engl. J. Med., 329:228-233 (1993); Adelman, A.G. et al., N. Engl. J. Med., 329:228-233 (1993); Serruys, P.W. et al., Circulation, 84:1568-1580 (1991)), it has a major drawback because diagnosis of target vessel stenoses in asymptomatic patients often leads to repeat procedures that would not have occurred in practice. In contrast, the present design offers a simulation of clinical practice in a large population of patients. It is the net objective of restenosis trials to demonstrate a significant reduction in the need for repeat revascularization procedures, because angiographic benefit per se, which has been documented in some recent restenosis trials, is not singularly adequate or completely clinically relevant. Furthermore, since death and myocardial infarction are unusual in patients following percutaneous coronary intervention, the main outcome that should be modulated by an effective pharmacologic intervention is repeat target vessel revascularization, as observed here. Because the patients in the study were treated similarly except for the study drug bolus and infusion, including careful preservation of the double-blind until the follow-up was complete, it reasonable to conclude that a reduction in renarrowing explains the observed clinically meaningful benefit.

This represents the first large-scale randomized trial to demonstrate a clinically meaningful reduction in the need for subsequent revascularization procedures that can be interpreted as less clinical restenosis. This was achieved with a bolus and infusion of IIb/IIIa blockade and points towards true passivation of the injured vessel wall. Even through the infusion of c7E3 was only maintained for 12 hours, the agent has an extended anti-platelet effect for several days and there was no evidence of rebound in ischemic events in the acute phase. Importantly, the independent benefit of reduced target vessel revascularization at 6 months suggests a lasting effect of the acute c7E3 pharmacologic intervention and can be offered as clinical proof of vessel wall passivation.

The finding of less clinical evidence of restenosis with platelet IIb/IIIa blockade further emphasizes the role of the platelet-thrombus in restenosis, which has been raised as a potentially key pathway in the development of the post-angioplasty or endothelial injury neointimal lesion (Schwartz, R.S. et al., J. Am. Coll. Cardiol., 20:1284-1293 (1992); Topol, E.J. Mayo Clin. Proc., 68:88-90 (1993); Willerson, J.T. et al., Proc. Natl. Acad. Sci. USA, 88:10624-10628 (1991) ). While the proliferation of medial smooth muscle cells may also play a prominent role in restenosis (Forrester, J.S. et al., J. Am. Coll. Cardiol., 17:758-769 (1991); Ip, J.H. et al., J. Am. Coll. Cardiol., 17:77B-88B (1991); Casscells, W., Circulation, 86:723-729, (1993)), the results here suggest that potent anti-platelet and anti-thrombotic approaches to this important clinical phenomenon may be particularly fruitful. The trial supports the notion that the current approach of using aspirin during coronary intervention (Schwartz, L. et al., N. Engl. J. Med., 318:1714-1719 (1988)) as the sole anti-platelet agent is insufficient to antagonize the platelet response to vascular injury.

Example 8

Additional Findings From the Randomized, Double-Blind, Placebo-Controlled Trial

Sheath Size and Bleeding Complications

Bleeding complications were assessed in the EPIC trial (see Examples 6 and 7) to determine if sheath size during PTCA/DCA correlates with bleeding complications . Sheath and guiding catheter size were determined clinically by the interventionalist. Major bleeding episodes, groin bleeding, transfusion, vascular repair, nadir Hct were prospectively assessed.

Sheath size predicted groin bleeding even after adjustment for known bleeding predictors, including treatment assignment and heparin use during catheterization ($p = 0.0004$). The trend toward more vascular repair with larger sheath sizes was not significant ($p = 0.0004$). Major bleeds (10.5%), transfusions (11.8%), and nadir Hct (34) did not vary with sheath size. c7E3 Fab patients had more groin bleeds than non-7E3 patients (55% vs. 30%, $p < 0.0001$).

|  | | 6F-7.5F n = 375 | 8F-8.5F n = 1416 | 9F-11F n = 291 |
|---|---|---|---|---|
| Groin Bleed | | 140 (38%) | 674 (48%) | 147 (51%) |
| Vasc Repair | | 5 (1.3%) | 17 (1.2%) | 7 (2.4%) |

These results indicate that larger sheath sizes are associated with more groin bleeding but do not contribute to major bleeding complications of PTCA/DCA. c7E3 Fab is associated with increased groin bleeding, but this may be minimized by using smaller sheaths and guiding catheters.

Co-Incidence of Ischemic and Bleeding Events After Platelet GPIIb/IIIa Receptor Inhibition for Coronary Intervention

The EPIC (Evaluation of c7E3 in the Prevention of Ischemic Complications) trial (see Examples 6 and 7) demonstrated that therapy with c7E3 Fab, a potent platelet receptor GPIIb/IIIa antagonist, prevents ischemic complications in the setting of high risk coronary angioplasty (PTCA), but bleeding episodes requiring transfusion increased from 7% in the placebo group to 14% in c7E3 Fab group. To investigate this further, the relationship between bleeding indices (nadir hematocrit, bleeding index, change in hematocrit, units of packed red cells transfused) and the primary endpoint of the study (death, myocardial infarction, coronary artery bypass grafting (CABG) or PTCA for acute ischemia or insertion of a coronary stent for procedural failure) was investigated. A strong association between bleeding and the primary endpoint was noted (p = 0.0001 for all bleeding indices). This association was also present for each of the therapies: placebo, bolus c7E3 Fab, and bolus plus infusion c7E3 Fab. Thus, patients with significant bleeding were more likely to have ischemic complications. This strong relationship may be due to the increased bleeding associated with primary endpoint events (e.g. CABG). Alternatively, bleeding with associated hypotension may be a major contributor to post-procedural ischemic complications. In support of this, patients who developed hypotension (excluding hypotension after a primary outcome event) after successful PTCA were significantly more likely to have a primary outcome event and were also more likely to experience major bleeding and a primary outcome event.

|  | Hypotension | No Hypotension | P |
|---|---|---|---|
| Number | 239 | 1597 | - |
| Primary Endpoint (%) | 46(16.1) | 103(6.5) | <.001 |
| Endpoint + Major Bleed (%) | 18(39.9) | 10(9.7) | <.001 |

In conclusion, bleeding appears to induce ischemic complications in some patients, and measures to reduce bleeding (for example, modifications of heparin dosage) may further enhance the anti-ischemic efficacy of GPIIb/IIIa inhibition for coronary intervention.

Economic Benefits and Detriments of Aggressive Platelet Inhibition During High Risk Angioplasty

In the 2,100 patient EPIC randomized trial (see Examples 6 and 7), aggressive platelet inhibition with high dose c7E3 Fab was associated with a 35% decrease in subsequent death, reinfarction and recurrent ischemia during high-risk coronary angioplasty (PTCA), but twice as many post-PTCA major bleeds. To evaluate the economic consequences of this combination of clinical effects, a prospective economic substudy was conducted. Hospital costs (not charges) and resource use data were collected prospectively for each participant for six months following enrollment. Average baseline hospital costs for patients with an uncomplicated hospital course was $9300. The effects of major complications on baseline hospital costs were examined using a multivariable linear regression model:

$$\text{Mean hospital costs} = \$9065 + \$5923 * \text{urgent PTCA} +$$

$$\$28{,}219 * \text{urgent CABG} + \$3645 * \text{(re) infarction} +$$

$$\$3462 * \text{major bleed}$$

This model shows that by reducing urgent PTCA from 4.5% to 0.8%, urgent CABG from 3.6% to 2.4%, and (re) infarction from 8.6% to 5.2%, therapy with chimeric 7E3 fragment saved an average of $682 per patient over placebo therapy.

However, by doubling the rate of major bleeding from 7% to 14%, the therapy lost $242 of its potential cost advantage, resulting in a projected net cost savings per person of $440. The observed cost average difference between high dose c7E3 Fab (X = $10,970 $\pm$ 7,284) and placebo (X = $11,376 $\pm$ 12,555) was $406, agreeing closely with the model prediction.

Thus, by significantly reducing the ischemic complications of high risk PTCA, aggressive platelet inhibition with a chimeric anti-GPIIb/IIIa fragment offers both improved medical outcomes and a net cost savings. Measures to lower major bleeding rates upon administration of c7E3, while preserving treatment benefits, could yield a projected net cost savings of up to $700 per patient

Activated Clotting Time is Increased During Coronary Interventions with Platelet GPIIb/IIIa Antagonism

The activated clotting time (ACT) has been used during percutaneous transluminal coronary angioplasty (PTCA) to monitor the extent of thrombin inhibition and anticoagulation in an attempt to minimize untoward thrombotic events. With the introduction of potent platelet inhibitors, such as chimeric monoclonal antibody c7E3 Fab, the utility of measuring and regulating ACT during PTCA has not been examined. To this point, an effect of c7E3 on ACT was not known or suspected. The possible influence of platelet GPIIb/IIIa antagonism on procedural ACT was investigated. In the trial, 2099 subjects undergoing PTCA were randomized to receive placebo (n = 696) or GPIIb/IIIa antagonist, c7E3 Fab (n=1403). Despite receiving a similar amount of heparin and fewer patients receiving very high heparin doses (>14,000 units) compared to the placebo group, those receiving c7E3 Fab had a significantly higher (p < 0.001) ACT when corrected for bodyweight.

| Procedural Heparin | Placebo subjects % | c7E3 Fab subjects % |
|---|---|---|
| <10,000 units or infusion only | 112 (16) | 265 (19) |
| 10,000 units | 220 (32) | 497 (36) |
| 10,000-14,000 units | 141 (21) | 298 (22) |
| >14,000 units | 209 (31) | 316 (23) |

In conclusion, the activated clotting time is increased 35-40 seconds by the platelet GPIIb/IIIa antagonist c7E3 Fab. This has important implications for dosing conjunctive heparin therapy and performing coronary artery interventions in the setting of GPIIb/IIIa-directed therapy.

Gender Differences in a Trial Evaluating the Prevention of Ischemic Complications of Angioplasty with a Monoclonal Antibody to the Platelet GPIIb/IIIa Receptor

Gender differences were explored in a trial evaluating the prevention of ischemic complications of angioplasty with the use of c7E3 Fab, a monoclonal antibody to the platelet GPIIb/IIIa receptor (see Examples 6 and 7). Patients underwent percutaneous intervention (PTCA) and received one of three blinded treatments shortly preceding the intervention: c7E3 bolus followed by a 12 hour c7E3 infusion, c7E3 bolus only, or placebo.

Despite the fact that women tended to be older, lighter in weight, and had more cardiovascular risk factors than men, women and men did not differ in frequency of death (2.2% vs. 1.3%), MI (7.5% vs. 6.3%), emergency PTCA (2.6% vs. 3.1%), urgent bypass surgery (1.7% vs. 3.2%), stent placement, intraaortic balloon pump placement or a composite measure of those adverse ischemic events to 30 days (10.5% vs. 11.0%, p=0.74). Adjustment for known predictors of the composite measure (treatment assignment, weight, hypertension, peripheral vascular disease), as well as for any potential statistical interactions with gender did not alter these results. Treatment with a c7E3 Fab bolus and infusion decreased the frequency of ischemic events similarly in both sexes.

Women displayed more serious bleeding (12.6% vs. 9.8%), required transfusion (PRBC) more frequently (19.5% vs. 9.0%), and had a higher bleeding index ($\Delta$ hematocrit/3 + units PRBC, 2.4 vs. 1.9) than men. In a regression model to predict the bleeding index, despite adjustment for known predictors of bleeding (treatment allocations, age, weight, baseline hematocrit, hypertension) gender remained a statistically independent predictor (p = .0041).

In conclusion, women experienced more bleeding than men, but did not exhibit an excess of other adverse outcomes following high risk angioplasty and treatment with c7E3 Fab.

**Claims**

**1.** Use of a compound which binds selectively to glycoprotein IIb/IIIa, for the manufacture of a medicament for inhibiting restenosis in a human, wherein the compound is a chimeric immunoglobulin or immunoglobulin fragment comprising an antigen binding region of nonhuman origin from monoclonal antibody 7E3, and a human constant region.

2. Use of claim 1 for the manufacture of a medicament for inhibiting restenosis in a human patient having coronary artery disease.

3. Use of claim 1 or 2 for the manufacture of a medicament for inhibiting restenosis following a coronary artery intervention procedure in a human.

4. Use of claim 1 or 2 for the manufacture of a medicament for inhibiting restenosis following angioplasty in a human, for example percutaneous transluminal coronary angioplasty.

5. Use of any preceding claim, wherein said immunoglobulin or immunoglobulin fragment inhibits platelet aggregation.

6. Use of any preceding claim wherein the immunoglobulin fragment is an Fab, Fab' or F(ab')$_2$ fragment.

7. Use of a compound which binds selectively to glycoprotein IIb/IIIa, for the manufacture of a medicament for reducing or preventing non-acute ischemic complications of angioplasty in a human, wherein the compound is a chimeric immunoglobulin or immunoglobulin fragment comprising an antigen binding region of nonhuman origin from monoclonal antibody 7E3, and a human constant region.

8. Use of claim 7, wherein said immunoglobulin or immunoglobulin fragment inhibits platelet aggregation.

9. Use of claim 7 or 8 wherein the immunoglobulin fragment is an Fab, Fab' or F(ab')$_2$ fragment.

10. Use of any one of the preceding claims wherein the compound binds selectively to glycoprotein IIb/IIIa and the vitronectin receptor.

11. Use according to any one of the preceding claims wherein the medicament is for administration in conjunction with an agent selected from a thrombolytic agent, an anticoagulant, or an anti-platelet agent.

12. The use of claim 11 wherein the anti-platelet agent is heparin.

**Patentansprüche**

1. Verwendung einer Verbindung, die selektiv an Glykoprotein IIb/IIIa bindet, zur Herstellung eines Medikamentes zur Hemmung einer Restenose bei einem Menschen, wobei die Verbindung ein chimäres Immunglobulin oder ein Immunglobulin-Fragment mit einer Antigen-bindenden Region nicht menschlichen Ursprungs aus dem monoclonalen Antikörper 7E3 und mit einer menschlichen konstanten Region umfasst.

2. Verwendung nach Anspruch 1 zur Herstellung eines Medikamentes zur Hemmung der Restenose bei einem menschlichen Patienten mit einer Koronararterien-Erkrankung.

3. Verwendung nach Anspruch 1 oder 2 zur Herstellung eines Medikamentes zur Hemmung der Restenose im Anschluss an ein Koronararterien-Eingriffsverfahren bei einem Menschen.

4. Verwendung nach Anspruch 1 oder 2 zur Herstellung eines Medikamentes zur Hemmung der Restenose im Anschluss an eine Angioplastie bei einem Menschen, beispielsweise einer perkutanen transluminalen Koronar-Angioplastie.

5. Verwendung nach einem der vorhergehenden Ansprüche, wobei das Immunglobulin oder das Immunglobulin-Fragment die Blutplättchenaggregation hemmt.

6. Verwendung nach einem der vorhergehenden Ansprüche, wobei das Immunglobulin-Fragment ein Fab, Fab' oder F(ab')$_2$ Fragment ist.

7. Verwendung einer Verbindung, die selektiv an Glykoprotein IIb/IIIa bindet, zur Herstellung eines Medikamentes zur Reduzierung oder Prävention nicht-akuter ischämischer Komplikationen einer Angioplastie bei einem Menschen, wobei die Verbindung ein chimäres Immunglobulin oder Immunglobulin-Fragment mit einer Antigen-bindenden Region nicht menschlichen Ursprungs aus dem monoclonalen Antikörper 7E3 und eine humane konstante Region aufweist.

**8.** Verwendung nach Anspruch 7, wobei das Immunglobulin oder Immunglobulin-Fragment die Blutplättchenaggregation hemmt.

**9.** Verwendung nach Anspruch 7 oder 8, wobei das Immunglobulin-Fragment ein Fab, Fab' oder $F(ab')_2$ Fragment ist.

**10.** Verwendung nach einem der vorhergehenden Ansprüche, wobei die Verbindung selektiv an Glykoprotein IIb/IIIa und den Vitronectinrezeptor bindet.

**11.** Verwendung nach einem der vorhergehenden Ansprüche, wobei das Medikament zur Verabreichung in Verbindung mit einem Mittel vorgesehen ist, das aus einem thrombolytischen Mittel und einem Antikoagulans oder einem Antithrombozyten-Mittel ausgewählt ist.

**12.** Verwendung nach Anspruch 11, wobei das Antithrombozyten-Mittel Heparin ist.

**Revendications**

**1.** Utilisation d'un composé qui se lie de manière sélective à la glycoprotéine IIb/IIIa pour la préparation d'un médicament destiné à inhiber la resténose chez l'être humain, dans laquelle le composé est une immunoglobuline chimère ou un fragment d'immunoglobuline comprenant une région d'origine non humaine issue de l'anticorps monoclonal 7E3, se liant à un antigène et une région constante humaine.

**2.** Utilisation selon la revendication 1 pour la préparation d'un médicament destiné à inhiber la resténose chez un être humain souffrant d'une insuffisance coronarienne.

**3.** Utilisation selon la revendication 1 ou 2 pour la préparation d'un médicament destiné à inhiber la resténose suite à une intervention chirurgicale touchant l'artère coronarienne chez un être humain.

**4.** Utilisation selon la revendication 1 ou 2 pour la préparation d'un médicament destiné à inhiber la resténose suite à une angioplastie chez l'être humain, par exemple l'angioplastie coronaire transluminale percutanée.

**5.** Utilisation selon l'une quelconque des revendications précédentes, dans laquelle ladite immunoglobuline ou ledit fragment d'immunoglobuline inhibe l'agrégation plaquettaire.

**6.** Utilisation selon l'une quelconque des revendications précédentes, dans laquelle le fragment d'immunoglobuline est un fragment Fab, Fab' ou $F(ab')_2$.

**7.** Utilisation d'un composé qui se lie de manière sélective à la glycoprotéine IIb/IIIa pour la préparation d'un médicament destiné à réduire ou à empêcher des complications ischémiques non aiguës de l'angioplastie chez l'être humain, dans laquelle le composé est une immunoglobuline chimère ou un fragment d'immunoglobuline comprenant une région d'origine non humaine se liant à un antigène issue de l'anticorps monoclonal 7E3, et une région constante humaine.

**8.** Utilisation selon la revendication 7, dans laquelle ladite immunoglobuline ou ledit fragment d'immunoglobuline inhibe l'agrégation plaquettaire.

**9.** Utilisation selon la revendication 7 ou 8, dans laquelle le fragment d'immunoglobuline est un fragment Fab, Fab' ou $F(ab')_2$.

**10.** Utilisation selon l'une quelconque des revendications précédentes, dans laquelle le composé se lie de manière sélective à la glycoprotéine IIb/IIIa et au récepteur de la vitronectine.

**11.** Utilisation selon l'une quelconque des revendications précédentes, dans laquelle le médicament est destiné à une administration de manière conjointe avec un agent choisi parmi le groupe comprenant un agent thrombolytique, un anticoagulant ou un agent antiplaquettaire.

12. Utilisation selon la revendication 11, dans laquelle l'agent antiplaquettaire est l'héparine.

FIG. 1

FIG. 2A p7E3V$_K$hC$_K$

FIG. 2B p7E3V$_H$hC$_{\gamma 4}$

HUMAN PLATELET BINDING

○────○ 7E3 IgG
●────● 7E3 CHIMERIC

FIG. 3

EP 0 725 655 B2

PLATELET AGGREGATION, HUMAN

FIG. 4

CLEARANCE DATA FOR CHIMERIC 7E3
FREE CHIMERIC 7E3 THROUGH 72 HOURS

FIG. 5

**Dose Response at 2 Hours**
Bolus Injection

FIG. 6 (A - C)

FIG. 7 (A - C)

FIG. 8 (A - C)

FIG. 9

FIG. 10

Primary Efficacy Endpoint

**FIG. 11**

**FIG. 12**

**FIG. 13**

**FIG. 14**

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 205207 A
- EP 206532 A
- GB 8500392 W
- WO 8601533 A
- WO 8911538 A
- WO 9306863 A
- MD 20852


### Non-patent literature cited in the description

- *Proc. Natl. Acad. Sci. USA,* 1984, vol. 81
- **POTTER et al.** *Proc. Natl. Acad. Sci. USA,* 1984, vol. 81, 7161
- **PLUCKTHUN, A.** *Bio/Technology,* 1991, vol. 9, 545-551
- **SKERRA, A. et al.** *Bio/Technology,* 1991, vol. 9, 273-278
- **JOLLEY, M.E. et al.** *J. Immunol. Meth.,* 1984, vol. 67, 21
- **SUN, L. et al.** *Proc. Natl. Acad. Sci. USA,* 1987, vol. 84, 214-218
- **COLLER, B. et al.** *J. Clin. Invest.,* 1983, vol. 73, 325-338
- **SUN et al.** *Proc. Natl. Acad. Sci. USA,* 1987, vol. 84, 214-218
- **GOLD, H.K. et al.** *J. Clin. Invest.,* 1990, vol. 86, 651-659
- **DETRE, K.M. et al.** *Circulation,* 1991, vol. 82, 739-750
- **RAO et al.** *J. Am. Coll. Cardiol.,* 1988, vol. 11, 1-11
- **DE FEYTER, P.J.** *Editorial. Am. Heart J.,* 1989, vol. 118, 860-868
- **RUPPRECHT, H.J. et al.** *Eur Heart J.,* 1990, vol. 11, 964-973
- Elective coronary angioplasty: technique and complications. **ELLIS, S.G.** Textbook of Interventional Cardiology. W.B. Saunders Co, 1990
- **DE FEYTER, P.J. et al.** *Circulation,* 1991, vol. 83, 927-936
- **ELLIS, S.G. ; TOPOL, E.J.** *Am. J. Cardiol.,* 1990, vol. 66, 932-937
- ACC/AHA Task Force Report: Guidelines for percutaneous transluminal coronary angioplasty. *J. Am. Coll. Cardiol.,* 1988, vol. 12, 529-545
- **SAMSON, M. et al.** *Am. Heart J.,* 1990, vol. 120, 1-12
- Elective coronary angioplasty: technique and complications. **ELLIS, S.G.** Textbook of Interventional Cardiology. W.B. Saunders Co, 1990
- **DE FEYTER, P.J.** *Circulation,* 1991, vol. 83, 927-936
- **ELLIS, S.G. et al.** *Circulation,* 1988, vol. 77, 372-379
- **DEFEYTER, P.J.** *Circulation,* 1991, vol. 83, 927-936
- **GRUENTZIG, A.R. et al.** *N. Engl. J. Med.,* 1987, vol. 316, 1127-1132
- **PARISI, A.F. et al.** *N. Engl. J. Med.,* 1992, vol. 326, 10-16
- **LINCOFF, A.M. et al.** *J. Am. Coll. Cardiol,* 1992, vol. 19, 926-938
- **TENAGLIA, A.N. et al.** *Am. J. Cardiol,* 1993
- **DETRE, K.M. et al.** *Circulation,* 1990, vol. 82, 739-750
- **ELLIS, S.G. et al.** *Circulation,* 1988, vol. 77, 372-379
- **ELLIS, S.G. et al.** *J. Am. coll. Cardiol,* 1991, vol. 17, 89B-95B
- **MOUSHMOUSH, B. et al.** *Cath. Cardiovasc. Diagn,* 1992, vol. 27, 97-103
- **MYLER, R.K. et al.** *Circulation,* 1990, vol. 82, II-88-II-95
- **SCHWARTZ, L. et al.** *N. Engl. J. Med,* 1988, vol. 318, 1714-1719
- **BARNATHON, E.S. et al.** *Circulation,* 1987, vol. 76, 125-134
- **TENAGLIA, A.M. et al.** *J. Am. Coll. Cardiol.,* 1993
- **ELLIS, S.G. et al.** *Cor. Art. Dis,* 1993, vol. 4, 167-75
- **RYAN, T.J. et al.** *J. Am. Coll. Cardiol,* 1988, vol. 12, 529-45
- **ELLIS, S.G. et al.** *J. Am. Coll. Cardiol,* 1991, vol. 17, 89B-95B
- **DOUGHERTY, K.G. et al.** *Abstracts of the 63rd Scientific Sessions,* 1991, vol. III, 189
- **RATH, B. et al.** *Br. Heart. J., 63,* 1990, 18-21
- **OGILBY J.D. et al.** *Cath. Cardiovasc. Diag,* 1989, vol. 18, 206-9

- **SANE, D.C. et al.** *Ann. Intern. Med,* 1989, 1010-22
- **RAO, A.K. et al.** *J. Am. Coll. Cardiol,* 1988, vol. 11, 1-11
- **LANDEFIELD, C.S. et al.** *Am. J. Med.,* 1987, vol. 82, 703-13
- **KAPLAN, E.L. et al.** *J. Am. Stat. Assn,* 1958, vol. 53, 457-81
- **KALBFLEISCH, J.D. ; R.L. PRENTICE.** *The Statistical Analysis of Failure Time Data,* 1980
- **STACK, R.S. et al.** *J. Am. Coll. Cardiol,* 1988, vol. 11, 1141-49
- **SUGRUE, D.D. et al.** *Br. Heart J.,* 1986, vol. 56, 62-66
- **HETTLEMAN, B.D. et al.** *J. Am. Coll. Cardiol,* 1990, vol. 15, 154A
- **SINCLAIR, I.N. et al.** *Am. J. Cardiol.,* 1988, vol. 61, 61G-66G
- **RUOCCO, N.A. et al.** *Am. J. Cardiol,* 1992, vol. 69, 69-76
- **ELLIS, S.G. et al.** *Am. J. Cardiol,* 1989, vol. 63, 30-4
- **KLEIN, L.W. et al.** *J. Am. Coll. Cardiol,* 1991, vol. 17, 621-6
- **HUNT, A.C. et al.** *Eur. Heart J.,* 1991, vol. 12, 690-3
- **PAULETTO, P. et al.** *Am. J. Cardiol,* 1987, vol. 69, 999-1000
- **SPADARO, J.J. et al.** *Cath. Cardiovasc. Diagn,* 1986, vol. 12, 230-4
- **WALL, T.C. et al.** *J. Am. Coll. Cardiol,* 1993, vol. 21, 597-603
- **DONAHUE, J.G. et al.** *N. Engl. J. Med.,* 1992, vol. 327, 369-73
- **DODD, R.Y.** *N. Engl. J. Med,* 1992, vol. 327, 419-21
- **NELSON, K.E. et al.** *Ann. Intern. Med,* 1992, vol. 117, 554-9
- **POPMA, J.J. et al.** *Circulation,* 1991, vol. 84, 1426-1436
- **TOPOL, E.J. et al.** *Circulation,* 1993, vol. 87, 1489-1497
- **HERRMAN, J.-P.R. et al.** *Drugs,* 1993, vol. 46, 249-262
- **FORRESTER, J.S. et al.** *J. Am. Coll. Cardiol,* 1991, vol. 17, 758-769
- **IP, J.H. et al.** *J. Am. Coll. Cardiol,* 1991, vol. 17, 77B-88B
- **CASSCELLS, W.** *Circulation,* 1993, vol. 86, 723-729
- **MERCATOR STUDY GROUP.** *Circulation,* 1992, vol. 86, 100-110
- **PLOW, E.F. et al.** *Prog. Hemostas. Thromb,* 1988, vol. 296, 320-331
- **COLLER, B.S.** *J. Clin. Invest,* 1985, vol. 76, 101-108
- ACC/AHA Task Force Report. *J. Am. coll. Cardiol,* 1988, vol. 12, 529-545
- **DETRE, K.M. et al.** *J. Am. Coll. Cardiol,* 1989, vol. 13, 230A
- **KAPLAN, E.L. ; P. MEIER.** *J. Am. Stat. Assn,* 1958, vol. 53, 457-481
- **ELLIS, S.G. et al.** *Cor. Art. Dis,* 1993, vol. 4, 1675-175
- **TCHENG, J.E. et al.** *Circulation,* 1993, vol. 88
- **HYNES, R.O.** *Cell,* 1992, vol. 69, 11-25
- **SUTTON, J. et al.** *Clinical Research AFCR,* 1993, 118A
- **TOPOL, E.J. et al.** *N. Engl. J. Med,* 1993, vol. 329, 228-233
- **ADELMAN, A.G. et al.** *N. Engl. J. Med,* 1993, vol. 329, 228-233
- **SERRUYS, P.W. et al.** *Circulation,* 1991, vol. 84, 1568-1580
- **SCHWARTZ, R.S. et al.** *J. Am. Coll. Cardiol,* 1992, vol. 20, 1284-1293
- **TOPOL, E.J.** *Mayo Clin. Proc,* 1993, vol. 68, 88-90
- **WILLERSON, J.T. et al.** *Proc. Natl. Acad. Sci. USA,* 1991, vol. 88, 10624-10628